# EUROPEAN PATENT SPECIFICATION

(11) **EP 3 592 861 B1**
(45) Date of publication and mention of the grant of the patent: **03.09.2025**
(21) Application number: 18711860.9
(22) Date of filing: 07.03.2018
(51) Int. Cl.: C12Q 1/6806, C12Q 1/6883, C12Q 1/6886

(54) **RNA PROFILING FOR INDIVIDUALIZED DIET AND TREATMENT ADVICE**
RNA-PROFILERSTELLUNG ZUR INDIVIDUALISIERTEN DIÄT UND BEHANDLUNGSRATSCHLAG
PROFILAGE D'ARN POUR UN RÉGIME INDIVIDUALISÉ ET DES CONSEILS DE TRAITEMENT

(30) Priority: 07.03.2017 EP 17159630
(43) Date of publication of application: 15.01.2020
(73) Proprietor: Stichting Radboud universitair medisch centrum, 6525 GA Nijmegen (NL)
(72) Inventor: LEENDERS, Wilhelmus Petrus Johannes, 6546 DV Nijmegen (NL)
(74) Representative: Nederlandsch Octrooibureau
(86) International application number: PCT/EP2018/055548
(87) International publication number: WO 2018/162525

(56) References cited:
- WO-A1-2013/119827
- US-B1- 9 410 956
- KIFLEMARIAM SARA ET AL: "In situ sequencing identifies TMPRSS2-ERG fusion transcripts, somatic point mutations and gene expression levels in prostate cancers : In situ detection and identification of TMPRSS2-ERG fusion", THE JOURNAL OF PATHOLOGY, 1 August 2014 (2014-08-01), pages n/a - n/a, XP055804423, ISSN: 0022-3417, DOI: 10.1002/path.4392
- LIN SHENGRONG ET AL: "A molecular inversion probe assay for detecting alternative splicing", BMC GENOMICS, BIOMED CENTRAL, vol. 11, no. 1, 17 December 2010 (2010-12-17), pages 712, XP021086313, ISSN: 1471-2164, DOI: 10.1186/1471-2164-11-712
- HIATT J. B. ET AL: "Single molecule molecular inversion probes for targeted, high-accuracy detection of low-frequency variation", GENOME RESEARCH, vol. 23, no. 5, 4 February 2013 (2013-02-04), US, pages 843 - 854, XP055774259, ISSN: 1088-9051, Retrieved from the Internet <URL:https://www.ncbi.nlm.nih.gov/pmc/articles/PMC3638140/pdf/843.pdf> DOI: 10.1101/gr.147686.112
- RONGQIN KE ET AL: "In situ sequencing for RNA analysis in preserved tissue and cells", NATURE METHODS, vol. 10, no. 9, 14 July 2013 (2013-07-14), pages 857 - 860, XP055163946, ISSN: 1548-7091, DOI: 10.1038/nmeth.2563
- KUN ZHANG ET AL: "Digital RNA allelotyping reveals tissue-specific and allele-specific gene expression in human", NATURE METHODS, vol. 6, no. 8, 1 August 2009 (2009-08-01), pages 613 - 618, XP055068957, ISSN: 1548-7091, DOI: 10.1038/nmeth.1357
- GAMEZ-POZO ANGELO ET AL: "MicroRNA Expression Profiling of Peripheral Blood Samples Predicts Resistance to First-line Sunitinib in Advanced Renal Cell Carcinoma Patients", NEOPL, NEOPLASIA PRESS, ANN ARBOR, MI, US, vol. 14, no. 12, 1 December 2012 (2012-12-01), pages 1144 - 1152, XP009168146, ISSN: 1522-8002, DOI: 10.1593/NEO.12734
- CROSETTO: "Spatially resolved transcriptomics and beyond", NATURE REVIEW GENETICS, 1 January 2015 (2015-01-01), pages 57 - 66, XP055284555, Retrieved from the Internet <URL:http://www.nature.com/nrg/journal/v16/n1/pdf/nrg3832.pdf> [retrieved on 20160629], DOI: 10.1038/nrg3832
- PANAGIOTIS A KONSTANTINOPOULOS ET AL: "Gene-expression profiling in epithelial ovarian cancer", NATURE CLINICAL PRACTICE ONCO, NATURE PUBLISHING GROUP, US, vol. 5, no. 10, 1 October 2008 (2008-10-01), pages 577 - 587, XP007910974, ISSN: 1743-4254, DOI: 10.1038/NCPONC1178
- ASTRID EIJKELENBOOM ET AL: "Reliable Next-Generation Sequencing of Formalin-Fixed, Paraffin-Embedded Tissue Using Single Molecule Tags", JOURNAL OF MOLECULAR DIAGNOSTICS,THE, vol. 18, no. 6, 1 November 2016 (2016-11-01), US, pages 851 - 863, XP055386640, ISSN: 1525-1578, DOI: 10.1016/j.jmoldx.2016.06.010
- J. B. HIATT ET AL: "Single molecule molecular inversion probes for targeted, high-accuracy detection of low-frequency variation", GENOME RESEARCH, vol. 23, no. 5, 4 February 2013 (2013-02-04), US, pages 843 - 854, XP055225609, ISSN: 1088-9051, DOI: 10.1101/gr.147686.112

## Description

### Field of the invention

The present invention relates to the field of medicine and molecular diagnostics. In particular, it relates to a novel RNA profiling assay allowing simultaneous detection of alternative splice variants from genes involved in disease, including genes involved in metabolism, resulting in a guidance for personalized treatment with drugs targeting disease-associated molecular aberrations, optionally in combination with dietary compounds, food supplements or inhibitors of metabolism.

### Background of the invention

Malfunctioning cells are typically distinct from healthy cells in that they have altered metabolism. As an example, cells in diabetic patients have adapted to cope with lack of glycogenesis and high extracellular glucose concentrations. In another example, aberrantly growing cells such as in hyperplasia and in cancer need to process excessive amounts of nutrients to produce nucleotides, amino acids and fatty acids for DNA/RNA synthesis, protein synthesis and membrane synthesis. To accommodate this demand, growing cells have adapted by an altered metabolism. There is a number of compounds that can serve as fuel for malfunctioning cells. These include glucose, fatty acids and amino acids, such as glutamine and glutamate. A selection of genes that are involved in cell metabolism are presented in Table I here below. It should be noted that genes involved in a metabolic pathway may also be involved in another metabolic pathway; the person skilled in the art is aware of this.

**Table I: Selection of genes that are involved in cell metabolism**

| *Glucose processing (GLY1: glucose to pyruvate; PPP: pentose phosphate pathway; GLY2: pyruvate to lactate; TCA: tricarboxylic acid cycle)* | |
|---|---|
| 1. | Transmembrane glucose transporters GLUT1 and GLUT3 (*SLC2A1* and *SLC2A*) to ensure glucose import into the cytosol |
| 2. | Hexokinase (HK1,2,3), to convert glucose to glucose-6-phosphate (GLY1) |
| 3. | Glucose-6-phosphate dehydrogenase (G6PD) to convert glucose-6-phosphate to 6-phosphogluconolactone (PPP) |
| 4. | Gluconolactonase to convert 6-phosphogluconolactone to 6-phosphogluconate (PPP) |
| 5. | 6-phosphogluconate dehydrogenase (PGD) to convert 6-phosphogluconate to ribulose-5-phosphate (PPP) |
| 6. | ribulose-5-phosphateisomerase (RPIA) to convert ribulose-5-phosphate to ribose-5-phosphate (PPP) |
| 7. | ribulose-5-phosphate 3-epimerase (RPE) to convert ribulose-5-phosphate to xylulose-5-phosphate (PPP) |
| 8. | Transketolase (TKT) to convert products of step 5 and step 6 to glyceraldehyde-3-phosphate and sedoheptulose 7-phosphate (PPP) |
| 9. | Transaldolase to convert the products of step 8 to fructose-6-phosphate and erythrose 4-phosphate (PPP) |
| 10. | Phosphoglucose isomerase (PGI) to convert glucose-6-hosphate to fructose 6-phosphate (GLY1) |
| 11. | Phosphofructokinase (PFK) to convert fructose-6-phosphate to fructose 1,6-biphosphate (GLY1) |
| 12. | Fructose bisphosphate aldolase (ALDOA) to convert fructose 1,6-biphosphate to dihydroxyacetone phosphate and glyceraldehyde-3-phosphate (GLY1) |
| 13. | Glyceraldehyde 3 phosphate dehydrogenase (GAPDH) to convert glyceraldehyde-3-phosphate to 1,3- biphosphoglycerate (GLY1) |
| 14. | Phosphoglycerate kinase (PGK) to convert 1,3- biphosphoglycerate to 3-phosphoglycerate (GLY1) |
| 15. | Phosphoglycerate mutase (PGAM1/2) to convert 3-phosphoglycerate to 2-phosphoglycerate (GLY1) |
| 16. | Enolase (ENO) to convert 2-phosphoglycerate to phosphoenolpyruvate (GLY1) |
| 17. | Pyruvate kinase (PKM1/2) to convert phosphoenolpyruvate to pyruvate (GLY1) |
| 18. | Pyruvate dehydrogenase (PDH) to convert pyruvate to Acetyl-CoA (TCA) |
| 19. | Pyruvate carboxylase (PC) to convert Acetyl-CoA to oxaloacetic acid (TCA) |
| 20. | Citrate synthase (CS) to produce citrate from Acetyl-CoA and oxaloacetic acid (TCA) |
| 21. | Acotinase (ACO1) to convert citrate to cis-acotinate and isocitrate (TCA) |
| 22. | Isocitrate dehydrogenase 1/2/3 (IDH1/2/3) to convert isocitrate to αKG (TCA) |
| 23. | αKG dehydrogenase (OGDH) to convert αKG to succinyl-CoA (TCA) |
| 24. | Succinyl-CoA synthetase (SUCLA2) to convert succinyl-CoA to succinate (TCA) |
| 25. | NADH-coenzyme Q oxidoreductase (OXPHOS) |
| 26. | Succinate-Q-oxidoreductase (OXPHOS) |
| 27. | Flavoprotein_Q oxidoreductase (OXPHOS) |
| 28. | Cytochrome C oxidase (OXPHOS) |
| 29. | ATP synthase (OXPHOS) |
| 30. | Succinate dehydrogenase (SDHA/B/C/D) to convert succinate to fumarate (TCA) |
| 31. | Fumarate hydratase (FH) to convert fumarate to malate (TCA) |
| 32. | Malate dehydrogenase (MDH1/2) to convert malate to oxaloacetate (TCA) |
| 33. | Pyruvate dehydrogenase kinase (PDK), to phosphorylate and block PDH (step 18) (GLY2) |
| 34. | Lactate dehydrogenase (LDHA) to produce lactate from pyruvate (GLY2) |
| 35. | Lactate dehydrogenase (LDHB) to convert lactate to pyruvate. |
| 36. | Monocarboxylate transporters MCT1 (SLC16A1) and MCT4 (SLC16A3) to transport lactate and associated protons from the cell, to regulate pH homeostasis |
| 37. | Carbonic anhydrases (CA9 and CA12) to produce HCO₃⁻ from H₂O and CO₂ at the cell surface |
| 38. | HCO₃⁻ importer (SLC4A10) to import HCO₃⁻ for pH homeostasis |

### Glutamine processing

There is a number of cells that also depend on the amino acid glutamine for proliferation. Genes that are involved in glutamine metabolism include
39. SLC1A5 or ASCT2, a membrane importer protein for glutamine
40. Glutaminase (GLS) to convert glutamine to glutamate
41. Glutamate dehydrogenase (GLUD1/2) to convert glutamate to alpha-ketoglutarate (αKG)
42. Branched chain amino acid transferase 1 and 2 (BCAT1/2) to produce glutamate from αKG
43. Excitatory amino acid transporter EAAT2 (SLC1A2) to import glutamate into the cell
44. System Xc₋ (SLC7A11) to export glutamate from the cell in exchange for cystin

### Fatty acids

Fatty acids are important building blocks for cells because they are the basis for synthesis of phospholipid bilayers that make up membranes for nuclei, mitochondria, endoplasmic reticulum, golgi apparatus and lysosomes and peroxisomes. Enzymes that are involved in fatty acid anabolism include
45. CIC (SLC25A1) to transport citrate from mitochondria to cytosol
46. ATP citrate lyase (ACLY) to convert citrate to oxaloacetate and acetyl-CoA
47. Acetyl CoA carboxylase (ACACA, ACACB) to convert acetyl-CoA to malonyl-CoA
48. Fatty acid synthase (FASN) to convert Acetyl-CoA, malonyl-CoA and NADPH to palmitate
49. Fatty acid transporter (CPT1) for uptake of fatty acids
50. choline transporter (SLC5A7) to import choline
51. Choline kinase (CHKA) to convert choline to phosphatidylcholine
52. Carnitine palmitoyltransferase 2 (CPT2) to convert acylcarnitine to long chain Acyl-CoA
53. Acyl CoA dehydrogenase (VLCAD) to convert long chain acyl-CoA to 2-Enoyl-CoA
54. Trifunctionalportein (HADHA/B) to convert 2-Enoyl-CoA to medium and short chain Acyl-CoA
55. Acyl CoA dehydrogenase (SCAD, MCAD, LCAD) to convert cyl-CoA to 2-Enoyl CoA
56. 2-Enoyl-VoA hydratase to convert 2-Enoyl-CoA to 3-hydroxyacyl-CoA
57. 3-hydroxyacyl-CoA dehydrogenase (SCHAD) to convert 3-hydroxyacyl-CoA to 3-ketoacyl CoA
58. 3-ketoacyl-CoA thiolase (MCKAT) to convert 3-ketoacyl-CoA to acetyl-CoA

### Metabolic alterations in cancer

Altered metabolism may be a result of cancerous transformation of cells, but for a number of cancer types it is also a cause of cancer. A well-known example of metabolic alterations (alterations within one or more metabolic pathway) resulting from cancer growth is the alterations that are a consequence of hypoxia, the lack of oxygen that occurs in growing tissues that have outgrown the vascular blood supply. Under oxygenated conditions, the transcription factors Hypoxia Inducible Factors HIF1α and HIF2α are hydroxylated by the oxygen-dependent enzyme proline hydroxylase (PHD). Proline-hydroxylated HIFs have binding sites for the VHL-E3 ubiquitin complex, resulting in HIF ubiquitinylation and proteasomal breakdown. This pathway is an important regulator of HIF-levels in cells. Under normoxic conditions glucose will be converted to pyruvate that will be processed to acetyl-CoA which enters the mitochondria for processing in the tricarboxylic acid (TCA) cycle. The TCA cycle is directly coupled to oxidative phosphorylation and yields for every mole of glucose the energy equivalent of 36 moles of ATP, CO₂ and H₂O. Full processing of glucose via this pathway does not yield carbon building blocks.

Under hypoxic conditions PHDs are inactive and as a result unhydroxylated HIF1/2α will accumulate in cells, heterodimerize with HIF-β (ARNT) and activate genes that are needed to survive hypoxia. These genes (Table I, steps 1-38) regulate different processing of glucose, using pyruvate for lactate instead of acetyl CoA production. Conversion of pyruvate to lactate yields only 2 moles of ATP for every mole of glucose. The inefficiency of this process in terms of energy production requires extra intake of glucose, which is accomplished by increased expression of glucose transporters (GLUT1, GLUT3). Genes involved in the next steps of glucose processing are also activated (especially hexokinase 2). HIF accumulation also results in activation of the gene encoding vascular endothelial growth factor (VEGF-A), resulting in an angiogenic response.

Glycolysis in cancer is not restricted to hypoxic areas but can also occur under normoxic conditions. There is a number of causes for glycolysis in normoxic cancers, for example elevated expression of the Myc oncogene [resulting in activation of PDK (Table I, step 33) and preventing influx of acetyl-CoA into the TCA cycle], decreased function of tumor suppressors (VHL, PTEN) and elevated activity of oncogenic pathways (e.g. PI3K, AKT) all leading to increased HIF activity. Aerobic glycolysis in cancers is known as the Warburg effect.

Whereas aberrations in oncogenes and tumor suppressor genes in a cancer and conditions such as hypoxia induce metabolic alterations, these alterations depend on the specific nature of the molecular aberrations. As a consequence each tumor has its own specific metabolic demands.

Adding an extra level of complexity, instead of being a consequence of carcinogenesis, altered metabolism may also drive carcinogenesis. Hotspot mutations in *isocitrate dehydrogenase* 1 and 2 (Table I, step 22) in substantial percentages of diffuse gliomas of the brain, acute myeloid leukemia, chondrosarcomas and hepatic cholangiocarcinomas result in consumption of alpha ketoglutarate (α-KG) and NADPH to produce the oncometabolite D-2-hydroxyglutarate (D-2HG) that can accumulate to milliMolar concentrations. The small difference between the chemical structures of α-KG and D-2HG in combination with the high concentrations of the latter, results in competitive displacement of α-KG from α-KG-dependent enzymes that subsequently cannot function properly. Important examples are the Ten Eleven Translocation (TET)-family of enzymes that are involved in demethylation of CpG islands in the DNA, and JmJ proteins that are involved in histone demethylation. Consequently IDH-mutated cancers present with hypermethylated CpG islands and histones, resulting in deranged gene transcription profiles. Of importance, the consumption of NADPH in IDH-mutated cancer cells results in low levels of reduced glutathione and decreased resistance to reactive oxygen species (ROS). The increased activity of ROS in IDH mutated cancer cells may increase the chance of second hits in oncogenes and tumor suppressor genes, and result in cancer. Except for the known hotspot mutation that leads to 2-HG production, IDH-mutants have been described that are defective in NADPH production, but do not produce D-2HG (1).

Other examples of cancers in which mutations in metabolic enzymes are cancer drivers are phaeochromocytomas and paragangliomas, that carry inactivating mutations in one of the SDH subunits A-D (Table I, step 30) or in SDH assembly factor SDHAF2 (2). These mutations can be hereditary, leading to the HPGL/PCC syndrome, or somatic. Other mutations in metabolic genes that cause cancer occur in FH (Table I, step 31). Mutations in FH are associated with leyomyomatosis and papillary renal cell cancer (3). Mutations in VHL protein occur in clear cell renal cell cancers, and directly result in glycolysis via a defect in HIF breakdown, as described above. The IDH, SDH and FH genes can therefore be considered tumor suppressor genes.

Dietary compounds, food supplements or safe to use drugs exist that can inhibit metabolic pathways, and the use of such drugs have been considered as potentially beneficial for the treatment of cancer. Examples are deoxyglucose, inhibiting glucose uptake by the cell and preventing glycolysis (Table I, step 1 and following) (4), 3-bromopyruvate, blocking the activity of hexokinases (Tabel I, step 2 and following) (5, 6), 6-amino-niocotinamide (6-AN, blocking G6PD in the pentose phosphate pathway, Table I, step 3 (7)), metformin, blocking OXPHOS (8), bis-2-(5-phenylacetamido-1,2,4-thiadiazol-2-yl)ethyl sulfide (BPTES, blocking glutaminase, Table I, step 28), epigallo-3-catechin gallate (EGCG, blocking glutamate dehydrogenases and other NADPH-generating enzymes (Table I, step 41) and fatty acid synthase Table I, step 36 (9)) (10), cerulenin, inhibiting fatty acid synthase (Table I, step 48) (9). These inhibitors have been shown to have antitumor effects in different models of cancers.

Although these compounds have also been tested in humans, anti-cancer effects may require systemic concentrations that are not tolerated by healthy tissues. At non-toxic concentrations, treatment with metabolic inhibitors below maximal tolerated doses can however augment the activity of other treatments, such as radiotherapy, chemotherapy or targeted therapy.

Current treatment protocols for patients with cancers who cannot be cured by surgery are ineffective in that cancers generally develop resistance to treatment (11-14).

There is therefore a great need for safe and adjuvant treatments that increase the efficacy of the state of the art therapies. These state of the art therapies are applied according to guidelines that are based on the outcomes of phase III clinical trials. For some cancer types, the effects of treatment can be predicted (e.g. colon cancers with KRAS mutations do not respond to EGFR inhibitors, cancers with the BRAF-V600E mutation develop resistance to the BRAF inhibitor vemurafenib by upregulating signaling from EGFR, gliomas with hypermethylation of the DNA repair gene MGMT respond better to the DNA alkylating chemotherapy Temozolomide). Although some cancer types are now routinely analyzed for so called companion biomarkers -biomarkers based on which a personalized treatment can be initiated- analyses on such markers cannot be performed if tissue cannot be made available, such is e.g. the case in patients with inoperable cancer.

Therefore there is an urgent need for a test that measures parameters in a patient (subject) that are relevant for treatment decision making, with treatment protocols consisting of the most appropriate metabolic inhibitors, combined with the most appropriate available targeted drugs or radiotherapy or chemotherapy. Currently available tests to investigate metabolism in cancer include magnetic resonance spectroscopic imaging (MRSI), but this is a technically challenging technique that can only be performed in specialized centers and requires concomitant in depth knowledge of MR principles and cell biology. Furthermore, MRSI can only measure a limited number of metabolites. An alternative method to investigate metabolism in cancer is to make extracts of metabolites of a cancer and perform mass spectroscopy. In this case, results will be influenced by the fact that these assays are performed on tissues that have seen hypoxia after surgery.

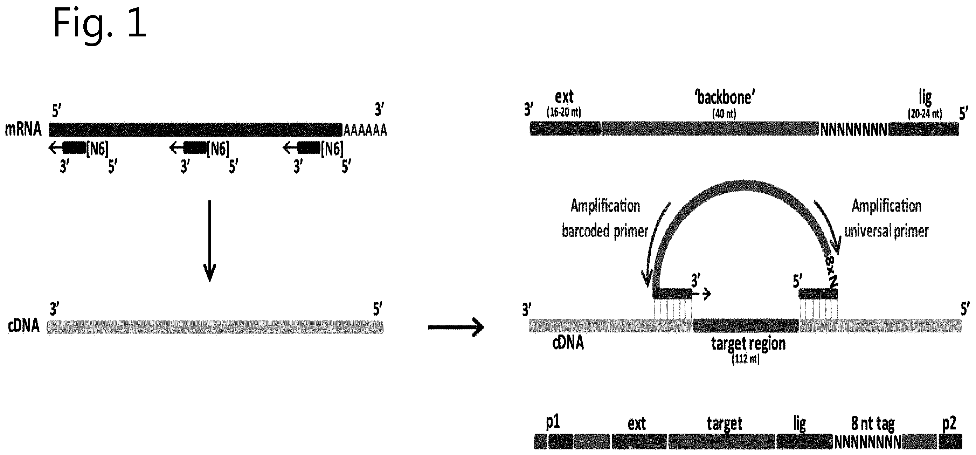

Molecular diagnosis of cancer is currently performed by analysis of tumor DNA and aims for detection of actionable mutations. DNA analyses allow the identification of mutations and variations in metabolic enzymes such as FH, SDH and IDH, and actionable mutations and amplifications in oncogenes and tumor suppressor genes. DNA analyses can be performed using whole genome analyses or whole exome analyses but can also be performed with Molecular Inversion Probes as described in the literature (15). The technique is depicted in Figure 1. MIPs inversely hybridize to a DNA of interest via an extension probe and a ligation probe that are connected by a backbone sequence, leaving a small gap on the target sequence. This gap is enzymatically filled and ligated, leaving a circular molecule that can be purified by exonuclease-based degradation of non-circularized nucleotide strands. PCR-based amplification of the filled gap using oligonucleotide primers in the backbone generates a library of amplicons that can be analyzed using e.g. next generation sequencing methodology. To make the assay quantitative, a unique molecular identifier (UMI) of e.g. 8 random nucleotides can be incorporated in the MIP, to allow a back calculation of all PCR products with the same UMI to one MIP. The chance of 2 different smMIPs having the same UMI is (¼)⁸ = 1:65,536 which makes these UMIs unique for each MIP. MIPs with a UMI are called single molecule MIPs or smMIPs (16). The technique of smMIPs for analyses of DNA sequences is described in the literature.

DNA analyses cannot measure gene activity, e.g. activity of metabolic genes, which is regulated by epigenetic processes and the presence of transcription factors and transcription repressors (17).

### Description of the figures

**Fig. 1** Principle of smMIP-based targeted RNA sequencing. The procedure depends on the hybridization of molecular inversion probes consisting of a ligation and an extension probe that are connected via a backbone sequence. Capture hybridization leaves for each smMIP a gap of 112 nt that is enzymatically extended and closed by ligation. After exonuclease digestion of non-ligated probes the remaining library of circularized smMIPs is PCR-amplified with primers in the smMIP backbone. The ligation probe is flanked by a random 8N unique molecular identifier (UMI) sequence that allows correction for PCR duplicates. During PCR, for each sample a unique barcode primer is used allowing identification of sample-specific reads.

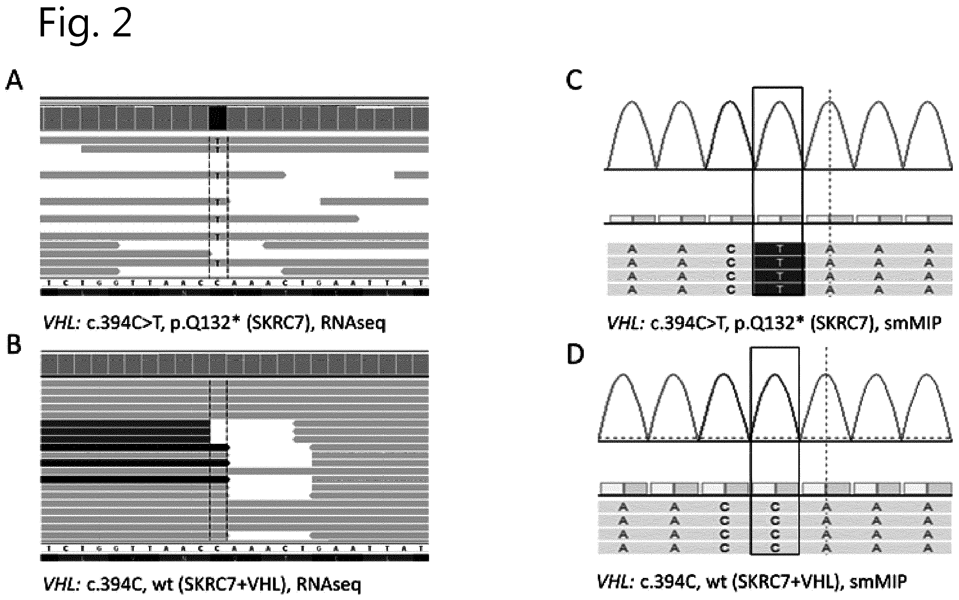

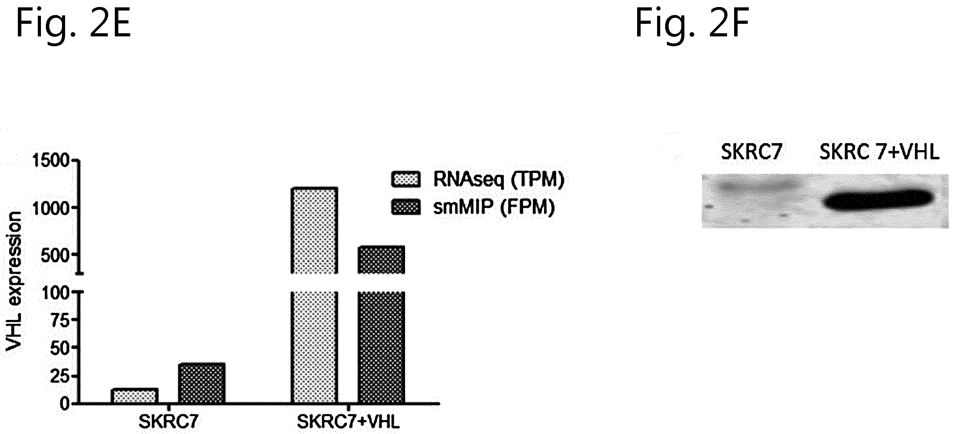

**Fig. 2** A,B Inegrative Genome Viewer (IGV) representation of the VHL locus of SKRC7 and SKRC7-VHL^{HA} cells. BAM files containing whole RNAseq data from these cell lines were loaded into IGV. Note the CAA-UAA mutation, resulting in the VHL ^{Q132-stop} mutation at the protein level. C and D show SeqNext representations of the same VHL locus of SKRC7 (C) and SKRC7-VHL^{HA} cells. E) bar graph showing VHL-related TPM and FPM values of SKRC7 and SKRC7-VHL^{HA}. F) Western blot of SKRC7 cells and the VHL-expressing derivative, stained with an anti-HA antibody.

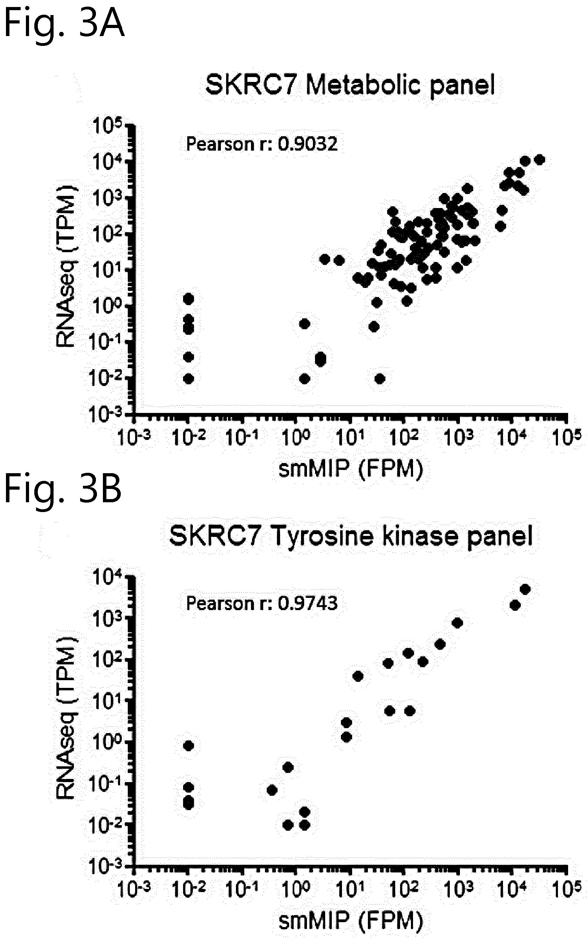

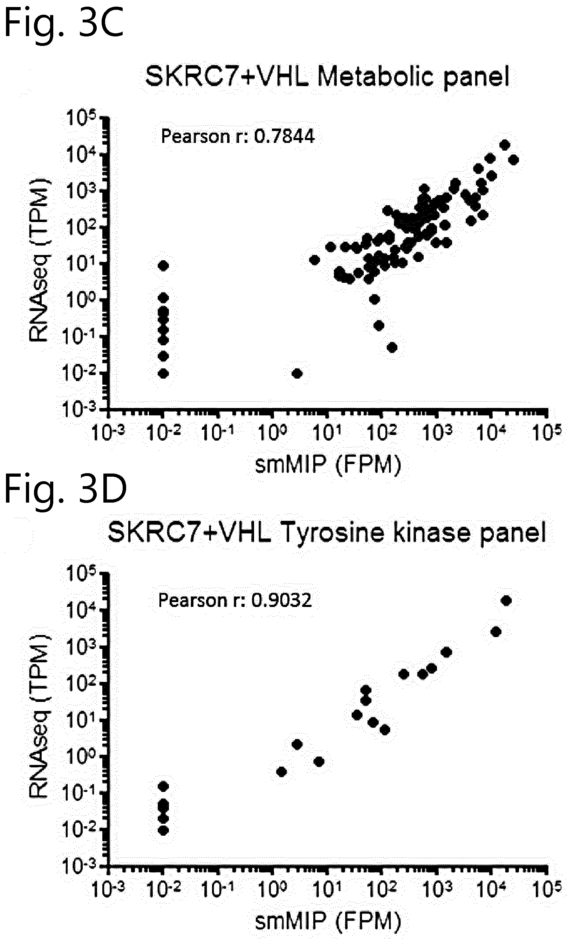

**Fig. 3** smMIP-based targeted RNA sequencing correlates well with whole transcriptome RNAseq. Mean smMIP-based metabolic FPM levels (A,C) and tyrosine kinase transcript FPM levels (B,D) were plotted to TPM levels of the same transcripts, extracted from whole RNAseq data. Note that the transcripts with very low FPM values (10⁻²FPM) were not detected in the RNAseq dataset. We included these transcripts in these analyses although they may have lowered the Pearson coefficient.

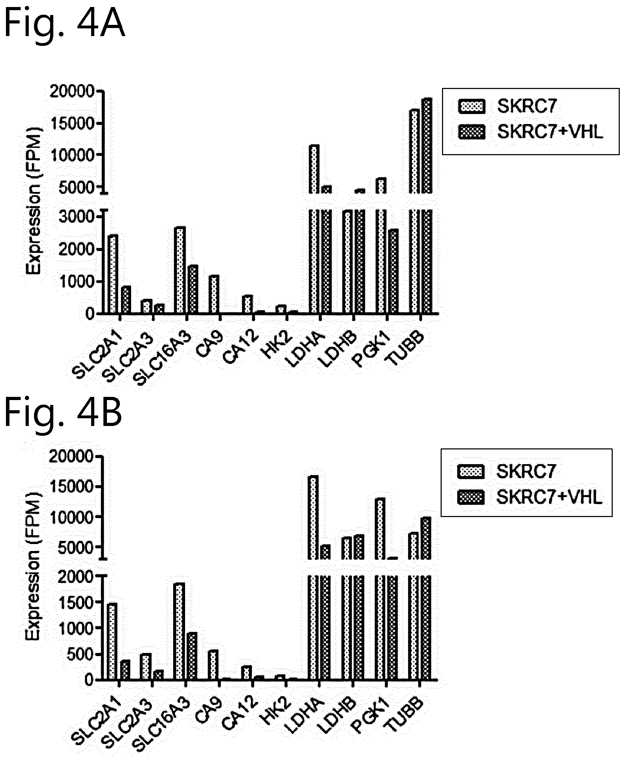

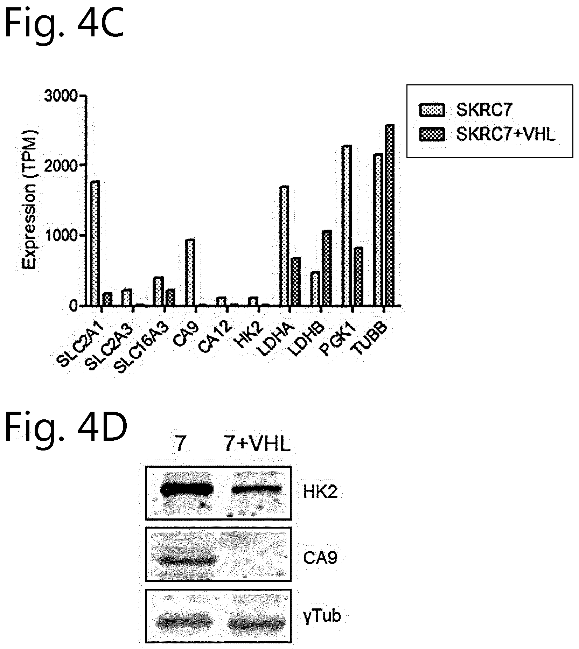

**Fig.4** SmMIP-based targeted RNAseq reveals decreased expression levels of glycolysis related genes a.o. SLC2A1, CA9, HK2 and LDHA in two independent duplicate experiments (A,B). Relative values were comparable to those obtained from whole transcriptome RNA seq analysis (C), which is in agreement with the correlation shown in Figure 3. Differences in expression levels were validated on the protein level for HK2 and CA9, using tubulin as housekeeping control (D).

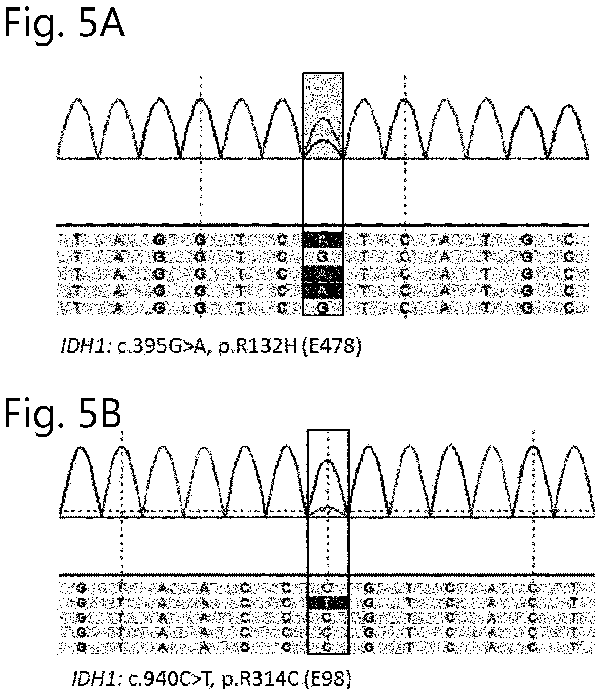

**Fig.5** smMIP-based targeted RNA next generation sequencing can be used for adequate variant calling. Shown are the loci containing the IDH1-R132H mutation in E478 xenografts (A) and in a clinical grade III astrocytoma (C, this mutation was confirmed by genetic analysis), whereas the IDH1-R314C mutation in E98 cells could also be identified (B).

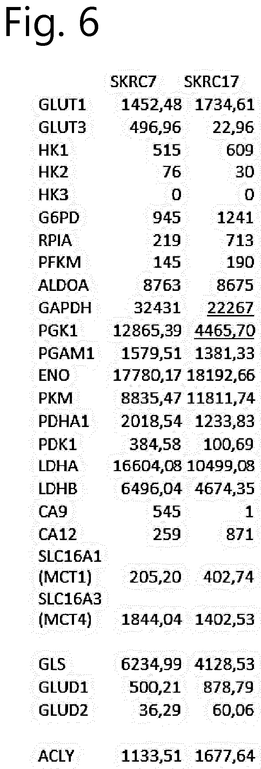

**Fig.6** Example of smMIP analysis of RNAs encoding metabolic enzymes in two different ccRCC cell lines.

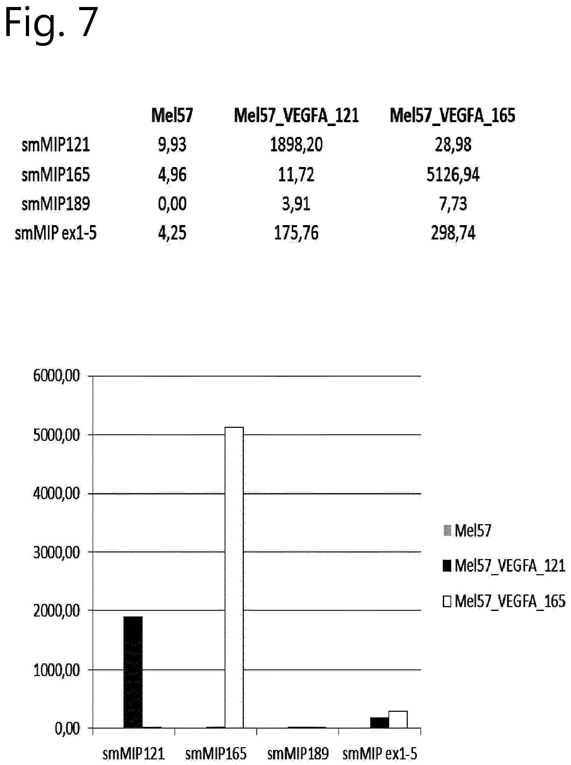

**Fig.7** smMIPs allow specific detection of splice variants. The Mel57 cell line that does not express endogenous VEGF-A, was transfected with expression plasmids pIRESneo-VEGF-A121 and pIRESneo-VEGF-A165, and cultured in medium containing neomycin to generate stable transfectants. RNA from these transfectants were subjected to smMIP profiling with a panel of smMIPs, among which smMIP121 with ligation and extension probes in exons 5 and 8 of the VEGF-A transcript, respectively, hence detecting only VEGF-A121, and smMIPs with ligation and extension probes in exons 5 and 7 of the VEGF-A transcript respectively, hence detecting only VEGF-A165. Note that smMIP121 detects VEGF-A121, but not VEGF-A165, and smMIP165 detects only VEGF-A165, but not VEGF-A121.

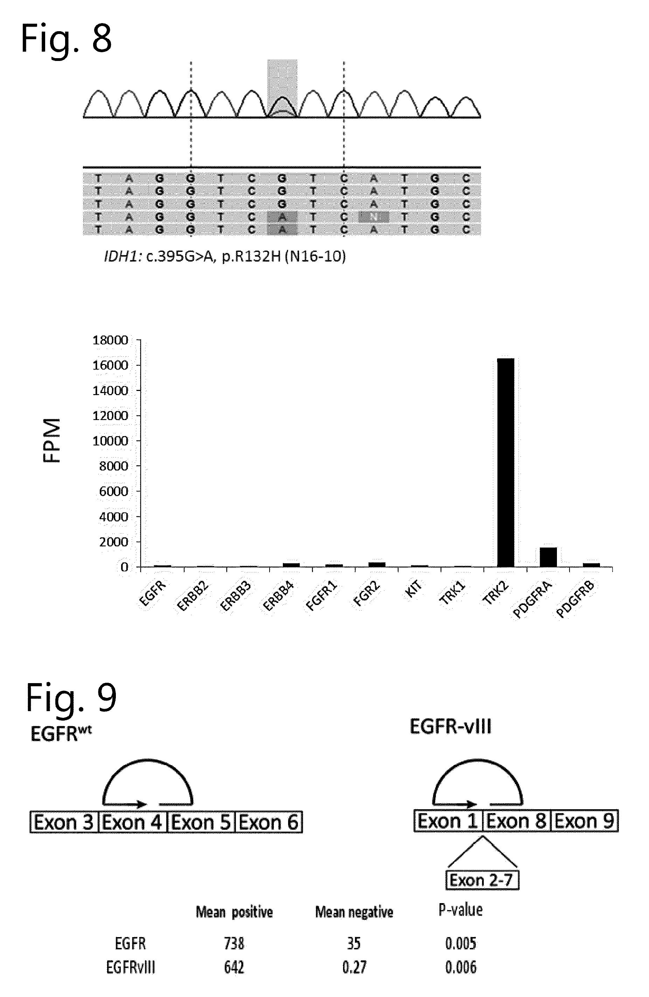

**Fig.8** smMIP-based targeted RNA next generation sequencing can be used for adequate diagnosis. Shown is the IDH locus containing the IDH1-R132H mutation in a clinical grade III astrocytoma. Analysis of the tyrosine kinase transcriptome reveals high expression levels of the genes encoding the tyrosine kinases NTRK2 and PDGFRA in this tumor, suggestive of responsiveness to the corresponding tyrosine kinase inhibitors.
**Fig. 9** smMIP based detection of EGFR splice variants in gliomas. Shown is that in the group of gliomas there is elevated expression of EGFR in 39/75 brain tumors (52%; mean FPM 738 in positives vs mean FPM 35 in negatives, using an arbitrary cut off FPM value of 100) and expression of EGFRvIII in 12/75 brain tumors (16%; mean FPM 642 in positives vs mean FPM 0.27 in negatives, using an arbitrary cut-off value of 6).

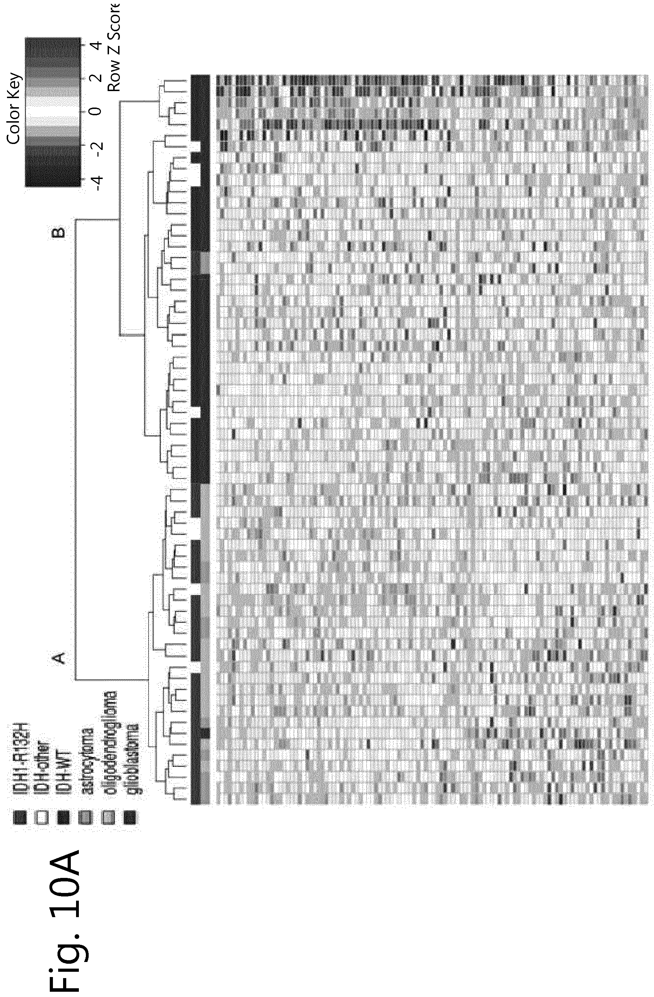

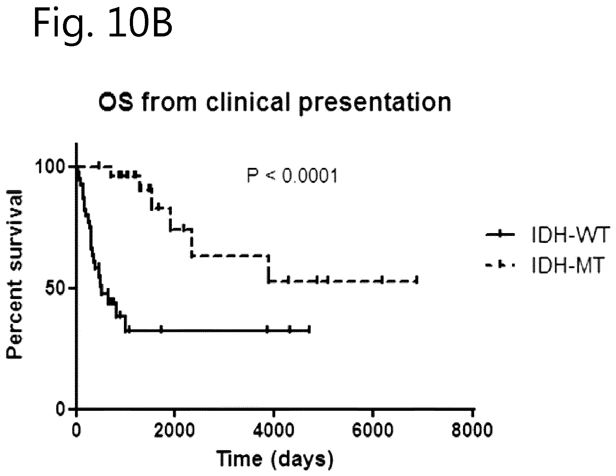

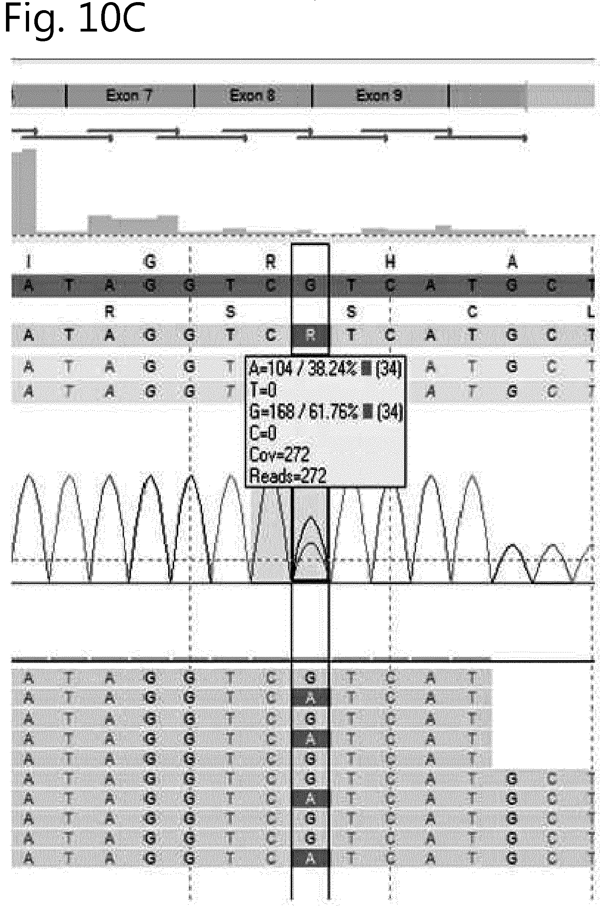

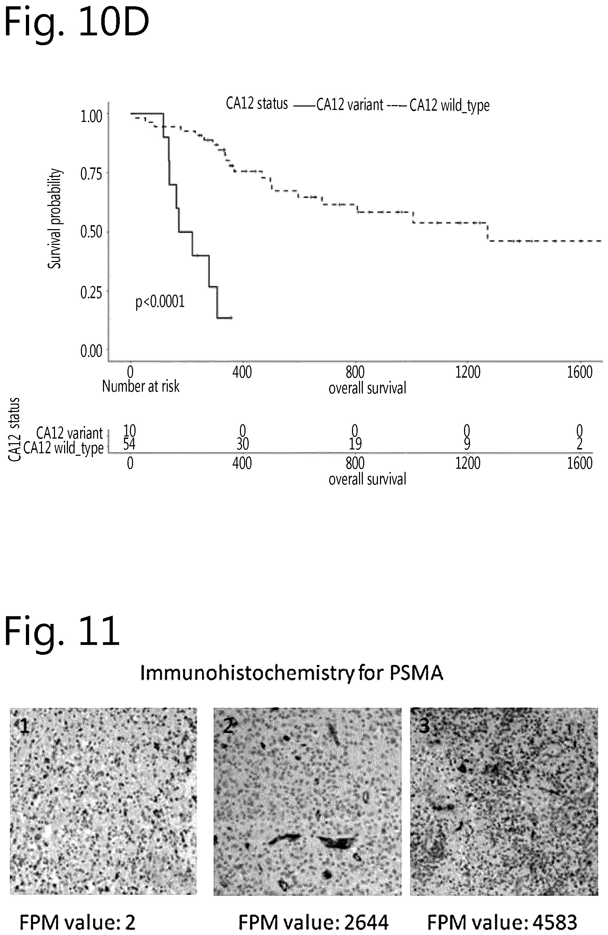

**Fig.10** smMIP based targeted RNA sequencing can be used for accurate diagnosis and prognosis.
   A) Heat map of the individual gene profiles. Unsupervised agglomerative clustering of log-transformed expression levels of the targeted genes of interest was performed. Agglomerative clustering was performed according to WardD2 method by calculating Manhattan distance between individual profiles using bio-informatic R-software scripts.
   B) Kaplan-Meier curve displaying the overall survival data of the computer-generated groups A and B of the heat-map in panel a). The results show that groups A and B have different survival with high significance (Fisher's exact test; p<0.0001), demonstrating that this test has high prognostic value in gliomas. Groups A and B are here annotated as IDH-MT and IDH-WT.
   C) heterozygous IDH1R132H detection in one of the samples, in this case with 38% of transcripts being from the mutant allele and 62% of transcripts from the wt allele
   D) Subgroup analysis of IDH-wild-type patients with very poor survival (OS<12 months) versus IDH-wild-type patients with better prognosis (OS>14 months) showed that high expression levels of carbonic anhydrase 12 are associated with poor prognosis (p<0.001; Fisher's exact test, see Kaplan-Meier curve in D).
**Fig. 11** Immunhistochemistry of tumors with high and low PSMA transcript levels. Blood vessel expression of PSMA protein is observed in blood vessels from tumors with high transcript levels and not in tumors with low transcript levels (see FPM values under the different photographs .

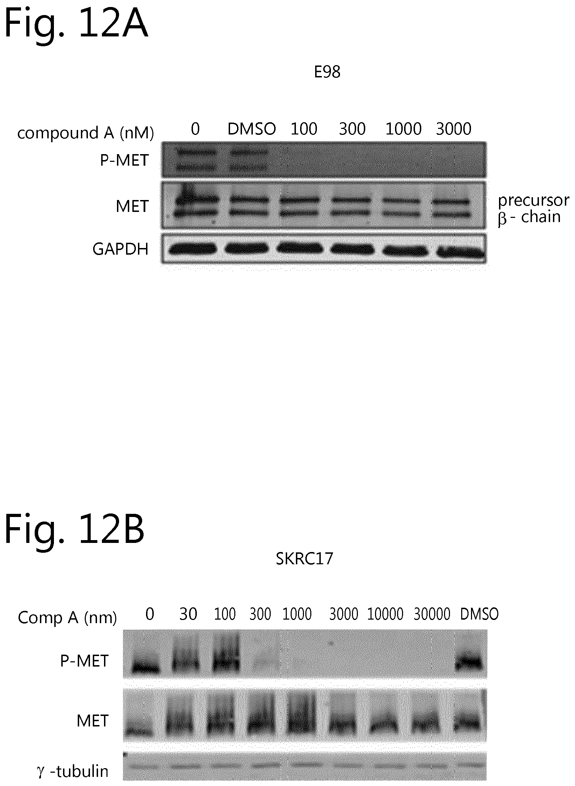

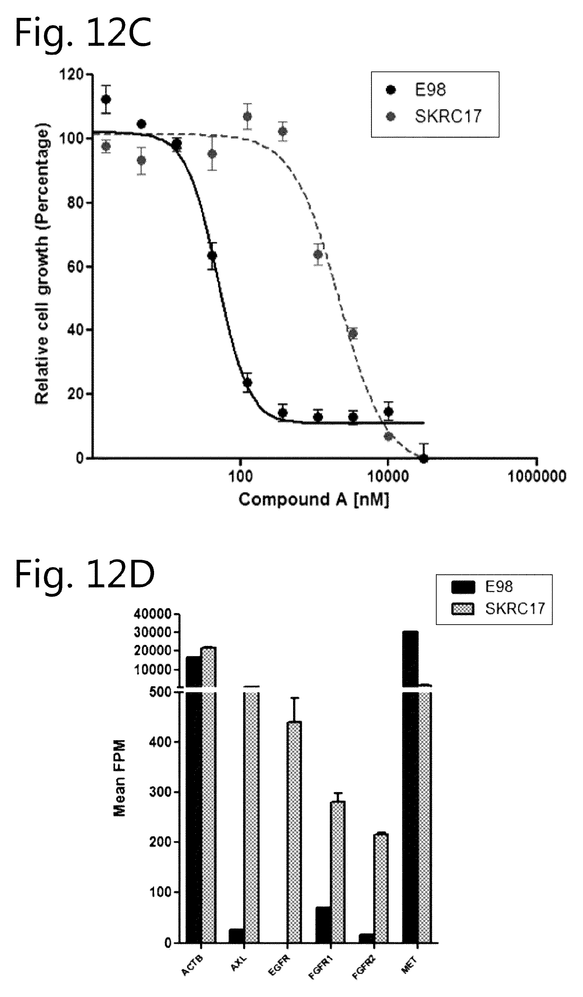

**Fig. 12** Tyrosine kinase profiles predict sensitivity and non-sensitivity to targeted therapies in vitro. A) the astrocytoma cell line E98 expresses similar levels of MET as the renal cancer cell line SKRC17 depicted in (B). C) However, in contrast to E98 cells, SKRC17 cells do not respond to compound A with decreased proliferation rates. D) Profiling of membrane tyrosine kinases reveals that within the selected group of membrane tyrosine kinases that are measured in the assay, MET is the only one expressed by E98, whereas SKRC17 cells express an additional number of other tyrosine kinase inhibitors, including AXL, EGFRs, FGFRs.

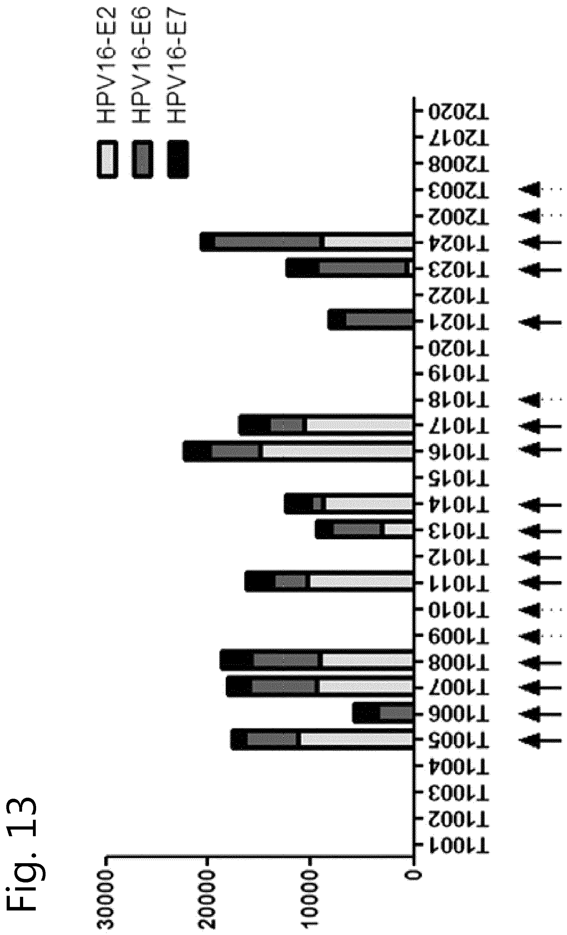

**Fig. 13** HPV RNA profiling. Profile of 29 gynecological tissues, ranging from normal uterus extirpations to ovarian cancer, endometrial cancers and cervix carcinomas. HPV16 E6/E7 RNA expression was observed in 12 samples. All HPV16-positive samples were confirmed on DNA level, but five tissues that were negative in the HPV-RNA test, were positive in the HPV-DNA test arrow heads.

### Detailed description of the invention

The present inventors have used multiplex profiling of RNA transcripts to determine which genes that are involved in metabolism are active, and which genes that are involved in pathologies are active. The inventors have found that from the combined information in the RNA profiles, the metabolic pathways that are most prominent in the pathological tissue can be deduced, and the genes that are actively involved in pathologies can be identified. This information can result in a personalized advice to treat an individual suffering from a disease with e.g. drugs that target the product of the gene that is aberrantly expressed and is involved in disease progression. These drugs (pharmaceutical compounds) include but are not limited to drugs that are approved by the United States Food and Drug Administration (FDA) and/or the European Medicines Agency (EMA) and are known as targeted drugs to the person skilled in the art. Such treatment advice can be combined with an advice to treat the disease further with a compound that inhibits the most essential metabolic pathways in the pathological tissue. This concept is known as synthetic lethality to the person skilled in the art. Added value of the test is generated by concomitant information on mutation status of metabolic genes.

The test requires a small aliquot of an RNA of interest that may be derived from solid tissue, isolated cells or bodily fluids, including, but not limited to, saliva, urine, sperm, blood, blood platelets and cerebrospinal fluid. The sample RNA can be converted to copy-DNA (cDNA) using a method known in the art, such as using oligo-dT primers or a mixture of random hexamer oligonucleotide primers. These techniques are standard techniques and are known to the person skilled in the art ((see e.g. Green and Sambrook (2012) Molecular Cloning: A Laboratory Manual, Fourth Edition, Cold Spring Harbor Laboratory Press, NY).

The RNAs of interest may be from human genes but may also be from genes of pathogens such as DNA viruses and RNA viruses, including but not limited to human immune deficiency virus (HIV); human papilloma viruses, including but not limited to the subtypes HPV16 and HPV18; hepatitis A virus; hepatitis B virus; hepatitis C virus; hepatitis E virus; Ebola virus; Epstein Bar Virus (EBV); influenza viruses; West-Nile virus, chikungunya virus, polyoma virus; cytomegalovirus; rhinovirus, but also genes from the category of oncolytic viruses that are known to persons skilled in the art to treat cancers. The RNA of interest may also be from genes of parasites, including but not limited to Plasmodium falciparum and Plasmodium vivax, parasites causing malaria, and trypanosoma. In addition, the RNA of interest may be from (pathogenic) fungi, including but not limited to Aspergillus. The RNA of interest may also be from (pathogenic) bacteria, such as Listeria, Legionella, Staphylococcus, Streptococcus, Mycobacterium and/or Yersinia.

The subject (interchangeably also referred to as patient) may be a human or an animal. Accordingly, the RNA of interest may also be from genes from domesticated, wild and farm animals and/or from genes that are present in pathogens such as the pathogens listed here above or their counterparts that cause disease in animals.

A set of single molecule molecular inversion probes (smMIPs) may be provided to detect the RNAs of interest that carry the information that is needed to formulate a treatment advice. A preferred set is selected from the group listed in Table II.

A preferred method of generating RNA profiles is by using smMIPs that can be designed with the published MIPGEN protocol (18) that selects optimal ligation and extension probe sequences that are predicted to hybridize against a cDNA of interest while leaving a gap between the ligation and extension parts of the probe. The ligation and extension parts of the probes may hybridize to any part of the cDNA, including sequences that are protein encoding and untranslated regions. Extension and ligation parts of the probes can be located in the same exon.

A preferred method is to locate the ligation and extension parts of the probes in different exons of a cDNA, which allows detection of specific splice variants.

A preferred method according to the invention is to contact a library of designed smMIPs, that may consist of any number of smMIPs, with a population of cDNA molecules. After an initial heating and denaturation step followed by cooling, each smMIP will hybridize to its target cDNA sequence. By incubating the mixture with a DNA polymerase enzyme, all four deoxynucleotides and DNA ligase in an appropriate buffer, the extension probe part of the MIP will be extended until the 5' end of the ligation probe is reached. The DNA ligase will then covalently link the 3' end of the extended extension probe part to the ligation probe part, producing a circular smMIP molecule.

In the next step, a method known to the person skilled in the art, is used to remove unreacted, linear smMIPs and cDNA from the reaction mixture by exonuclease treatment, leaving a purified library of circular smMIPs.

Using a forward and a reverse oligonucleotide primer that specifically anneal to the backbone sequence that connects the ligation and extension probes parts of the MIP, a PCR amplification of the gap sequence is performed. Preferably, one of the oligonucleotide primers that are used in this PCR is equipped with a barcode, allowing easy selection of all PCR products that are obtained from a specific sample. In a next step, the library of PCR amplicons are preferably analyzed on a next generation sequencing platform that yields FASTQ files containing information on nucleotide sequences of all PCR amplicons in the sample. Using an algorithm all PCR amplicons with the same barcode are grouped, producing a list of sequences for each individual cDNA sample.

Next, using another algorithm that uses the UMI, all identical PCR products will be considered to be derived from one originating smMIP. In this manner for each original RNA sample a list can be created that contains values that represent the original number of circularized smMIPs in the original library. This number is proportional to the number of cDNAs in the original sample.

In a preferred method of interpretation, the values obtained for each individual smMIP are divided by the summated values of all smMIPs for each sample, followed by multiplying with a factor of one million, thus yielding a fragments per million value for each smMIP.

In a preferred method of interpretation, the mean FPM values of all different smMIPs that correspond to one transcript, are considered to be proportional to the number of transcripts that were present in the initial RNA sample of the analysis.

In another preferred method of interpretation, mean FPM values of individual transcripts are divided by mean FPM values of so-called house-keeping genes, to yield a relative abundance value of a transcript of interest.

In another preferred method, mean FPM values for transcripts from genes that are involved in metabolic pathways are used to deduce the predominant metabolic pathways in a tissue.

A preferred method to analyze the FASTQ files further is to detect mutations in the next generation sequencing data. Preferably, mutations are considered as relevant if they are detected in more than two reads. The sequence information as provided in the FASTQ files should not be so narrowly construed as to require inclusion of erroneously identified bases. The skilled person is capable of identifying such erroneously identified bases and knows how to correct for such errors. A list of relevant mutations in a sample can be included in a database, preferably a standard query language (SQL)-based database that allows statistical analyses, for example by multivariate analysis.

A preferred method of analysis of the database results in a list of metabolic pathways that are active in a tissue or in a person with a disease and that can be used to give a dietary advice to relieve the symptoms of the disease and to improve the efficacy of other therapies.

Another preferred method of analysis of the database results in a list of aberrancies that can be treated with available pharmacological drugs.

Yet another preferred method of the invention uses a software algorithm that translates RNA profiles of diseased tissues directly to a treatment advice that can be given via an application that can be installed on a personal computer or a mobile device.

The method according to the invention can be readily implemented in routine patient care in case RNA from diseased tissue or blood platelets is available.

Accordingly, in a first aspect, the present invention provides for a method for *in vitro* determination of the susceptibility and/or resistance of a subject suffering from or at risk of a disease or condition for a drug to treat the disease or condition, comprising:
- performing RNA profiling on an *ex vivo* sample obtained from the subject,
- determining the susceptibility and/or resistance of the subject suffering from or at risk of a disease or condition for a drug to treat the disease or condition,

wherein the presence of an alternative splice variant is an indication for the susceptibility and/or resistance,
wherein the RNA profiling is performed by multiplex mRNA sequencing, targeting multiple regions of interest using molecular inversion probes (MIPs),
wherein the sample RNA is converted to cDNA and wherein the MIPs of interest hybridize to the cDNA via an extension probe and a ligation probe that are connected by a backbone sequence, leaving a 112nt gap on the cDNA upon hybridization, and
wherein the MIPs further comprise a detectable moiety, preferably a unique identifier sequence of random nucleotides adjacent to the ligation part of the MIP or to the extension part of the MIP sequence..

Said method is herein referred to as the method according to the invention. "RNA profiling" is herein also referred to as targeted RNA sequencing of transcripts.

In a method according to the invention, RNA profiling is performed by multiplex mRNA sequencing, targeting multiple regions of interest. The sample RNA of interest is first converted to copy-DNA (cDNA) using a method known in the art, such as using oligo-dT primers or a mixture of random hexamer oligonucleotide primers. The RNA of interest may be from human genes but may also be from genes of pathogens such as DNA viruses and RNA viruses, including but not limited to human immune deficiency virus (HIV); human papilloma viruses, including but not limited to the subtypes HPV16 and HPV18; hepatitis A virus; hepatitis B virus; hepatitis C virus; hepatitis E virus; Ebola virus; Epstein Bar Virus (EBV); influenza viruses; West-Nile virus, chikungunya virus, polyoma virus; cytomegalovirus; rhinovirus, but also genes from the category of oncolytic viruses that are known to persons skilled in the art to treat cancers. The RNA of interest may also be from genes of parasites, including but not limited to Plasmodium falciparum and Plasmodium vivax, parasites causing malaria, and trypanosoma. In addition, the RNA of interest may be from (pathogenic) fungi, including but not limited to Aspergillus. The RNA of interest may also be from (pathogenic) bacteria, such as Listeria, Legionella, Staphylococcus, Streptococcus, Mycobacterium and/or Yersinia.

In a method according to the invention, the multiplex mRNA sequencing is performed using molecular inversion probes (MIPs), comprising a detectable moiety, preferably a unique identifier sequence of a string of 3 to 10 random nucleotides (depicted as "N" in a sequence listing), more preferably a string of 3, more preferably 4, more preferably 5, more preferably 6, more preferably 7, most preferably 8, or preferably more than 8 random nucleotides (N) adjacent to the ligation part of the MIP or to the extension part of the MIP sequence (smMIPs).

In a method according to the invention, an alternative splice variant is linked to a an aberrance in a metabolic pathway which is in turn linked to the susceptibility and/or resistance of a subject suffering from or at risk of a disease or condition for a drug. In all embodiments of the invention, a drug is as meant in the art, a pharmaceutical compound. Such pharmaceutical compound may be comprised in a pharmaceutical composition. In all embodiments of the invention, a subject is a human or an animal, preferably a human. An animal may be any animal, preferably a domestic, wild or farm animals.

Preferably, in a method according to the invention, the disease or condition is at least one selected from the group consisting of a cancer, a viral infection, a bacterial infection, an autoimmune disease and a genetic disease.

In a method according to the invention, the sample may be any appropriate sample known to the person skilled in the art, preferably selected from the group consisting of a tissue, a tumor tissue, urine, sperm, saliva, blood, blood plasma, cerebrospinal fluid, blood platelets, and/or exosomes, more preferably selected from tumor tissue and blood platelets.

In a method according to the invention, the metabolic pathway is preferably selected from the group consisting of a glucose processing pathway, a glutamine processing pathway and/or a fatty acid pathway.

Preferably, in a method according to the invention, the multiple regions of interest are within the mRNA of- glucose processing genes, glutamine processing genes, fatty acid anabolism genes, transporter genes, redox homeostasis genes, genes with potential involvement in cancer, such as oncogenes, genes involved in angiogenesis, genes involved in immune suppression, and viral genes.

Preferably, in a method according to the invention, the multiple regions of interest are within the mRNA of at least one, two, three, four, five or at least six genes selected from the group consisting of: ABAT, ACACA, ACACB, ACLY, ACO2, ACSS2, ADPGK, ALDOA, ARHGAP26, ATG4A. ATP5A1, CBR1, CBS, CHKA, CKB, CPT1A, CYCS, EGLN1, ENO1, G6PC, GAD1, GCLC, GCLM, GFPT1, GLDC, GSS, HK1, HK2, HK3, GLY1, G6PD, RGN , PGD, RPIA, RPE, TKT, PGI, ALDOA, GAPDH, PGAM1/2, ENO, PKM1/2, PDHA1, PDK1, PFKB1, PFKMb, PGAM1, PGD, PGK1, PKM, PRDX1, PRKAA1, RPIA, PC, CS, ACO1, IDH1, IDH2, IDH3A, IDH3B, IDH3G, OGDH, SUCLA2, SDHA/B/C/D, FH, MDH1, MDH2, PDK, LDHA, LDHB, SLC16A1, SLC16A3, CA9, CA12, SLC4A10, VHL, SDH, SDHAF2, HPGL/PCC, FH, CS, D-2HGDH, L-2HGDH, FH, IDH1-3A-G, MDH1-2, MYC, OGDH, SDHA-D, VHL, PHD, HIF1a, EPAS2 PDCD1, SLC1A5, ASCT2, GLS, GLUD1/2, GOT, GPI, GS, BCAT1, BCAT2, SLC1A2, SLC7A11, SLC25A1, ACLY, ACACA, ACACB, FASN, CPT1, SLC5A7, CHKA, CPT2, VLCAD, HADHA/B, SCAD, MCAD, LCAD, SCHA-D, 2-Enoyl-VoA hydratase, MCKAT, SLC16A1, SLC16A7, SLC2A1, SLC2A3, SLC5A1, SLC5A5, SLC7A1, SLC9A1, SLCA12, redox homeostasis genes: NAMPT, NAPRT1, NOX1, NOX3, NOX4A, NQO1, SOD, SOD2, CAT, TAL, TIGAR, TRX, PARP1, ALK, AXL, BRAF, KRAS, TP53, MAPK8, MYC, TP53I3, FGFR1, FGFR2, IGF1-R, KDR, NTRK1, NTRK2, PDGFRA, PDGFRB, EGFR, EGFRvIII, ERBB2, ERBB3, ERBB4, MERTK, PLXND1, RET, Androgen receptor (AR), AR variant 7, AR variant 12, FOLH1, KLK3, MET, METdelta14, METdelat7-8, KIT, RON PTEN, VEGF-A121, VEGF-A144, VEGF-A165, VEGF-A189, CD274, CTLA4, HPV-E2, HPV-E6, and HPV-E7.

Preferably, in a method according to the invention, the multiple regions of interest are within the mRNA of:
- glucose processing genes, such as, but not limited to: ABAT, ACACA, ACACB, ACLY, ACO2, ACSS2, ADPGK, ALDOA, ARHGAP26, ATG4A. ATP5A1, CBR1, CBS, CHKA, CKB, CPT1A, CYCS, EGLN1, ENO1, G6PC, GAD1, GCLC, GCLM, GFPT1, GLDC, GSS, HK1, HK2, HK3, GLY1, G6PD, Gluconolactonase , PGD, RPIA, RPE, TKT, PGI, ALDOA, GAPDH, PGAM1/2, ENO, PKM1/2, PDHA1, PDK1, PFKB1, PFKMb, PGAM1, PGD, PGK1, PKM, PRDX1, PRKAA1, RPIA, PC, CS, ACO1, IDH1, IDH2, IDH3A, IDH3B, IDH3G, OGDH, SUCLA2, SDHA/B/C/D, FH, MDH1, MDH2, PDK, LDHA, LDHB, SLC16A1, SLC16A3, CA9, CA12, SLC4A10, VHL, SDH, SDHAF2, HPGL/PCC, FH, CS, D-2HGDH, L-2HGDH, FH, IDH1-3A-G, MDH1-2, MYC, OGDH, SDHA-D, VHL, PHD, HIF1a, EPAS2 and/or PDCD1;
- glutamine processing genes, such as, but not limited to: SLC1A5, ASCT2, GLS, GLUD1/2, GOT, GPI, GS, BCAT1, BCAT2, SLC1A2 and/or SLC7A11;
- fatty acid anabolism genes, such as, but not limited to: SLC25A1, ACLY, ACACA, ACACB, FASN, CPT1, SLC5A7, CHKA, CPT2, VLCAD, HADHA/B, SCAD, MCAD, LCAD, SCHA-D, 2-Enoyl-VoA hydratase and/or MCKAT;
- transporter genes, such as, but not limited to; SLC16A1, SLC16A7, SLC2A1, SLC2A3, SLC5A1, SLC5A5, SLC7A1, SLC9A1 and/or SLCA12;
- redox homeostasis genes, such as, but not limited to: NAMPT, NAPRT1, NOX1, NOX3, NOX4A, NQO1, SOD, SOD2, CAT, TAL, TIGAR and/or TRX;
- DNA repair genes, such as, but not limited to: PARP1;
- genes with potential involvement in cancer, such as, but not limited to: ALK, AXL, BRAF, KRAS, HRAS, NRAS, GNAQ, GNA11, TP53, MAPK8, MYC, TP53I3, FGFR1, FGFR2, IGF1-R, KDR, NTRK1, NTRK2, PDGFRA, PDGFRB, EGFR, EGFRvlll, ERBB2, ERBB3, ERBB4, MERTK, PLXND1, RET, Androgen receptor (AR), AR variant 7, AR variant 12, FOLH1, KLK3, MET, METdelta14, METdelat7-8, KIT, RON and/or PTEN;
- genes involved in angiogenesis, such as, but not limited to: VEGF-A121, VEGF-A144, VEGF-A165 and/or VEGF-A189
- genes involved in immune suppression, such as, but not limited to: CD274 and/or CTLA4; and/or,
- viral genes, such as, but not limited to: HPV-E2, HPV-E6 and/or HPV-E7.

Preferably, in a method according to the invention, the presence of an alternative splice variant also provides an indication for treatment with dietary compounds or phytochemicals, optionally in combination with a drug. The person skilled in the art knows that drug treatment can beneficially be combined with treatment with dietary compounds or phytochemicals.

The method according to the invention can conveniently be used for guiding treatment in a subject (personalized medicine). Further disclosed but not part of the invention is a method of treatment of a subject suffering from or at risk of a disease or condition, comprising:
- requesting performance or performing a method according to the invention, thus determining the susceptibility and/or resistance of the subject suffering from or at risk of a disease or condition for a drug to treat the disease or condition, and
- treating the disease or condition of the subject with a drug where the disease or condition of the subject is susceptible to. In this method, all features are preferably those of the first aspect.

Preferably, in a method of treatment, the disease or condition is at least one selected from the group consisting of: a cancer, including but not limited to glioma, meningioma, ependymoma, pilocytic astrocytoma, adenocarcinomas, sarcomas, hemangioma, head and neck cancer, breast cancer, lung cancer, prostate cancer, kidney cancer, ovarian cancer, endometrial cancer, cervical cancer, colon cancer, rectal cancer, pancreatic cancer, esophagus cancer, basal cell cancer, penile cancer, vulva cancer, melanoma, uveal melanoma, lymphoma, acute myeloid leukemia, acute lymphoblastic leukemia, cholangiocarcinoma, hepatocellular carcinoma, soft tissue sarcoma, and osteosarcoma; a viral infection; a bacterial infection; an autoimmune disease and a genetic disease.

Preferably, in a method of treatment, the drug treatment is supplemented with treatment with dietary compounds or phytochemicals.

Further disclosed but not part of the invention is a medicament (drug) for use in the treatment of a subject suffering from or at risk of a disease or condition, wherein:
- a method according to the invention is performed or requested to be performed, thus determining the susceptibility and/or resistance of the subject suffering from or at risk of a disease or condition for a drug to treat the disease or condition, and
- administrating to a subject suffering from or at risk of a disease or condition with a drug where the disease or condition of the subject is susceptible to.

Preferably, in the medicament (drug) for use, the disease or condition is at least one selected from the group consisting of a cancer, a viral infection, a bacterial infection, an autoimmune disease and a genetic disease.

Preferably, in the medicament (drug) for use, the drug treatment is supplemented with treatment with dietary compounds or phytochemicals.

Further disclosed but not part of the invention is a method for the production of a medicament (drug) for the treatment of a subject suffering from or at risk of a disease or condition, comprising:
- requesting performance or performing a method according to the invention, thus determining the susceptibility and/or resistance of the subject suffering from or at risk of a disease or condition for a drug to treat the disease or condition, and
- treating the disease or condition of the subject with a drug where the disease or condition of the subject is susceptible to.

Preferably, in the method for the production of a medicament (drug) for the treatment, the disease or condition is at least one selected from the group consisting of a cancer, a viral infection, a bacterial infection, an autoimmune disease and a genetic disease.

Preferably, in the method for the production of a medicament (drug) for the treatment, the drug treatment is supplemented with treatment with dietary compounds or phytochemicals.

Further disclosed but not part of the invention is a molecular inversion probe selected from the group as set forward in Table II. Further disclosed but not part of the invention is a set of molecular inversion probes of at least two, three, four, five, six or more selected from the group as set forward in Table II.

Further disclosed but not part of the invention is a library of circularized molecular inversion probes obtainable by a method according to the first or second aspect of the invention.

### Definitions

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The word "about" or "approximately" when used in association with a numerical value (e.g. about 10) preferably means that the value may be the given value (of 10) more or less 5% of the value. The sequence information as provided herein should not be so narrowly construed as to require inclusion of erroneously identified bases. The skilled person is capable of identifying such erroneously identified bases and knows how to correct for such errors. In case of sequence errors, the sequence of the polypeptides obtainable by expression of the genes present in SEQ ID NO: 1 containing the nucleic acid sequences coding for the polypeptides should prevail.

**Table II Description of the sequences**

| **Seq ID NO:** | **Seq name** | **Sequence** |
|---|---|---|
| 1 | ABAT_0817 | CGTTGAATTTGATTATGATGGGCCTCTGNNNNNNNNCTTCAGCTT CCCGATATCCGACGGTAGTGTTGTCCCTCAAGGGGTCA |
| 2 | ABAT_0820 | CAACAGACCCGCCCTCGGAATCNNNNNNNNCTTCAGCTTCCCGAT ATCCGACGGTAGTGTTTACCTGGTTGATGTGGACGGC |
| 3 | ABAT_0823 | CCTCTCCTTCATGGGCGCGTTCCATGGNNNNNNNNCTTCAGCTTC CCGATATCCGACGGTAGTGTAAAACGCCTTAAAGACCA |
| 4 | ABAT_0827 | GCCTTCTTGGTGGACGAGGTCCAGACCGGNNNNNNNNCTTCAGCT TCCCGATATCCGACGGTAGTGTGGAAAAAGAAGAAGAC |
| 5 | ABAT_0831 | GATTCCATACGGAATAAGCTCATTTTAATNNNNNNNNCTTCAGCT TCCCGATATCCGACGGTAGTGTATAATGCAGCCCATGC |
| 6 | ACACA_3334 | GCTCATTTTGGAGGAATAATGGATGANNNNNNNNCTTCAGCTTCC CGATATCCGACGGTAGTGTAGAGGCCCAAATTGAGG |
| 7 | ACACA_3360 | GCTGGGAAGTTAATCCAGTACATTGTAGANNNNNNNNCTTCAGCT TCCCGATATCCGACGGTAGTGTATGATGGCAGCAGTTA |
| 8 | ACACA_3375 | CTCCTCCAACCTCAACCACNNNNNNNNCTTCAGCTTCCCGATATC CGACGGTAGTGTTATTGCCTATGAACTTAACAGCGTAC |
| 9 | ACACA 3390 | GCAATGACATCACATACCGAATTGGGNNNNNNNNCTTCAGCTTCC CGATATCCGACGGTAGTGTGGATGATCAAGGTCAGCTG |
| 10 | ACACA_3408 | GCGCTGGTTTGTGGAAGTGGAAGGAACAGNNNNNNNNCTTCAGCT TCCCGATATCCGACGGTAGTGTAAAACATCCCGTACCT |
| 11 | ACACB_0664 | TCCCACCAGAAGCCCCCAANNNNNNNNCTTCAGCTTCCCGATATC CGACGGTAGTGTTCTGATAACTCAGGGGAGACACCGCA |
| 12 | ACACB_0681 | GCAGGGACAGTGGAATACCTCTATANNNNNNNNCTTCAGCTTCCC GATATCCGACGGTAGTGTTGCTCCATCCAGCGGCGGCA |
| 13 | ACACB_0698 | CCCAGAGCATCGTGCAGTTGGTCCAGANNNNNNNNCTTCAGCTTC CCGATATCCGACGGTAGTGTCAGTATGCCAGCAACATC |
| 14 | ACACB_0714 | CCACTGTCATCATGGACCCCTTCAAGATCNNNNNNNNCTTCAGCT TCCCGATATCCGACGGTAGTGTCTTCGAATACCTGCAG |
| 15 | ACACB_0730 | GCAGGCAGGACAGGTGTGGTTNNNNNNNNCTTCAGCTTCCCGATA TCCGACGGTAGTGTAGACCGTGGTGACAGGACGA |
| 16 | ACLY_1628 | ACCACCTCAGCCATCCAGNNNNNNNNCTTCAGCTTCCCGATATCC GACGGTAGTGTTTCGTCGGGCCTGTGGAAGAAGCGCCG |
| 17 | ACLY_1636 | GCTGACCTTGCTGAACCCCAAAGGGANNNNNNNNCTTCAGCTTCC CGATATCCGACGGTAGTGTACTGCCGACTACATCTGCA |
| 18 | ACLY_1644 | GCTCCCGAGACGAGCCCTCAGTGGCTNNNNNNNNCTTCAGCTTCC CGATATCCGACGGTAGTGTAAAGAAGGCCAAGCCT |
| 19 | ACLY_1652 | GCCAAGAACCAGGCTTTGAAGGAAGNNNNNNNNCTTCAGCTTCCC GATATCCGACGGTAGTGTAAGGAGGGCCGCCTCACTAA |
| 20 | ACLY_1660 | GCTCGATTATGCACTGGAAGTAGAGAAGNNNNNNNNCTTCAGCTT CCCGATATCCGACGGTAGTGTGATGAAGAAGGAAGGGA |
| 21 | ACO2_0767 | CCTGGATGACCCCGCCAGCCAGGAANNNNNNNNCTTCAGCTTCCC GATATCCGACGGTAGTGTGGTGGCGATGAGCCACTTTG |
| 22 | ACO2_0773 | CGGTGAAAGGTGGCACAGGTGCAANNNNNNNNCTTCAGCTTCCCG ATATCCGACGGTAGTGTGTGGATGTCATGGCTGGG |
| 23 | ACO2_0777 | GCTGCACCAATTCAAGCTATGAAGATATGNNNNNNNNCTTCAGCT TCCCGATATCCGACGGTAGTGTTGAGCTGAAGCCACAC |
| 24 | ACO2_0783 | GCAAGGACCTGGAGGACCTGCAGATNNNNNNNNCTTCAGCTTCCC GATATCCGACGGTAGTGTAAGGGGAGTTTGACCCAGGG |
| 25 | ACO2_0787 | CGAGACCAACCTGAAGAAACAGGGCNNNNNNNNCTTCAGCTTCCC GATATCCGACGGTAGTGTTGGCATCAGGTGGGTGGTG |
| 26 | ACSS2_1192 | GCAATGAGCCAGGGGAGACCACTCAGATCNNNNNNNNCTTCAGCT TCCCGATATCCGACGGTAGTGTGACTAAAGGGAAAATC |
| 27 | ACSS2_1196 | GCTGCATTGTGGTCAAGCACCTNNNNNNNNCTTCAGCTTCCCGAT ATCCGACGGTAGTGTTCTTGGATTCCAGCTGCAGTCTT |
| 28 | ACSS2_1202 | AGCCTGTCACCAAGCATAGCCGNNNNNNNNCTTCAGCTTCCCGAT ATCCGACGGTAGTGTGTGTTTTGTTTGAGGGGATTCCC |
| 29 | ACSS2_1206 | CGCTTTGAGACAACCTACNNNNNNNNCTTCAGCTTCCCGATATCC GACGGTAGTGTTTTCCCATTCTTTGGTGTAGCTCCTGC |
| 30 | ACSS2_1210 | CTTGCCTAAAACCCGCTCAGNNNNNNNNCTTCAGCTTCCCGATAT CCGACGGTAGTGTTGCCTCTACTGCTTTGTCACCTTGT |
| 31 | ALDOA_0076 | CACTGGGAGCATTGCCAAGCGGCTNNNNNNNNCTTCAGCTTCCCG ATATCCGACGGTAGTGTAACTTGCTACTACCAGCACCA |
| 32 | ALDOA_0080 | GCCATCATGGAAAATGCCAATGTTCTGGCNNNNNNNNCTTCAGCT TCCCGATATCCGACGGTAGTGTAGACTACCACCCAAGG |
| 33 | ALDOA_0082 | GCTCTGAGTGACCACCACATCTACNNNNNNNNCTTCAGCTTCCCG ATATCCGACGGTAGTGTTTATGCCAGTATCTGCCAGCA |
| 34 | ALDOA_0084 | GAGGCGTCCATCAACCTCAATGCCATTNNNNNNNNCTTCAGCTTC CCGATATCCGACGGTAGTGTCCATGCTTGCACTCAGAA |
| 35 | ALDOA_0086 | GCCTGTCAAGGAAAGTACACTCCGAGCGNNNNNNNNCTTCAGCTT CCCGATATCCGACGGTAGTGTGCCCTGACCTTCTCCT |
| 36 | ARHGAP26_2921 | GTGCATAGGAGATGCAGAAACAGATGATGNNNNNNNNCTTCAGCT TCCCGATATCCGACGGTAGTGTGACAAGACCAACAAAT |
| 37 | ARHGAP26_2925 | GTTTGTGGAGCCTCTGCTGGCCTTNNNNNNNNCTTCAGCTTCCCG ATATCCGACGGTAGTGTCCAAAAAGAAAGAATCTCAGC |
| 38 | ARHGAP26_2931 | GTGAAGGGACTGCGCAGTTGGACAGCATTNNNNNNNNCTTCAGCT TCCCGATATCCGACGGTAGTGTGGAAGCAGTAGACAGG |
| 39 | ARHGAP26_2939 | CAGCATCCTTAATTCCAGCAGCNNNNNNNNCTTCAGCTTCCCGAT ATCCGACGGTAGTGTTGACTCCAAGCCCCCGTCCTGCA |
| 40 | ARHGAP26_2944 | GTTCACAGCAGGCACGGTCTTCGATAACGNNNNNNNNCTTCAGCT TCCCGATATCCGACGGTAGTGTCATCCAAACCTGCACT |
| 41 | ATG4A_3103 | GCTGGTATGGATCTTAGGGAAGCAGNNNNNNNNCTTCAGCTTCCC GATATCCGACGGTAGTGTCGTAGTCAAGTTGCCGGTGG |
| 42 | ATG4A_3107 | CAGTTGCACAGGTGTTAAAAAAACTTGCNNNNNNNNCTTCAGCTT CCCGATATCCGACGGTAGTGTAAGAATACCAACGCATC |
| 43 | ATG4A_3110 | GTGTTTTAAGATGCCACAGTCTTTAGGGGNNNNNNNNCTTCAGCT TCCCGATATCCGACGGTAGTGTGCTTCAAACCAGAGTA |
| 44 | ATG4A_3112 | TCCATTGCCTGCAGTCCCCACAGCGAATGNNNNNNNNCTTCAGCT TCCCGATATCCGACGGTAGTGTAAAACCAAATAACGCG |
| 45 | ATG4A_3114 | GCCAAGCCAGAAGTGACAACCACTGGGNNNNNNNNCTTCAGCTTC CCGATATCCGACGGTAGTGTCAAAGAAGAAAAAGACT |
| 46 | ATP5A1_1339 | GCCCGCGTACATGGGCTGAGGAATGTTNNNNNNNNCTTCAGCTTC CCGATATCCGACGGTAGTGTCACTCATCTTCAAAAGAC |
| 47 | ATP5A1_1342 | GAGTTGGTCTGAAAGCCCCCGGTATCATTNNNNNNNNCTTCAGCT TCCCGATATCCGACGGTAGTGTAGTGAAGAGGACAGGA |
| 48 | ATP5A1_1347 | ATGTCTCTGTTGCTCCGCCGACCNNNNNNNNCTTCAGCTTCCCGA TATCCGACGGTAGTGTATGCTGCCCCACTTCAGTACCT |
| 49 | ATP5A1_1350 | GCTGCCCAAACCAGGGCTATGAAGCAGGTNNNNNNNNCTTCAGCT TCCCGATATCCGACGGTAGTGTCTTACATTCCAACAAA |
| 50 | ATP5A1_1353 | GCCTTGTTGGGCACTATCAGGGCTGATNNNNNNNNCTTCAGCTTC CCGATATCCGACGGTAGTGTGGCTATTGAAGAACAAGT |
| 51 | ATP5C1_0551 | GCTGAGAGAGAGCTGAAACCAGCTCNNNNNNNNCTTCAGCTTCCC GATATCCGACGGTAGTGTCCGCAATGGATTCAAGTTCG |
| 52 | ATP5C1_0553 | GTTATGCTTGTTGGAATTGGTGACAANNNNNNNNCTTCAGCTTCC CGATATCCGACGGTAGTGTAAGACAAGAAGAAACACC |
| 53 | ATP5C1_0555 | AAATTCAGGTCTGTCATCTCCNNNNNNNNCTTCAGCTTCCCGATA TCCGACGGTAGTGTTTCTGACCAGTTTCTGGTGGCATT |
| 54 | ATP5C1_0557 | GCCAGGATGACAGCCATGGACAATGCCNNNNNNNNCTTCAGCTTC CCGATATCCGACGGTAGTGTAAGTGCTGACAGCATGAG |
| 55 | ATP5C1_0559 | GTTCCATCCTCAGACAAGAGGTAAAGAAGNNNNNNNNCTTCAGCT TCCCGATATCCGACGGTAGTGTGAATGCTTCTGAGATG |
| 56 | BCAT1_0990 | TAGTCACACCAGCTACCANNNNNNNNCTTCAGCTTCCCGATATCC GACGGTAGTGTCCTTTTTTGTGTTTGCCTGGGTCCTGG |
| 57 | BCAT1_0993 | GCTGTGAGGGCAACTCTGCCGGTATTTNNNNNNNNCTTCAGCTTC CCGATATCCGACGGTAGTGTCCCTGGCTCATCAGCTTT |
| 58 | BCAT1_0996 | GTGGAACTGGGGACTGCAAGATGGGAGGGNNNNNNNNCTTCAGCT TCCCGATATCCGACGGTAGTGTCAAGAAGCCTACCAAA |
| 59 | BCAT1_0998 | GCATCATTCTTCCAGGAGTGACANNNNNNNNCTTCAGCTTCCCGA TATCCGACGGTAGTGTTGGGTGTCAGCAGGTCCTGTG |
| 60 | BCAT1_1000 | GCGAGACAATACACATTCCAACTATGGAGNNNNNNNNCTTCAGCT TCCCGATATCCGACGGTAGTGTGTCAGAGAGATACCTC |
| 61 | BCAT2_1494 | GCCCAGTGGGTGCCTACTTNNNNNNNNCTTCAGCTTCCCGATATC CGACGGTAGTGTTCATCGAAGTGGACAAGGACTGGGTC |
| 62 | BCAT2_1497 | GCCTGCCGAGTTTCGACAAGCTGGAGTTNNNNNNNNCTTCAGCTT CCCGATATCCGACGGTAGTGTGCCTCCACTACTCCCT |
| 63 | BCAT2_1499 | GTGGGAATTATGGGCCCACCGTNNNNNNNNCTTCAGCTTCCCGAT ATCCGACGGTAGTGTCGCTCCTGTTCGTCATTCTCT |
| 64 | BCAT2_1501 | GCCTGGAGTGGTCAGACAGAGTCTANNNNNNNNCTTCAGCTTCCC GATATCCGACGGTAGTGTGGTCCTCTGGCTGTATGGG |
| 65 | BCAT2_1503 | CCTGTACAAAGACAGGAACCTCCNNNNNNNNCTTCAGCTTCCCGA TATCCGACGGTAGTGTTGGGGTGAGTTCCGGGTGGTGG |
| 66 | CA12_3467 | CCTGATGGGGAGAATAGCTGGTCCAAGANNNNNNNNCTTCAGCTT CCCGATATCCGACGGTAGTGTCAGGAGCCCGCGAAGA |
| 67 | CA12_3470 | ACCCGCACGGCTCTGAGCACANNNNNNNNCTTCAGCTTCCCGATA TCCGACGGTAGTGTAACAAGCAGTTTCTCCTGACCAAC |
| 68 | CA12_3472 | CACCTTCAACATGTAAAGTACAAAGGCNNNNNNNNCTTCAGCTTC CCGATATCCGACGGTAGTGTCCATTATAACTCAGACCT |
| 69 | CA12_3474 | GCTGCTGGCTTTGGAGACAGNNNNNNNNCTTCAGCTTCCCGATAT CCGACGGTAGTGTAACATTGAAGAGCTGCTTCCGGAGA |
| 70 | CA12_3476 | GTGGTGGTGTCCATTTGGCTTTTNNNNNNNNCTTCAGCTTCCCGA TATCCGACGGTAGTGTCCCCCAGAGAAATGATCAACAA |
| 71 | CA9_1143 | GCCCAGTGAAGAGGATTCACCCAGAGAGGNNNNNNNNCTTCAGCT TCCCGATATCCGACGGTAGTGTCTGCTGCTGTCACTGC |
| 72 | CA9_1145 | GAGGCTCCTGGAGATCCTCAAGAACCNNNNNNNNCTTCAGCTTCC CGATATCCGACGGTAGTGTAGGATCCACCCGGAGAGGA |
| 73 | CA9_1148 | CCCTCTGACTTCAGCCGCTANNNNNNNNCTTCAGCTTCCCGATAT CCGACGGTAGTGTTGTTGGCCGCCTTTCTGGAGGAGGG |
| 74 | CA9_1150 | GCGACGCAGCCTTTGAATGGGCGAGTGANNNNNNNNCTTCAGCTT CCCGATATCCGACGGTAGTGTTGTGCCCAGGGTGTCA |
| 75 | CA9_1152 | GCAGATGAGAAGGCAGCACAGAAGGGGANNNNNNNNCTTCAGCTT CCCGATATCCGACGGTAGTGTTGGAGTGGACAGCAGT |
| 76 | CBR1_1512 | GTTGCTGATCCCACACCCTTTCATATNNNNNNNNCTTCAGCTTCC CGATATCCGACGGTAGTGTTGAGCCCGCGCTTCCACCA |
| 77 | CBR1_1515 | CCCCAAGCATCCTGCGTACTNNNNNNNNCTTCAGCTTCCCGATAT CCGACGGTAGTGTTGGTCAACAACGCGGGCATCGCCTT |
| 78 | CBR1_1516 | ATCTGCCGCTGCTTAACTCTGGGCCNNNNNNNNCTTCAGCTTCCC GATATCCGACGGTAGTGTTGCACAGAATTACTCCCTCT |
| 79 | CBR1_1517 | GTCTTTGGTTGTAAACTGCTGTGATAGTTNNNNNNNNCTTCAGCT TCCCGATATCCGACGGTAGTGTAGGAGGAAAGTCCAAG |
| 80 | CBS_0094 | CCCTGTGGATCCGGCCCGATGCTNNNNNNNNCTTCAGCTTCCCGA TATCCGACGGTAGTGTTCCCAGCATGCCTTCTGAGACC |
| 81 | CBS_0099 | GCTCTTGGCCAAGTGTGAGTTCTTNNNNNNNNCTTCAGCTTCCCG ATATCCGACGGTAGTGTAGTCCCCACATCACCACACT |
| 82 | CBS_0102 | CGGAGTCACACGTGGGGGTNNNNNNNNCTTCAGCTTCCCGATATC CGACGGTAGTGTTGGCTGCGGCAGTGAGGGGCTAT |
| 83 | CBS_0107 | GCAAGAGGGGCTGCTGTGCGGTGGCAGTGNNNNNNNNCTTCAGCT TCCCGATATCCGACGGTAGTGTACCTACGAGGTGGAAG |
| 84 | CBS_0112 | GCTCTCGCACATCCTGGAGATGGANNNNNNNNCTTCAGCTTCCCG ATATCCGACGGTAGTGTTGGGAATGGTGACGCTTGGG |
| 85 | CHKA_3492 | ACACCACAGCCACCCTTGGTGATGANNNNNNNNCTTCAGCTTCCC GATATCCGACGGTAGTGTGCAGGGCCTATCTGTGGTGC |
| 86 | CHKA_3494 | GCCGGCGATTAGATACTGAAGAATTAAGNNNNNNNNCTTCAGCTT CCCGATATCCGACGGTAGTGTTGCAGATGGGGGCTGAG |
| 87 | CHKA_3496 | GCTCAGTTACAATCTGCCCTTGGAACTGGNNNNNNNNCTTCAGCT TCCCGATATCCGACGGTAGTGTGGTATGAAAATGCCAT |
| 88 | CHKA_3499 | CCAGTTACTTGCCTGCATTCCAAAATGANNNNNNNNCTTCAGCTT CCCGATATCCGACGGTAGTGTGGGGATTCGACATTGG |
| 89 | CHKA_3501 | GCAAGGTTTGATGCCTATTTCCACCAGANNNNNNNNCTTCAGCTT CCCGATATCCGACGGTAGTGTAAAGAAGAAATGTTGC |
| 90 | CKB_1938 | CCACCTGCGGGTCATCTCCATNNNNNNNNCTTCAGCTTCCCGATA TCCGACGGTAGTGTACCACTTCCTCTTCGACAAGCCC |
| 91 | CKB_1940 | GCGACGACCTGGACCCCAACTACGTGCTGNNNNNNNNCTTCAGCT TCCCGATATCCGACGGTAGTGTCGACGAGGAGTCCTAC |
| 92 | CKB_1945 | GGACTATGAGTTCATGTGGAACCCTCANNNNNNNNCTTCAGCTTC CCGATATCCGACGGTAGTGTTGGTGTGGGTCAACGAGG |
| 93 | CKB_1947 | GCACAGGCGGTGTGGACACGGCTNNNNNNNNCTTCAGCTTCCCGA TATCCGACGGTAGTGTCTACATCCTCACCTGCCCAT |
| 94 | CKB_1948 | GTGGTGGACGGAGTGAAGCTGCTNNNNNNNNCTTCAGCTTCCCGA TATCCGACGGTAGTGTAGGTGCTTAAGCGGCTGCGAC |
| 95 | CPT1A_0611 | GGACTTCATTCCTGGAAAAAGAAGTTCNNNNNNNNCTTCAGCTTC CCGATATCCGACGGTAGTGTGCTGACTCGGTACTCTCT |
| 96 | CPT1A_0615 | GATCTGGATGGGTATGGTCAAGATCTTTTNNNNNNNNCTTCAGCT TCCCGATATCCGACGGTAGTGTGTGCTGTTTGGCACCG |
| 97 | CPT1A_0621 | GCACATGAGAGACAGCAAGCACATCGNNNNNNNNCTTCAGCTTCC CGATATCCGACGGTAGTGTAAACTGGACCGGGAGGAAA |
| 98 | CPT1A_0629 | GCTGGCGCACTACAAGGACATGGGCAAGTNNNNNNNNCTTCAGCT TCCCGATATCCGACGGTAGTGTAACACCGCAAATCTTC |
| 99 | CPT1A_0633 | GTTTGACTTGGAGAATAACCCAGAGTACGNNNNNNNNCTTCAGCT TCCCGATATCCGACGGTAGTGTCACCTCTTCTGCCTTT |
| 100 | CYCS_3031 | GGTCTCTTTGGGCGGAAGACAGGTCAGNNNNNNNNCTTCAGCTTC CCGATATCCGACGGTAGTGTGCGACTAAAAAGAGAAT |
| 101 | CYCS_3032 | GGGAGAGGATACACTGATGGAGTATTTGGNNNNNNNNCTTCAGCT TCCCGATATCCGACGGTAGTGTACCGTTGAAAAGGGAG |
| 102 | CYCS_3033 | GGAAGAAAGGGCAGACTTAATAGCTTATCNNNNNNNNCTTCAGCT TCCCGATATCCGACGGTAGTGTTACACAGCCGCCAATA |
| 103 | CYCS_3034 | CTTTTTTATGTGTACCATCCTTTAATAGNNNNNNNNCTTCAGCTT CCCGATATCCGACGGTAGTGTTGATCTTTGTCGGCATT |
| 104 | EGLN1_3069 | CGGGCAGCTGGTCAGCCAGAAGAGTGNNNNNNNNCTTCAGCTTCC CGATATCCGACGGTAGTGTGAGTACATCGTGCCGTG |
| 105 | EGLN1_3075 | GGAACGGGTTATGTACGTCATGTTGNNNNNNNNCTTCAGCTTCCC GATATCCGACGGTAGTGTGGCTGCGAAACCATTGGG |
| 106 | EGLN1_3077 | GATAGACTGCTGTTTTTCTGGTCTGNNNNNNNNCTTCAGCTTCCC GATATCCGACGGTAGTGTGGAGATGGAAGATGTGTG |
| 107 | EGLN1_3079 | GGTCGGTAAAGACGTCTTCTAGAGCCNNNNNNNNCTTCAGCTTCC CGATATCCGACGGTAGTGTGCATATGCTACAAGGTACG |
| 108 | EGLN1_3080 | GTGAATACGAATAAATGGGATAAAGANNNNNNNNCTTCAGCTTCC CGATATCCGACGGTAGTGTGGTGAAAAAGGTGTGAGGG |
| 109 | ENO1_1724 | GCTGTGCCCAGTGGTGCTTCAACTGGTANNNNNNNNCTTCAGCTT CCCGATATCCGACGGTAGTGTAGACCCAGTGGCTAGAA |
| 110 | ENO1_1728 | GCGGTTCTCATGCTGGCAANNNNNNNNCTTCAGCTTCCCGATATC CGACGGTAGTGTTTCTGGGGGTGTCCCTTGCCGTCTG |
| 111 | ENO1_1732 | GCCTGACCAGCTGGCTGACCTGTACAANNNNNNNNCTTCAGCTTC CCGATATCCGACGGTAGTGTTTGGGAAAGCTGGCTACA |
| 112 | ENO1_1735 | GCCAATGGTTGGGGCGTCATGGTGTCTCNNNNNNNNCTTCAGCTT CCCGATATCCGACGGTAGTGTACAGTGACCAACCCAAA |
| 113 | ENO1_1737 | CGGCAGGAACTTCAGAAACCCCTTGNNNNNNNNCTTCAGCTTCCC GATATCCGACGGTAGTGTTGACCTGGTTGTGGGGCTGT |
| 114 | FASN_2387 | CCCAGCCCCCACCCACAANNNNNNNNCTTCAGCTTCCCGATATCC GACGGTAGTGTTCCACAGCCTGGCTGCCTACTACATC |
| 115 | FASN_2394 | CGTGGAGCAGCTGAGGAAGGAGGGTGTNNNNNNNNCTTCAGCTTC CCGATATCCGACGGTAGTGTTGGCAGCCGTGGGCTT |
| 116 | FASN_2423 | GCCATCCAGATAGGCCTCATAGACNNNNNNNNCTTCAGCTTCCCG ATATCCGACGGTAGTGTCTTCCGAGATTCCATCCTAC |
| 117 | FASN_2438 | GCGTTCTTCAACGAGAGCAGTGCTGANNNNNNNNCTTCAGCTTCC CGATATCCGACGGTAGTGTGCGTGAGGTGCTTGGCT |
| 118 | FASN_2445 | GTGCTGGCTGAGAAGGCTGNNNNNNNNCTTCAGCTTCCCGATATC CGACGGTAGTGTGTGGGCATTTTGGTGGAGACGAT |
| 119 | FASN_2447 | GGGCCTAGAGGAGCGTGTGGCAGCNNNNNNNNCTTCAGCTTCCCG ATATCCGACGGTAGTGTAGAGCTACCGGGCAAAG |
| 120 | G6PC_0139 | GCTGTGGGCATTAAACTCCTTTGGGTAGCNNNNNNNNCTTCAGCT TCCCGATATCCGACGGTAGTGTCAACACATTACCTCCA |
| 121 | G6PC_0142 | GCCGACCTACAGATTTCGGTGCTTGNNNNNNNNCTTCAGCTTCCC GATATCCGACGGTAGTGTGATAAAGCAGTTCCCTGTA |
| 122 | G6PC_0144 | GCATCTATAATGCCAGCCNNNNNNNNCTTCAGCTTCCCGATATCC GACGGTAGTGTTGGTTGGGATTCTGGGCTGTGCAGCTG |
| 123 | G6PC_0146 | CACCCTTTGCCAGCCTCCTNNNNNNNNCTTCAGCTTCCCGATATC CGACGGTAGTGTTTACCTTCTTCCTGTTCAGCTTCGCC |
| 124 | G6PC_0148 | CGTCTTGTCCTTCTGCAAGAGTGCGGTNNNNNNNNCTTCAGCTTC CCGATATCCGACGGTAGTGTAGAGCTGCAAGGGGAAA |
| 125 | G6PD_0394 | CAGAGTGAGCCCTTCTTCAAGGCCACCCCNNNNNNNNCTTCAGCT TCCCGATATCCGACGGTAGTGTACCTGGCCAAGAAGAA |
| 126 | G6PD_0397 | ACCTGCAGAGCTCTGACCGGCTGTCNNNNNNNNCTTCAGCTTCCC GATATCCGACGGTAGTGTCCAACCGCCTCTTCTACCT |
| 127 | G6PD_0401 | GTACGTGGGGAACCCCGATGGAGANNNNNNNNCTTCAGCTTCCCG ATATCCGACGGTAGTGTTGCAGATGCTGTGTCTGGTGG |
| 128 | G6PD_0405 | GACGTCTTCTGCGGGAGCCAGATGCANNNNNNNNCTTCAGCTTCC CGATATCCGACGGTAGTGTACACCAAGATGATGACCAA |
| 129 | G6PD_0407 | CCAGTATGAGGGCACCTACAAGTGNNNNNNNNCTTCAGCTTCCCG ATATCCGACGGTAGTGTGTGAGGCCTGGCGTATTTTC |
| 130 | GAD1_0451 | GAAGAGTCGCCTTGTGAGTGCCTTCAANNNNNNNNCTTCAGCTTC CCGATATCCGACGGTAGTGTACCCCAATACCACTAACC |
| 131 | GAD1_0455 | GCACAGGTCATCCTCGATTTTTCAACCANNNNNNNNCTTCAGCTT CCCGATATCCGACGGTAGTGTACTTTCATCACCCACAC |
| 132 | GAD1_0459 | GATAAAGTGCAATGAAAGGGGGAAAATANNNNNNNNCTTCAGCTT CCCGATATCCGACGGTAGTGTGGAAGTTAAGACAAAGG |
| 133 | GAD1_0463 | GATGTCTCCTACGACACCGGGGACAAGGNNNNNNNNCTTCAGCTT CCCGATATCCGACGGTAGTGTAACCCTCACAAGATGA |
| 134 | GAD1_0467 | TTCCGGATGGTCATCTCCAACCCAGCCNNNNNNNNCTTCAGCTTC CCGATATCCGACGGTAGTGTTGTGCCAGACAGCCCTCA |
| 135 | GAPDH_1973 | CCCCTTCATTGACCTCAACTACATGGTTTNNNNNNNNCTTCAGCT TCCCGATATCCGACGGTAGTGTCCACATCGCTCAGACA |
| 136 | GAPDH_1975 | GCTGGCGCTGAGTACGTCGTGGAGTNNNNNNNNCTTCAGCTTCCC GATATCCGACGGTAGTGTCAATATGATTCCACCCATGG |
| 137 | GAPDH_1978 | CCATCACTGCCACCCAGAAGACNNNNNNNNCTTCAGCTTCCCGAT ATCCGACGGTAGTGTATGAGAAGTATGACAACAGCCTC |
| 138 | GAPDH_1980 | GCCAACGTGTCAGTGGTGGACCTNNNNNNNNCTTCAGCTTCCCGA TATCCGACGGTAGTGTTGTGGCGTGATGGCCGCGGGG |
| 139 | GAPDH_1982 | GCATTGCCCTCAACGACCACTTTGTCAANNNNNNNNCTTCAGCTT CCCGATATCCGACGGTAGTGTAGAAGGTGGTGAAGCAG |
| 140 | GCLC_1788 | GAAAATAAAAAAGTCCGGTTGGTCCTGTCNNNNNNNNCTTCAGCT TCCCGATATCCGACGGTAGTGTACCACGTGCGGCGGCA |
| 141 | GCLC_1792 | CCTCGCTTCAGTACCTTAACNNNNNNNNCTTCAGCTTCCCGATAT CCGACGGTAGTGTCTCTTTGCACAATAACTTCATTTCC |
| 142 | GCLC_1796 | GATCAGTAAATCCCGATATGACTCAATAGNNNNNNNNCTTCAGCT TCCCGATATCCGACGGTAGTGTTCTCCCTTTTACCGAG |
| 143 | GCLC_1800 | CCTACAAATTGGATTTTCTCATTCCACTGNNNNNNNNCTTCAGCT TCCCGATATCCGACGGTAGTGTCCCTCCTCCAAACTCA |
| 144 | GCLC_1804 | GAACTAATGACAGTTGCCAGATGGATGNNNNNNNNCTTCAGCTTC CCGATATCCGACGGTAGTGTGGAAGGTGTGTTTCCTGG |
| 145 | GCLM_1678 | GAAATGAAAGTTTCTGCAAAACTGTTCATNNNNNNNNCTTCAGCT TCCCGATATCCGACGGTAGTGTTGCTGAACTGGGGCCG |
| 146 | GCLM_1680 | GCAAAAAGATTGTTGCCATAGGTACCNNNNNNNNCTTCAGCTTCC CGATATCCGACGGTAGTGTTTCAGTCCTTGGAGTTGCA |
| 147 | GCLM_1682 | GCTTTCTGAAGCAAGTTTCCAAGAAGCNNNNNNNNCTTCAGCTTC CCGATATCCGACGGTAGTGTCACAGGTAAAACCAAATA |
| 148 | GCLM_1683 | GCTACTGCGGTATTCGGTCATTGTGAAANNNNNNNNCTTCAGCTT CCCGATATCCGACGGTAGTGTACAATTTGACATACAGC |
| 149 | GCLM_1684 | CTTACCTGTAATTTCCTTCAATATGAGAGNNNNNNNNCTTCAGCT TCCCGATATCCGACGGTAGTGTGAGTGGGTGCCGCTGT |
| 150 | GFPT1_1220 | GCACTGGATGAAGAAGTTCACAAGCNNNNNNNNCTTCAGCTTCCC GATATCCGACGGTAGTGTGGCCTTCAGAGACTGGAGTA |
| 151 | GFPT1_1224 | GCCCTCTGTTGATTGGTGTACGGAGTGNNNNNNNNCTTCAGCTTC CCGATATCCGACGGTAGTGTGAAAGTCAAGATACCA |
| 152 | GFPT1_1228 | CACTCCAGATGGAACTCCAGCAGATCANNNNNNNNCTTCAGCTTC CCGATATCCGACGGTAGTGTATAGAACACACCAATCGC |
| 153 | GFPT1_1234 | GTTTGCCCTTATGATGTGTGATGATCGGNNNNNNNNCTTCAGCTT CCCGATATCCGACGGTAGTGTCACAAACACAGTTGGCA |
| 154 | GFPT1_1238 | GCTCTTCAGCAAGTGGTTGCTCGGNNNNNNNNCTTCAGCTTCCCG ATATCCGACGGTAGTGTTACTTATATGCACTCTGAAGG |
| 155 | GLDC_0162 | GACGGTCCCTGCCAACATCCGTTTGAAAANNNNNNNNCTTCAGCT TCCCGATATCCGACGGTAGTGTCTGGAGCGCCTTCTGC |
| 156 | GLDC_0168 | CAGACACGGAGGGGAAGGTGGAAGACTTNNNNNNNNCTTCAGCTT CCCGATATCCGACGGTAGTGTACCCACAGACAATAGC |
| 157 | GLDC_0177 | GCATGATTCCACTGGGATCCTGCACCANNNNNNNNCTTCAGCTTC CCGATATCCGACGGTAGTGTGGGTCTGTGTTCAAGAGG |
| 158 | GLDC_0183 | GCCCTGGAGACTTCGGGTCTGATGTCTNNNNNNNNCTTCAGCTTC CCGATATCCGACGGTAGTGTACATACCCATCCACCAA |
| 159 | GLDC_0189 | ACTGAGTCGGAGGACAAGGCAGAGCTGGNNNNNNNNCTTCAGCTT CCCGATATCCGACGGTAGTGTACGAGACCCTTCAAAAA |
| 160 | GLS_1282 | GCTGAAGGACAAGAGAAAATACCTGNNNNNNNNCTTCAGCTTCCC GATATCCGACGGTAGTGTAAGGACGGCCCCGGGGAGA |
| 161 | GLS_1285 | GGTTGCAGATTATATTCCTCAACTGGCCANNNNNNNNCTTCAGCT TCCCGATATCCGACGGTAGTGTCAGAGCAACATTGTTT |
| 162 | GLS_1288 | GCTGGAGCAATTGTTGTGACTTCACNNNNNNNNCTTCAGCTTCCC GATATCCGACGGTAGTGTGCCATTGCTGTTAATGATCT |
| 163 | GLS_1292 | GCAGTTCGAAATACATTGAGTTTGATGCANNNNNNNNCTTCAGCT TCCCGATATCCGACGGTAGTGTTAGACTTCTACTTCCA |
| 164 | GLS_1295 | GAAGGTGGTGATCAAAGGCATTCCTTTGGNNNNNNNNCTTCAGCT TCCCGATATCCGACGGTAGTGTCTCTGGATAAGATGGG |
| 165 | GLUD1_2495 | GCGGCATCCTGCGGATCATCAAGCCCTGNNNNNNNNCTTCAGCTT CCCGATATCCGACGGTAGTGTACGACCCCAACTTCTT |
| 166 | GLUD1_2501 | GTGAGCGGGAGATGTCCTGGATCGCTGATNNNNNNNNCTTCAGCT TCCCGATATCCGACGGTAGTGTCAATCCCAAGAACTAT |
| 167 | GLUD1_2504 | GCTAAATGTATTGCTGTTGGTGAGTCTNNNNNNNNCTTCAGCTTC CCGATATCCGACGGTAGTGTCATCAATGAAGCTTCTTA |
| 168 | GLUD1_2507 | GCTGACAAGATCTTCCTGGAGAGAAACANNNNNNNNCTTCAGCTT CCCGATATCCGACGGTAGTGTTTGGAGGCCGACTGTG |
| 169 | GLUD1_2510 | GCACTCTGGCTTGGCATACACAATGGAGCNNNNNNNNCTTCAGCT TCCCGATATCCGACGGTAGTGTTTGCTCATGTCTGTTC |
| 170 | GLUD2_2853 | GCGAGGAGCAGAAGCGGAACCGGGTNNNNNNNNCTTCAGCTTCCC GATATCCGACGGTAGTGTACTACAGCGAGTTGGTGG |
| 171 | GLUD2_2856 | ACCGAAAATGAATTGGAAAAGATCACAAGNNNNNNNNCTTCAGCT TCCCGATATCCGACGGTAGTGTGCACTGATGTGAGTGT |
| 172 | GLUD2_2859 | GCATTTTAGGAATGACACCAGGGTTTAGNNNNNNNNCTTCAGCTT CCCGATATCCGACGGTAGTGTATGCACACGCCTGTGTT |
| 173 | GLUD2_2862 | TCGACTGTGACATACTGATCCCAGCTNNNNNNNNCTTCAGCTTCC CGATATCCGACGGTAGTGTGTCTGATGGGAGTATATGG |
| 174 | GLUD2_2867 | GCCAGGCAAATTATGCACACAGCCATGAANNNNNNNNCTTCAGCT TCCCGATATCCGACGGTAGTGTAATTTGGAAAGCATGG |
| 175 | GOT_1990 | GACCCCCGCAAGGTCAACCTGGGAGNNNNNNNNCTTCAGCTTCCC GATATCCGACGGTAGTGTGGGCGTGGGGTGAAAT |
| 176 | GOT_1993 | CAAACAACAAGAACACACCNNNNNNNNCTTCAGCTTCCCGATATC CGACGGTAGTGTGTGCTTCTCGTCTTGCCCTTGGGG |
| 177 | GOT_1996 | GACTCAGCCTATCAGGGCTTCGCATCNNNNNNNNCTTCAGCTTCC CGATATCCGACGGTAGTGTCTCCTGAGTTCTCCATTG |
| 178 | GOT _1998 | CCTGCAAGTCCTTTCCCAGNNNNNNNNCTTCAGCTTCCCGATATC CGACGGTAGTGTTGCCTGGGCCATTCGCTATTTTGTGT |
| 179 | GOT_2000 | AGCCCTCAAAACCCCTGGGACCTGGAANNNNNNNNCTTCAGCTTC CCGATATCCGACGGTAGTGTAGGGAGCACGAATTGTGG |
| 180 | GPI_1522 | GCTTTGACCAGTGGGGAGTGGAGCTGGGANNNNNNNNCTTCAGCT TCCCGATATCCGACGGTAGTGTTGAAGGAAATCGCCCA |
| 181 | GPI_1523 | GCTCATCAACTTCATCAAGCAGCAGNNNNNNNNCTTCAGCTTCCC GATATCCGACGGTAGTGTCATCATCTGGGACATCAACA |
| 182 | GPI_1524 | GTGCTCATCTGCAGCCTCCTCTGTNNNNNNNNCTTCAGCTTCCCG ATATCCGACGGTAGTGTAAGCAGCTGGCTAAGAAAATA |
| 183 | GPT_2527 | GGAGCTGCGCCAGGGTGTGAAGAANNNNNNNNCTTCAGCTTCCCG ATATCCGACGGTAGTGTGGAGCCAGGCGGTGAG |
| 184 | GPT_2528 | GCATGGACTGAGGGCGAAGGTGCTGACGNNNNNNNNCTTCAGCTT CCCGATATCCGACGGTAGTGTCACTAAGCCAGACCCA |
| 185 | GPT_2536 | GGGCAGAGGCCCATCACCTTNNNNNNNNCTTCAGCTTCCCGATAT CCGACGGTAGTGTGGAGAGTGGAGTACGCAGTGCGTGG |
| 186 | GPT_2537 | CGATGCCAAGAAAAGGGCGGAGNNNNNNNNCTTCAGCTTCCCGAT ATCCGACGGTAGTGTTTTCACCGAGGTCATCCGTGCC |
| 187 | GPT_2540 | GCTGGGTCGCCCTGGACTGTGTNNNNNNNNCTTCAGCTTCCCGAT ATCCGACGGTAGTGTAAGGCACCTACCACTTCC |
| 188 | GS_0645 | TGGAGAAGGACTGCGCTGCAAGACCCGNNNNNNNNCTTCAGCTTC CCGATATCCGACGGTAGTGTAACGAACACCTTCCACCA |
| 189 | GS_0648 | ACATGGTGAGCAACCAGCACCCNNNNNNNNCTTCAGCTTCCCGAT ATCCGACGGTAGTGTTCGTGCCTGCTGCCATGTTTCGG |
| 190 | GS_0650 | GCTTGTATGCTGGAGTCAAGATTGCGGNNNNNNNNCTTCAGCTTC CCGATATCCGACGGTAGTGTACAGATGGGCACCCCTTT |
| 191 | GS_0653 | CGAGGAGGCCATTGAGAAACTAAGCNNNNNNNNCTTCAGCTTCCC GATATCCGACGGTAGTGTGTGTGAAGACTTTGGAGTG |
| 192 | GS_0656 | CCTCATCCGCACGTGTCTTCTCNNNNNNNNCTTCAGCTTCCCGAT ATCCGACGGTAGTGTCTTTTCTGCTGGTGTAGCCAATC |
| 193 | GSS_0206 | GCTGTCAGCCAGAACGCTGCCTNNNNNNNNCTTCAGCTTCCCGAT ATCCGACGGTAGTGTACCTCACAGGAGCCCACTTCCT |
| 194 | GSS_0208 | GCAGCGCAGATGGCTCCCCAGCCCTGAAANNNNNNNNCTTCAGCT TCCCGATATCCGACGGTAGTGTCAGCACCATCAAACAG |
| 195 | GSS_0212 | GCTGTTTGTGGATGGCCAGGAAATTGNNNNNNNNCTTCAGCTTCC CGATATCCGACGGTAGTGTGAGAAGGAAAGAAACATAT |
| 196 | GSS_0215 | GATGTGGGTGAAGAAGGGGACCAGGCCATNNNNNNNNCTTCAGCT TCCCGATATCCGACGGTAGTGTTGGCTGGGACTAAGAA |
| 197 | GSS_0218 | GCAGGAAAAGACACTCGTGATGAACAANNNNNNNNCTTCAGCTTC CCGATATCCGACGGTAGTGTCCTCCTACATCCTCAT |
| 198 | HIF1A_1815 | GTTTTTTATGAGCTTGCTCATCAGTTGCCNNNNNNNNCTTCAGCT TCCCGATATCCGACGGTAGTGTGCGGGGACCGATTCAC |
| 199 | HIF1A_1821 | GCTTGGTGCTGATTTGTGAACCCATTNNNNNNNNCTTCAGCTTCC CGATATCCGACGGTAGTGTCCGAGGAAGAACTATGAAC |
| 200 | HIF1A_1827 | GATGCTTTAACTTTGCTGGCCCCAGCNNNNNNNNCTTCAGCTTCC CGATATCCGACGGTAGTGTCCCTTCAACAAACAGAATG |
| 201 | HIF1A_1833 | CACCATTAGAAAGCAGTTCCGCAAGCCCTNNNNNNNNCTTCAGCT TCCCGATATCCGACGGTAGTGTTGCTGAAGACACAGAA |
| 202 | HIF1A_1839 | GCAGCTACTACATCACTTTCTTGGAAACGNNNNNNNNCTTCAGCT TCCCGATATCCGACGGTAGTGTAAGAACTAAATCCAAA |
| 203 | HIF2A_1750 | GCCTCCATCATGCGACTGGCAATCAGCTTNNNNNNNNCTTCAGCT TCCCGATATCCGACGGTAGTGTAGAGGAGGAAGGAGAA |
| 204 | HIF2A-1754 | CACGGTCACCAACAGAGGCCNNNNNNNNCTTCAGCTTCCCGATAT CCGACGGTAGTGTATCTTTGACTTCACTCATCCCTGCG |
| 205 | HIF2A_1760 | GGACCAGACTGAATCCCTGTTCNNNNNNNNCTTCAGCTTCCCGAT ATCCGACGGTAGTGTTGGGGGCTACGTGTGGCTGG |
| 206 | HIF2A_1768 | GTCTGCAAAGGGTTTTGGGGCTCGAGGNNNNNNNNCTTCAGCTTC CCGATATCCGACGGTAGTGTCAGATCCACCATTACA |
| 207 | HIF2A_1772 | TTCCCCCCACAGTGCTACGCCANNNNNNNNCTTCAGCTTCCCGAT ATCCGACGGTAGTGTCTGAGCGCAAATGTACCCAATG |
| 208 | HK1_0224 | TGGCCTCTCCCGGGATTTTAATCCAACNNNNNNNNCTTCAGCTTC CCGATATCCGACGGTAGTGTCCTATTACTTCACGGAGC |
| 209 | HK1_0230 | GCACATTGATCTGGTGGAAGGAGACGNNNNNNNNCTTCAGCTTCC CGATATCCGACGGTAGTGTAACATCGTAGCTGTGGTGA |
| 210 | HK1_0236 | GCGCTTCCTCCTCTCGGAGAGTGGCAGNNNNNNNNCTTCAGCTTC CCGATATCCGACGGTAGTGTATAACAAGGGCACACCCA |
| 211 | HK1_0242 | GCGGGAATCTTGATCACGTGGACAANNNNNNNNCTTCAGCTTCCC GATATCCGACGGTAGTGTGGGGAAGAGCTGTTTGATCA |
| 212 | HK1_0248 | GCTATCCTCCAGCAGCTAGGTCTGAANNNNNNNNCTTCAGCTTCC CGATATCCGACGGTAGTGTACTTCACCAAGAAGGGATT |
| 213 | HK2_0268 | CTACCACATGCGCCTCTCTGATNNNNNNNNCTTCAGCTTCCCGAT ATCCGACGGTAGTGTTCTCGCGTCTCCGCCTCGGTTTC |
| 214 | HK2_0274 | GTTGGGACCATGATGACCTGTGGTTATGNNNNNNNNCTTCAGCTT CCCGATATCCGACGGTAGTGTTCATGGACCAAGGGAT |
| 215 | HK2_0283 | GCTGGTCCGTGTTCGGAATGGGAAGTGGGNNNNNNNNCTTCAGCT TCCCGATATCCGACGGTAGTGTAAGGAGACTCATGCCA |
| 216 | HK2_0291 | GTCTCAGATTGAGAGTGACTGCCTGGCNNNNNNNNCTTCAGCTTC CCGATATCCGACGGTAGTGTGTGGAATGTACCTGGGTG |
| 217 | HK2_0295 | GCGGCGCTCATCACTGCTGTGGNNNNNNNNCTTCAGCTTCCCGAT ATCCGACGGTAGTGTGTGGGTGTGGATGGGACCCTCTA |
| 218 | HK3_2013 | CGTCTGTGCGGCCGTGTGNNNNNNNNCTTCAGCTTCCCGATATCC GACGGTAGTGTTGAGCCAAGGCAGCATCCTCCTG |
| 219 | HK3_2028 | CTCTTTCCCTTGTCACCAGACGGNNNNNNNNCTTCAGCTTCCCGA TATCCGACGGTAGTGTTTTGTGATCCCCCAAGAGGTG |
| 220 | HK3_2032 | CGGAGGCCTGTACCTGGGTGAGCTNNNNNNNNCTTCAGCTTCCCG ATATCCGACGGTAGTGTTCTGCGTCAGCGTCGAGT |
| 221 | HK3_2041 | GGCCTCATTGTCGGAACCGGCANNNNNNNNCTTCAGCTTCCCGAT ATCCGACGGTAGTGTGATGTCGTGAGTCTGTTGCGGG |
| 222 | HK3_2045 | GAGATCGAAAGTGACAGCCTGGCCCTGCNNNNNNNNCTTCAGCTT CCCGATATCCGACGGTAGTGTATGTACCTGGGGGAGA |
| 223 | Housekeeping_A CTB_0800 | GCCCAGAGCAAGAGAGGCATCCTNNNNNNNNCTTCAGCTTCCCGA TATCCGACGGTAGTGTGCATGTGCAAGGCCGGCTT |
| 224 | Housekeeping_A CTB_0802 | GAAGATGACCCAGATCATGTTTGAGACCTNNNNNNNNCTTCAGCT TCCCGATATCCGACGGTAGTGTACCAACTGGGACGACA |
| 225 | Housekeeping_A CTB_0805 | GCTACGTCGCCCTGGACTTCGAGCAAGANNNNNNNNCTTCAGCTT CCCGATATCCGACGGTAGTGTTGCGTCTGGACCTGGCT |
| 226 | Housekeeping_A CTB_0808 | ACCACCATGTACCCTGGCATTGCCGACANNNNNNNNCTTCAGCTT CCCGATATCCGACGGTAGTGTTTCCAGCCTTCCTTCCT |
| 227 | Housekeeping_A CTB_0810 | GTGGATCAGCAAGCAGGAGTATGACGANNNNNNNNCTTCAGCTTC CCGATATCCGACGGTAGTGTAGAAGGAGATCACTGCC |
| 228 | Housekeeping_T UBB_1551 | GCTCAGGTCCTTTTGGCCAGATCTTNNNNNNNNCTTCAGCTTCCC GATATCCGACGGTAGTGTGAGGCGAGCAAAAAAATTAA |
| 229 | Housekeeping_T UBB_1554 | GCCTTCACCCAAAGTGTCTGACACNNNNNNNNCTTCAGCTTCCCG ATATCCGACGGTAGTGTACTGCCTGCAGGGCTTCCAGC |
| 230 | Housekeeping_T UBB_1557 | GTCCCCTTCCCACGTCTCCATTTCTTTATNNNNNNNNCTTCAGCT TCCCGATATCCGACGGTAGTGTTGACCACACCAACCTA |
| 231 | Housekeeping_T UBB_1559 | GTGGTCGGATGTCCATGAAGGAGGTCGATNNNNNNNNCTTCAGCT TCCCGATATCCGACGGTAGTGTAAGCCAGCAGTATCGA |
| 232 | Housekeeping_T UBB_1563 | GCCGAAGAGGAGGCCTAAGGCAGAGNNNNNNNNCTTCAGCTTCCC GATATCCGACGGTAGTGTTACACAGGCGAGGGCATGGA |
| 233 | IDH3A_2545 | GCCATTCAAGGACCTGGAGGAAAGTGGANNNNNNNNCTTCAGCTT CCCGATATCCGACGGTAGTGTGGTGGTGTTCAGACAGT |
| 234 | IDH3A_2546 | TAGCAGCCGGTCACCCATCTATGAANNNNNNNNCTTCAGCTTCCC GATATCCGACGGTAGTGTTTCAGTGGGAGGAGCGG |
| 235 | IDH3A_2547 | CCCCTTACACCGATGTAAATATTGTGNNNNNNNNCTTCAGCTTCC CGATATCCGACGGTAGTGTGGGCTTGAAAGGCCCTTTG |
| 236 | IDH3A_2548 | CGTGCAGAGTATCAAGCTCATCACCGAGNNNNNNNNCTTCAGCTT CCCGATATCCGACGGTAGTGTTGTCCGACCATGTGTCT |
| 237 | IDH3A_2549 | CGGAGCAACGTCACGGCGGTGCACANNNNNNNNCTTCAGCTTCCC GATATCCGACGGTAGTGTGAAGGAGAATACAGTGG |
| 238 | IDH3A_2550 | GAGATGTACCTTGATACAGTATGNNNNNNNNCTTCAGCTTCCCGA TATCCGACGGTAGTGTATGCCCGGAACAACCAC |
| 239 | IDH3A_2551 | GTGACTTGTGTGCAGGATTGATCGGAGNNNNNNNNCTTCAGCTTC CCGATATCCGACGGTAGTGTGCAGAAAGCTGTAAAGAT |
| 240 | IDH3A_2552 | GTCGGTTCATGGGACGGCTCCNNNNNNNNCTTCAGCTTCCCGATA TCCGACGGTAGTGTCCCAATTTGATGTTCTTGTTATGC |
| 241 | IDH3A_2553 | GATGCTGCGCCACATGGGACTTTTTGACCNNNNNNNNCTTCAGCT TCCCGATATCCGACGGTAGTGTACACCAAGTGGCAACA |
| 242 | IDH3A_2554 | GCAAAATGCTCAGACTTCACAGAGGANNNNNNNNCTTCAGCTTCC CGATATCCGACGGTAGTGTTCCTGCTCAGTGCCGTGAT |
| 243 | IDH3A_2555 | TCTACAACTGGCATTTACATCAGTCACNNNNNNNNCTTCAGCTTC CCGATATCCGACGGTAGTGTGAGGCTGCGTGTTTTG |
| 244 | IDH3B_2791 | GCTGAGTTCCATGAAGGAGAACAANNNNNNNNCTTCAGCTTCCCG ATATCCGACGGTAGTGTGTGTGGGGCCTGAGCTGATG |
| 245 | IDH3B_2792 | GCGGCTGAGGCGTAAGTTGGACTTATTTNNNNNNNNCTTCAGCTT CCCGATATCCGACGGTAGTGTGAATATGGCATCTGAGG |
| 246 | IDH3B_2793 | GTGATCATTCGAGAGCAGACAGAAGGGNNNNNNNNCTTCAGCTTC CCGATATCCGACGGTAGTGTGGAGTATAAGGGGGAG |
| 247 | IDH3B_2794 | GCGGATTGCAAAGTTCGCCTTTGACTATGNNNNNNNNCTTCAGCT TCCCGATATCCGACGGTAGTGTCACAACAATCTAGACC |
| 248 | IDH3B_2795 | GAAACTTGGGGATGGGTTGTTCCTNNNNNNNNCTTCAGCTTCCCG ATATCCGACGGTAGTGTGGGGTGTGATTGAGTGTTTG |
| 249 | IDH3B_2796 | GTGCAGAATCCTTACCAGTTTGATGTGCTNNNNNNNNCTTCAGCT TCCCGATATCCGACGGTAGTGTACAAGGCCAACATCAT |
| 250 | IDH3B_2797 | GCTGGTGTGGTCCCTGGTGAGAGCTNNNNNNNNCTTCAGCTTCCC GATATCCGACGGTAGTGTGAGACAATGATCATAGACAA |
| 251 | IDH3B_2798 | GCCATGCTGCTGTCGGCTTCNNNNNNNNCTTCAGCTTCCCGATAT CCGACGGTAGTGTTGGCTGCTGGCCTGGTTGGGGG |
| 252 | IDH3B_2799 | GCAAGGTGCGGACTCGAGACATGGNNNNNNNNCTTCAGCTTCCCG ATATCCGACGGTAGTGTGCAGTGGGCAGGAATATAG |
| 253 | IDH3B_2800 | GCCCTTTATTTCTTCCAACCTTGCAAGGANNNNNNNNCTTCAGCT TCCCGATATCCGACGGTAGTGTAGATGCGGTGAAGAAG |
| 254 | IDH3G_3240 | GCACACGGTGACCATGATCCCAGGGGATNNNNNNNNCTTCAGCTT CCCGATATCCGACGGTAGTGTGAAGGCGGTGCTCGGG |
| 255 | IDH3G_3241 | GTACCAGTGGACTTTGAAGAGGTGCACGTNNNNNNNNCTTCAGCT TCCCGATATCCGACGGTAGTGTCAGAACAAACAATTCC |
| 256 | IDH3G_3242 | GCCCTGAAGGGCAACATCGAAACNNNNNNNNCTTCAGCTTCCCGA TATCCGACGGTAGTGTTGCTGCATGTCAAGTCCGTCTT |
| 257 | IDH3G_3243 | CGTCATCCACTGTAAGAGCCTTCCNNNNNNNNCTTCAGCTTCCCG ATATCCGACGGTAGTGTATGCCATCATGGCCATCCGCC |
| 258 | IDH3G_3244 | GTACAGCAGCCTGGAGCATGAGAGTNNNNNNNNCTTCAGCTTCCC GATATCCGACGGTAGTGTAAACAACATCCTTCGCACCA |
| 259 | IDH3G_3245 | GCATTGCCGAGTATGCCTTCAAGCTGGCGNNNNNNNNCTTCAGCT TCCCGATATCCGACGGTAGTGTCAAGGACATAGACATC |
| 260 | IDH3G_3246 | GCTTTTCCTCCAGTGCTGCAGGGAGGTGGNNNNNNNNCTTCAGCT TCCCGATATCCGACGGTAGTGTAAGATCATCACCAAGG |
| 261 | IDH3G_3247 | CGGCCCCAGCAGTTTGATGTCATGGTGATNNNNNNNNCTTCAGCT TCCCGATATCCGACGGTAGTGTCCAACATCATGAAACT |
| 262 | IDH3G_3248 | GTGGCTGGGGCCAACTATGGCCATGTGTANNNNNNNNCTTCAGCT TCCCGATATCCGACGGTAGTGTTGGATAACACCACCAT |
| 263 | IDH3G_3249 | CCAACCCCACGGCCACCCTNNNNNNNNCTTCAGCTTCCCGATATC CGACGGTAGTGTTCGTCAACAATGTCTGCGCGGGACTG |
| 264 | IDH3G_3250 | GCTGTCCTGGCATCCATGGACAATGAGNNNNNNNNCTTCAGCTTC CCGATATCCGACGGTAGTGTTACGAGGAACACCGGCAA |
| 265 | IDH3G_3239 | CACTGACCACAGCCCCCANNNNNNNNCTTCAGCTTCCCGATATCC GACGGTAGTGTTGCACTCCTATGCCACCTCCATCCGT |
| 266 | L2HGDH_3084 | GTCATCGTTGGTGGCGGANNNNNNNNCTTCAGCTTCCCGATATCC GACGGTAGTGTTTTGGTTGGTGCCTGCGGACGGG |
| 267 | L2HGDH_3085 | GTTCTGGAAAAGGAGAAAGATTTAGCTGTNNNNNNNNCTTCAGCT TCCCGATATCCGACGGTAGTGTTGTGTGGAGGTAGCCG |
| 268 | L2HGDH_3086 | TGTGTACAAGGTGCAGCCCTCCTCTNNNNNNNNCTTCAGCTTCCC GATATCCGACGGTAGTGTCATCCATCACTTTCTATTGG |
| 269 | L2HGDH_3087 | TTCCCAGACTTCAGGCCCNNNNNNNNCTTCAGCTTCCCGATATCC GACGGTAGTGTTATTATAAACCTGAGTCTCTGAAAGCC |
| 270 | L2HGDH_3088 | GCCATATTGTAGGGGTCTAATGGCTATTGNNNNNNNNCTTCAGCT TCCCGATATCCGACGGTAGTGTAAGCTTATAGTAGCTG |
| 271 | L2HGDH_3089 | GCAGGTGGCTCTGTCTTGNNNNNNNNCTTCAGCTTCCCGATATCC GACGGTAGTGTGAGGCTGATCCAGCAGGAGGAT |
| 272 | L2HGDH_3090 | GAATACAAAGGGAGAGGAAATTCGATGTCNNNNNNNNCTTCAGCT TCCCGATATCCGACGGTAGTGTGCCCAGGATTTCCAAG |
| 273 | L2HGDH_3091 | GGCTGCACTCCTGATCCTCGAATTGNNNNNNNNCTTCAGCTTCCC GATATCCGACGGTAGTGTCCTTCAAGAAGTATAGATGG |
| 274 | L2HGDH_3092 | GCCGGTTTCCTTTCCTAGGAGTTCNNNNNNNNCTTCAGCTTCCCG ATATCCGACGGTAGTGTGACCGTATTTCAGAGTTGAGT |
| 275 | L2HGDH_3093 | CCCTTTGACTTCAGTGCCACAGATGNNNNNNNNCTTCAGCTTCCC GATATCCGACGGTAGTGTGGAAATATTTATCCGGTCCC |
| 276 | L2HGDH_3094 | GCATGTTTTCTTGGTGCAACAGTGAAGTNNNNNNNNCTTCAGCTT CCCGATATCCGACGGTAGTGTAAACGAGAGGGTTACAG |
| 277 | L2HGDH_3095 | GCCCAGCTGGAGTAAGAGCCCAGNNNNNNNNCTTCAGCTTCCCGA TATCCGACGGTAGTGTGATTAAACTGGCATCCCAGAAT |
| 278 | L2HGDH_3096 | GGGGATATTGGAAATCGCATTCTTCATGNNNNNNNNCTTCAGCTT CCCGATATCCGACGGTAGTGTCTTCAAAAATTCATCCC |
| 279 | L2HGDH_3097 | GCAGATGAAGTACAACAAAGATTTGNNNNNNNNCTTCAGCTTCCC GATATCCGACGGTAGTGTGATGGAAATCTGGTAGAAG |
| 280 | L2HGDH_3098 | GCAACAAGAATGTACTAATTGCATTCTTTNNNNNNNNCTTCAGCT TCCCGATATCCGACGGTAGTGTGCACCTTCTCCTGCTG |
| 281 | LDHA_0840 | GCATGGCCTGTGCCATCAGTATCTNNNNNNNNCTTCAGCTTCCCG ATATCCGACGGTAGTGTGCATTCCCGATTCCTTTTGGT |
| 282 | LDHA_0842 | GTTATTGGAAGCGGTTGCAATCTGGANNNNNNNNCTTCAGCTTCC CGATATCCGACGGTAGTGTTGAAGGGAGAGATGATGGA |
| 283 | LDHA_0844 | GCACCCAGATTTAGGGACTGATAAAGANNNNNNNNCTTCAGCTTC CCGATATCCGACGGTAGTGTTAATGGGGGAAAGGCTGG |
| 284 | LDHA_0846 | GGATGATGTCTTCCTTAGTGTTCCTTGNNNNNNNNCTTCAGCTTC CCGATATCCGACGGTAGTGTAACTCAAAGGCTACACAT |
| 285 | LDHA_0848 | GCATGTTGTCCTTTTTATCTGATCTGTGNNNNNNNNCTTCAGCTT CCCGATATCCGACGGTAGTGTGGCCCGTTTGAAGAAGA |
| 286 | LDHB_0954 | GTTGGTATGGCGTGTGCTATCAGCATTCNNNNNNNNCTTCAGCTT CCCGATATCCGACGGTAGTGTAAAACCAGGCCCTACT |
| 287 | LDHB_0956 | GCAAGAAGGGGAGAGTCGGCTCAATCNNNNNNNNCTTCAGCTTCC CGATATCCGACGGTAGTGTAAAGGAGAAATGATGGATC |
| 288 | LDHB_0959 | GTGTGGCTGTGTGGAGTGGTGTGNNNNNNNNCTTCAGCTTCCCGA TATCCGACGGTAGTGTCAAACACCGCGTGATTGGAA |
| 289 | LDHB_0961 | GTGTGGCTGATCTTATTGAATCCATGTTGNNNNNNNNCTTCAGCT TCCCGATATCCGACGGTAGTGTGAATCCAGAAATGGGA |
| 290 | LDHB_0963 | GCTCAAGAAAAGTGCAGATACCCTGTGGGNNNNNNNNCTTCAGCT TCCCGATATCCGACGGTAGTGTACAATGGTAAAGGGGA |
| 291 | MAPK8_1429 | CCTATAGGCTCAGGAGCTCAAGGAATAGTNNNNNNNNCTTCAGCT TCCCGATATCCGACGGTAGTGTGGATGAAGCCATTAAA |
| 292 | MAPK8_1432 | GCAAATCTTTGCCAAGTGATTCAGATGNNNNNNNNCTTCAGCTTC CCGATATCCGACGGTAGTGTCAGAGAGCTAGTTCTTAT |
| 293 | MAPK8_1435 | CGTTGACATTTGGTCAGTTGGGTGNNNNNNNNCTTCAGCTTCCCG ATATCCGACGGTAGTGTGCACTTTGAAGATTCTTGACT |
| 294 | MAPK8_1438 | GCTGGTAATAGATGCATCTAAAAGGATCTNNNNNNNNCTTCAGCT TCCCGATATCCGACGGTAGTGTGAAAACAGACCTAAAT |
| 295 | MAPK8_1441 | GCTCTCAGCATCCATCATCATCGTCGTCTNNNNNNNNCTTCAGCT TCCCGATATCCGACGGTAGTGTGGAACACACAATAGAA |
| 296 | MYC_2089 | CGACTCGGTGCAGCCGTATTTCTNNNNNNNNCTTCAGCTTCCCGA TATCCGACGGTAGTGTCTTCTCTGAAAGGCTCTCCTTG |
| 297 | MYC_2090 | TTCGAGCTGCTGCCCACCCCNNNNNNNNCTTCAGCTTCCCGATAT CCGACGGTAGTGTACAGGAACTATGACCTCGACT |
| 298 | MYC_2093 | TCTGTGGAAAAGAGGCAGGCTCCNNNNNNNNCTTCAGCTTCCCGA TATCCGACGGTAGTGTTGCTCTCCTCGACGGAGTCCT |
| 299 | MYC_2094 | CTGGTCCTCAAGAGGTGCCACGTCTNNNNNNNNCTTCAGCTTCCC GATATCCGACGGTAGTGTGAGGAGGAACAAGAAGA |
| 300 | MYC_2095 | CAGTGTCAGAGTCCTGAGACAGATCAGNNNNNNNNCTTCAGCTTC CCGATATCCGACGGTAGTGTACAGCAAACCTCCTCACA |
| 301 | MYC_2096 | GCGCCAGAGGAGGAACGAGCTAAAACGGANNNNNNNNCTTCAGCT TCCCGATATCCGACGGTAGTGTAAGAGGGTCAAGTTGG |
| 302 | MYC_2097 | AGCCACAGCATACATCCTGTCCGTCCNNNNNNNNCTTCAGCTTCC CGATATCCGACGGTAGTGTCGAACACACAACGTCTTGG |
| 303 | MYC_2098 | CTTGAACAGCTACGGAACTCTTGTGCGNNNNNNNNCTTCAGCTTC CCGATATCCGACGGTAGTGTGGCCCCCAAGGTAGTTAT |
| 304 | MYC_2099 | CCTTCTAACAGAAATGTCCTGAGCAATCANNNNNNNNCTTCAGCT TCCCGATATCCGACGGTAGTGTAAGCAGAGGAGCAAAA |
| 305 | NAMPT_2562 | CTTTGAATGCCGTGAAAAGAAGACAGAANNNNNNNNCTTCAGCTT CCCGATATCCGACGGTAGTGTTGTCCTCCGGCCCGAGA |
| 306 | NAMPT_2565 | GGAAATGTTCTCTTCACGGTGGAAAACACNNNNNNNNCTTCAGCT TCCCGATATCCGACGGTAGTGTACAAAGAACATTTCCA |
| 307 | NAMPT 2568 | GTAGCAGGACTTGCTCTAATTANNNNNNNNCTTCAGCTTCCCGAT ATCCGACGGTAGTGTGAAACTTCTGGTAACTTAGATGG |
| 308 | NAMPT_2572 | GCCACCTTATCTTAGAGTTATTCAAGGGGNNNNNNNNCTTCAGCT TCCCGATATCCGACGGTAGTGTACACAGGCACCACTAA |
| 309 | NAMPT_2575 | CGCCAGCAGGGAATTTTGTTACACTGGNNNNNNNNCTTCAGCTTC CCGATATCCGACGGTAGTGTCTCTTGAATTGTTCCTTC |
| 310 | NAPRT1_3185 | GCCCAGGTGGAGCCACTANNNNNNNNCTTCAGCTTCCCGATATCC GACGGTAGTGTTTTCCGGCTCCTGGGCTCTGACGGGT |
| 311 | NAPRT1_3193 | GTTCCAGGTGCCCTGGCTGGAGTNNNNNNNNCTTCAGCTTCCCGA TATCCGACGGTAGTGTAACTTCCTAGCAGTCGCCCT |
| 312 | NAPRT1_3194 | GTCATTGGCATTGGCACCAGTGTGGTCANNNNNNNNCTTCAGCTT CCCGATATCCGACGGTAGTGTGTCTTCCGAGCTGCTG |
| 313 | NAPRT1_3195 | CGAGGACCCCGAGAAGCAGACGTTGCCTGNNNNNNNNCTTCAGCT TCCCGATATCCGACGGTAGTGTGAGGGCAGTGAGGTGA |
| 314 | NOX1_2825 | GCCTTCCTGAAATATGAGAAGGCCGNNNNNNNNCTTCAGCTTCCC GATATCCGACGGTAGTGTTCTTCCCTGTTGCCTAGAAG |
| 315 | NOX1_2829 | CCCATCCAGTCCCGAAACACNNNNNNNNCTTCAGCTTCCCGATAT CCGACGGTAGTGTCTATTCACATCATTGCACACCTGTT |
| 316 | NOX1_2833 | GCACCGGTCATTCTTTATATCTGTGNNNNNNNNCTTCAGCTTCCC GATATCCGACGGTAGTGTGAGAGCATGAATGAGAGTCA |
| 317 | NOX1_2837 | GCTGGTTGGAGCAGGAATTGGGGTNNNNNNNNCTTCAGCTTCCCG ATATCCGACGGTAGTGTCAGCAGGGGACTGGACAGAAA |
| 318 | NOX1_2841 | GTCTGTAGTGGGAGTTTTCTTATGTGGCCNNNNNNNNCTTCAGCT TCCCGATATCCGACGGTAGTGTGGTCATGCAGCATTAA |
| 319 | NOX3_2954 | CGAGTTATTTTGGGTTCAACACTGGCNNNNNNNNCTTCAGCTTCC CGATATCCGACGGTAGTGTTGGGGTGCTGGATTTTGAA |
| 320 | NOX3_2958 | CCCCACAAACACAACCACNNNNNNNNCTTCAGCTTCCCGATATCC GACGGTAGTGTACATCGTGGCGCATTTCTTCAACCTGG |
| 321 | NOX3_2962 | GCGATTTCAACAAGAAGTTGTCATTACCANNNNNNNNCTTCAGCT TCCCGATATCCGACGGTAGTGTGCAGAATGGCAGACAG |
| 322 | NOX3_2966 | GCGTTGCCGCGGGGATCGGAGTCACTCNNNNNNNNCTTCAGCTTC CCGATATCCGACGGTAGTGTCTACTGGAGGCCTTTGG |
| 323 | NOX3_2970 | CAAGCAGATTGCCTACAATCACCCCANNNNNNNNCTTCAGCTTCC CGATATCCGACGGTAGTGTTTTCTTACCGGCTGGGATG |
| 324 | NOX4a_3007 | GTCCTGCTTTTCTGGAAAACCTTCNNNNNNNNCTTCAGCTTCCCG ATATCCGACGGTAGTGTTCCTTCTCGGTCCGGCGGGCA |
| 325 | NOX4a_3011 | ACTTCTCTTCACAACTGTTCCTGGNNNNNNNNCTTCAGCTTCCCG ATATCCGACGGTAGTGTCTATCTGTATTTTCTCAGGCG |
| 326 | NOX4a_3015 | GCCCAGATTCCAAGCTAATTTTCCACNNNNNNNNCTTCAGCTTCC CGATATCCGACGGTAGTGTACCAGCTCTCAGAATATTT |
| 327 | NOX4a_3019 | GAAATTCTGCCCTTCATTCAATCNNNNNNNNCTTCAGCTTCCCGA TATCCGACGGTAGTGTATCATCCATTTACCCTCACAAT |
| 328 | NOX4a_3023 | CGGTGGAAACTTTTGTTTGATGAAATAGCNNNNNNNNCTTCAGCT TCCCGATATCCGACGGTAGTGTGGCAAGAGAACAGACC |
| 329 | NQO1_0486 | GCGGCTTTGAAGAAGAAAGGATGGGANNNNNNNNCTTCAGCTTCC CGATATCCGACGGTAGTGTAACCACGAGCCCAGCCAAT |
| 330 | NQO1_0488 | GCTGGAAGCCGCAGACCTTGTGATATTCNNNNNNNNCTTCAGCTT CCCGATATCCGACGGTAGTGTCATTTCCAGAAAGGACA |
| 331 | NQO1_0490 | CATCACCACTGGTGGCAGTGGCTCCATGNNNNNNNNCTTCAGCTT CCCGATATCCGACGGTAGTGTGTGGTTTGGAGTCCCTG |
| 332 | NQO1_0492 | GCCCGAATTCAAATCCTGGAAGGATGGAANNNNNNNNCTTCAGCT TCCCGATATCCGACGGTAGTGTAAGGGATCCACGGGGA |
| 333 | NQO1_0494 | CAAGTCCATCCCAACTGACAACCNNNNNNNNCTTCAGCTTCCCGA TATCCGACGGTAGTGTACACCACTGTATTTTGCTCCAA |
| 334 | OGDH_0591 | GATTCGGTGCTATTCTGCACCTGTTGNNNNNNNNCTTCAGCTTCC CGATATCCGACGGTAGTGTAAAAACTTCAGGACAAAAA |
| 335 | OGDH_0592 | GCTGGAAAACCCCAAAAGTGTACATAAGNNNNNNNNCTTCAGCTT CCCGATATCCGACGGTAGTGTACAAAACAGACCAGCAG |
| 336 | OGDH_0593 | CTGCCTACCAGAGTCCCCTTCNNNNNNNNCTTCAGCTTCCCGATA TCCGACGGTAGTGTCCCTTTCTCAGTGGGACTAGTTCG |
| 337 | OGDH_0595 | GCTGATCTGGACTCCTCCGTGCCCGCTGANNNNNNNNCTTCAGCT TCCCGATATCCGACGGTAGTGTTAGAAGCACAGCCCAA |
| 338 | OGDH_0596 | TTCCACTTGCCCACCACCACNNNNNNNNCTTCAGCTTCCCGATAT CCGACGGTAGTGTACCATGTAGCACAGCTGGACCCCCT |
| 339 | OGDH_0597 | GCATATTGGGGTGGAGTTCATGTTCATNNNNNNNNCTTCAGCTTC CCGATATCCGACGGTAGTGTATGGCCTGGATGAGTCTG |
| 340 | OGDH_0598 | GCAGTTCACAAATGAGGAGAAACGGACNNNNNNNNCTTCAGCTTC CCGATATCCGACGGTAGTGTTGCGGGAGATCATCCG |
| 341 | OGDH_0599 | GCTTTGGTCTAGAAGGCTGCGAGGTACTNNNNNNNNCTTCAGCTT CCCGATATCCGACGGTAGTGTAGAAGTTTGAGACCCCT |
| 342 | OGDH_0600 | GAGGGCGGCTGAACGTGCTTGCAAATNNNNNNNNCTTCAGCTTCC CGATATCCGACGGTAGTGTGAAGTGGTCCTCTGAGAAG |
| 343 | OGDH_0601 | GCTGATGAGGGCTCCGGAGATGTGAAGTNNNNNNNNCTTCAGCTT CCCGATATCCGACGGTAGTGTGAGAATGGCGTGGACT |
| 344 | OGDH_0602 | CTTGTCCTTGGTGGCCAACCCTTCNNNNNNNNCTTCAGCTTCCCG ATATCCGACGGTAGTGTAGCTGGAACAGATCTTCTGTC |
| 345 | OGDH_0603 | GCGACACTGAAGGGAAAAAGGTAAGGCCNNNNNNNNCTTCAGCTT CCCGATATCCGACGGTAGTGTACCGCAGGATCAATCGT |
| 346 | OGDH_0604 | GGAGTTCCGCTCACCAACATAACCCAGANNNNNNNNCTTCAGCTT CCCGATATCCGACGGTAGTGTTGACCCCGTGGTGATGG |
| 347 | PARP1_1853 | AGCATCCCCAAGGACTCGCNNNNNNNNCTTCAGCTTCCCGATATC CGACGGTAGTGTTGTTTCTAGGTCGTGGCGTCGGGCTT |
| 348 | PARP1_1859 | GAGTGGATGAAGTGGCGAAGAAGAAATCTNNNNNNNNCTTCAGCT TCCCGATATCCGACGGTAGTGTCGAGTACAGTGCGAGT |
| 349 | PARP1_1868 | GCAAGGGCCAGGTCAAGGAGGAAGGTATCNNNNNNNNCTTCAGCT TCCCGATATCCGACGGTAGTGTCAAGAGCCTTCAGGAG |
| 350 | PARP1_1877 | GCTGGACATCGAGGTGGCCTACAGTNNNNNNNNCTTCAGCTTCCC GATATCCGACGGTAGTGTCTCTCAGATCCTGGATCTCT |
| 351 | PARP1_1883 | CCTTCAGCTAACATTAGTCTGGATGGTGNNNNNNNNCTTCAGCTT CCCGATATCCGACGGTAGTGTGGCTTAATCCTGTTGGG |
| 352 | PC_0499 | GTGGATGTGGCAGCTGATTCCATGTCTGNNNNNNNNCTTCAGCTT CCCGATATCCGACGGTAGTGTACCATGCTGGTCAGCT |
| 353 | PC_0507 | GCATGAGGGTGGTGCACAGCTANNNNNNNNCTTCAGCTTCCCGAT ATCCGACGGTAGTGTTTGCTGCGGGTGTTCCCGTTGTC |
| 354 | PC_0515 | CCAAAAGCTGTTGCACTACCTCGGCCNNNNNNNNCTTCAGCTTCC CGATATCCGACGGTAGTGTAAGACCAACATCGCCTTCC |
| 355 | PC_0524 | GCGCGTGTTTGACTACAGTGAGTACTGNNNNNNNNCTTCAGCTTC CCGATATCCGACGGTAGTGTAGGCTGGAGCTGATGTG |
| 356 | PC_0532 | CAAGGACACCCAGGCCATGAAGGAGATGNNNNNNNNCTTCAGCTT CCCGATATCCGACGGTAGTGTTGAGGTGGAGCTGGAG |
| 357 | PDHA1_0305 | GAAATTAAGAAATGTGACCTTCACCGGCNNNNNNNNCTTCAGCTT CCCGATATCCGACGGTAGTGTCACTGCCTGTGCTTCAT |
| 358 | PDHA1_0308 | GCTCACGGCTTTACTTTCACCCGGGGNNNNNNNNCTTCAGCTTCC CGATATCCGACGGTAGTGTCAGATCAGCTGTATAAACA |
| 359 | PDHA1_0311 | GTGGAAATTACCTTGTATTTTCATCTGTGNNNNNNNNCTTCAGCT TCCCGATATCCGACGGTAGTGTCTGGGCGCTGGGATTG |
| 360 | PDHA1_0313 | GTAGATCTGGGAAGGGGCCCATCCTGATGNNNNNNNNCTTCAGCT TCCCGATATCCGACGGTAGTGTTGTTGAGAGAGCGGCA |
| 361 | PDHA1_0316 | GGAAGAGCTGGGCTACCACATCTACTCCANNNNNNNNCTTCAGCT TCCCGATATCCGACGGTAGTGTAAGGACAGGATGGTGA |
| 362 | PDK1_1451 | GATAATCTTCTCAGGACACCATCCGNNNNNNNNCTTCAGCTTCCC GATATCCGACGGTAGTGTCTCTCCATGAAGCAGTTCCT |
| 363 | PDK1_1453 | GCAAGATGATCTTTACAGATACTGTGNNNNNNNNCTTCAGCTTCC CGATATCCGACGGTAGTGTCTGGTATATCCAGAGTCTT |
| 364 | PDK1_1456 | GCTATGAAAATGCTAGGCGTCTGTGNNNNNNNNCTTCAGCTTCCC GATATCCGACGGTAGTGTCAATTAGAATGTTACTCAAT |
| 365 | PDK1_1459 | GCGTTCCTTTGAGGAAAATTGACAGACTTNNNNNNNNCTTCAGCT TCCCGATATCCGACGGTAGTGTCAAGAATGCAATGAGA |
| 366 | PDK1_1462 | GCCTGGAAGCATTACAACACCAACCNNNNNNNNCTTCAGCTTCCC GATATCCGACGGTAGTGTTACGCACAATACTTCCAAGG |
| 367 | PFKB1_1411 | TGCGCCCTGGCAGCCCTGAAGGATGTTCANNNNNNNNCTTCAGCT TCCCGATATCCGACGGTAGTGTAAGAAAAAACCTCTAG |
| 368 | PFKB1_1414 | GAGGAACTGGACAGCCACCTGTCCTACATNNNNNNNNCTTCAGCT TCCCGATATCCGACGGTAGTGTCAGAAAACATCAGGCA |
| 369 | PFKB1_1417 | GTCACATGAAGAGGACCATCCAGACAGCNNNNNNNNCTTCAGCTT CCCGATATCCGACGGTAGTGTAACTCAACATCAGAGGC |
| 370 | PFKB1_1420 | GCTGTCATGCGGTGCCTCCTGGCCTATTTNNNNNNNNCTTCAGCT TCCCGATATCCGACGGTAGTGTACCAAGATAAATATCG |
| 371 | PFKB1_1422 | ACATCACCCGGGAACCTGANNNNNNNNCTTCAGCTTCCCGATATC CGACGGTAGTGTAGCTTCCATATCTCAAGTGCCCTCTG |
| 372 | PFKMb_0914 | GCTGGGGAAGCTTCTACTTCCAGCATGCTNNNNNNNNCTTCAGCT TCCCGATATCCGACGGTAGTGTAACCGCCTTCACAGCA |
| 373 | PFKMb_0920 | GTGGAGTGACTTGTTGAGTGACCTCCNNNNNNNNCTTCAGCTTCC CGATATCCGACGGTAGTGTAAGGACTTTCGGGAACGAG |
| 374 | PFKMb_0926 | GCAGGATGGGTGTGGAAGCAGTGATNNNNNNNNCTTCAGCTTCCC GATATCCGACGGTAGTGTAACCAATCACCTCAGAAGAC |
| 375 | PFKMb_0932 | CATTGGGGGCTTTGAGGCTTACACAGGGGNNNNNNNNCTTCAGCT TCCCGATATCCGACGGTAGTGTTATGTTGGGGGCTGGA |
| 376 | PFKMb_0940 | GCTGAAGGACCAGACAGATTTTGAGCANNNNNNNNCTTCAGCTTC CCGATATCCGACGGTAGTGTTGAACTGGATGTCTGGG |
| 377 | PGAM1_2160 | GATGTGGCTGCCAGTGGTGAGGACTTGNNNNNNNNCTTCAGCTTC CCGATATCCGACGGTAGTGTACGAGGAGGCGAAGCG |
| 378 | PGAM1_2162 | CGCAGGTATGCAGACCTCACAGAAGATNNNNNNNNCTTCAGCTTC CCGATATCCGACGGTAGTGTCAATAAAGCAGAAACTGC |
| 379 | PGAM1_2163 | CAGATCAAGGAGGGGAAACGTGTACTGATNNNNNNNNCTTCAGCT TCCCGATATCCGACGGTAGTGTACAGCAACATCAGTAA |
| 380 | PGAM1_2165 | GCGCAAAGCCATGGAAGCTGTGGCTNNNNNNNNCTTCAGCTTCCC GATATCCGACGGTAGTGTGGAGGGTCTCTCTGAAGAG |
| 381 | PGAM1_2166 | GCCGGCGGGGAGGATACTGTNNNNNNNNCTTCAGCTTCCCGATAT CCGACGGTAGTGTGGTATTCCCATTGTCTATGAATTGG |
| 382 | PGD_2169 | GCCAATGAGGCAAAGGGAACCAAAGTGGTNNNNNNNNCTTCAGCT TCCCGATATCCGACGGTAGTGTAAGCTGACATCGCGCT |
| 383 | PGD_2173 | GCTGCAAAAGTGGGAACTGGAGNNNNNNNNCTTCAGCTTCCCGAT ATCCGACGGTAGTGTGCCAAGGGAATTTTATTTGTGGG |
| 384 | PGD_2177 | CCCGTCACCCTCATTGGAGAAGCTGTCTTNNNNNNNNCTTCAGCT TCCCGATATCCGACGGTAGTGTCAGCCAATATTCTCAA |
| 385 | PGD_2181 | GTCAGCTGTTGAAAACTGCCAGGACNNNNNNNNCTTCAGCTTCCC GATATCCGACGGTAGTGTTTATGGTGGCATCGCCCTGA |
| 386 | PGD_2183 | CCAGGGCAGTTTATCCACACCAANNNNNNNNCTTCAGCTTCCCGA TATCCGACGGTAGTGTTTCCCATGCCCTGTTTTACCAC |
| 387 | PGI_1528 | ACCGCTTCAACCACTTCAGCNNNNNNNNCTTCAGCTTCCCGATAT CCGACGGTAGTGTCTCACTCAGTGTACCTTCTAGTCCC |
| 388 | PGI_1533 | GTGGTTTCTCCAGGCGGCCAAGGATNNNNNNNNCTTCAGCTTCCC GATATCCGACGGTAGTGTCAACATTGATGGAACTCACA |
| 389 | PGI_1536 | GCTGGGTATCTGGTACATCAACTGCTTTNNNNNNNNCTTCAGCTT CCCGATATCCGACGGTAGTGTACTCTCCATTGCCCTGC |
| 390 | PGI_1539 | GCATCACAAGATCCTCCTGGCCAANNNNNNNNCTTCAGCTTCCCG ATATCCGACGGTAGTGTTTGTGTGGGGGGAGCCAGGG |
| 391 | PGI_1542 | GCTGGCTAAGAAAATAGAGCCTGAGCTTGNNNNNNNNCTTCAGCT TCCCGATATCCGACGGTAGTGTACCAAGCTCACACCAT |
| 392 | PGK1_0371 | CAACCAGAGGATTAAGGCTGCTGTCCNNNNNNNNCTTCAGCTTCC CGATATCCGACGGTAGTGTCCCAGCTGTATTTCCAAAA |
| 393 | PGK1_0374 | GCTGGAGAACCTCCGCTTTCATGTGGNNNNNNNNCTTCAGCTTCC CGATATCCGACGGTAGTGTTCCTTAGAGCCAGTTGCTG |
| 394 | PGK1_0377 | GCAGACAAGATCCAGCTCATCANNNNNNNNCTTCAGCTTCCCGAT ATCCGACGGTAGTGTTCCATGGTAGGAGTCAATCTGCC |
| 395 | PGK1_0380 | GCTGGCTGGATGGGCTTGGACTGTNNNNNNNNCTTCAGCTTCCC GATATCCGACGGTAGTGTAAAGACCTAATGTCCAAAGC |
| 396 | PGK1_0383 | GTGGTGCCAGTTTGGAGCTCCTGGAAGGTNNNNNNNNCTTCAGCT TCCCGATATCCGACGGTAGTGTCATGGATGAGGTGGTG |
| 397 | PGK2_3123 | CCAGATTACAAACAACCAGAGGATCANNNNNNNNCTTCAGCTTCC CGATATCCGACGGTAGTGTGGTGTCAGCCTATGTCTTT |
| 398 | PGK2_3125 | GTTCCTGAAGGACTGTGTAGGCGCAGNNNNNNNNCTTCAGCTTCC CGATATCCGACGGTAGTGTAATGGAGCCAAGGCAGTAG |
| 399 | PGK2_3128 | GCTAAAGCCTTGGAAAACCCAGTGAGANNNNNNNNCTTCAGCTTC CCGATATCCGACGGTAGTGTGCTAGGGGACGTCTATGT |
| 400 | PGK2_3131 | GTTTGACGAGAACGCTCAGGTTGGAAAAGNNNNNNNNCTTCAGCT TCCCGATATCCGACGGTAGTGTATGGAGATTGGTGCTT |
| 401 | PGK2_3134 | GATAAAGTCAGCCATGTCAGCACTGGNNNNNNNNCTTCAGCTTCC CGATATCCGACGGTAGTGTGGGATGCCTTTGCTAAGGG |
| 402 | PKM_1091 | CCCAACCCCAGAGAACCAANNNNNNNNCTTCAGCTTCCCGATATC CGACGGTAGTGTAGAAGTCCCCAGCGCCGTTCCTTCCA |
| 403 | PKM_1095 | CACTAAAGGACCTGAGATCCGAACNNNNNNNNCTTCAGCTTCCCG ATATCCGACGGTAGTGTTGGCTCGTCTGAACTTCTCTC |
| 404 | PKM_1099 | GCAGGATGTTGATATGGTGTTTGCGTNNNNNNNNCTTCAGCTTCC CGATATCCGACGGTAGTGTCTGGTGACGGAGGTGGAAA |
| 405 | PKM_1103 | GCCAAAGGGGACTATCCTCTGGANNNNNNNNCTTCAGCTTCCCGA TATCCGACGGTAGTGTCTGTCATCTGTGCTACTCAGAT |
| 406 | PKM_1107 | ACCTCCGGGTGAACTTTGCCATGAATGTTNNNNNNNNCTTCAGCT TCCCGATATCCGACGGTAGTGTACGTGCCCCCATCATT |
| 407 | PRDX1_1078 | GTTGTGTTCTTCTTTTACCCTCTTGNNNNNNNNCTTCAGCTTCCC GATATCCGACGGTAGTGTGCTGATAGGAAGATGTCTTC |
| 408 | PRDX1_1080 | CGAAGCGCACCATTGCTCAGGATTATGNNNNNNNNCTTCAGCTTC CCGATATCCGACGGTAGTGTAAGAAACTCAACTGCCAA |
| 409 | PRDX1_1081 | GCAGATCACTGTAAATGACCTCCCTGNNNNNNNNCTTCAGCTTCC CGATATCCGACGGTAGTGTCCCATGAACATTCCTTTGG |
| 410 | PRDX1_1082 | ACAAACATGGGGAAGTGTGCCCAGCNNNNNNNNCTTCAGCTTCCC GATATCCGACGGTAGTGTTCTCGTTCAGGGGCCTTTTT |
| 411 | PRDX1_1083 | GCTGGGCTGTTTTAGTGCCAGGCTNNNNNNNNCTTCAGCTTCCCG ATATCCGACGGTAGTGTTAGTTCAGGCCTTCCAGTTCA |
| 412 | PRKAA1_2662 | CAGAACCTCAAGCTTTTCAGGCATCCNNNNNNNNCTTCAGCTTCC CGATATCCGACGGTAGTGTTCGGCACCTTCGGCAAAGT |
| 413 | PRKAA1_2666 | CACCCAACTATGCTGCACCAGAAGTANNNNNNNNCTTCAGCTTCC CGATATCCGACGGTAGTGTGGTGGTCCATAGAGATTTG |
| 414 | PRKAA1_2670 | GAGTGCTCAGAAGAGGAAGTTCTCAGCTGNNNNNNNNCTTCAGCT TCCCGATATCCGACGGTAGTGTAAGATATCAGGGAACA |
| 415 | PRKAA1_2674 | GGATTATGAATGGAAGGTTGTAAACCCATNNNNNNNNCTTCAGCT TCCCGATATCCGACGGTAGTGTGAATTAAATCCACAGA |
| 416 | PRKAA1_2678 | GTGCAAATCTAATTAAAATTCTTGCACAANNNNNNNNCTTCAGCT TCCCGATATCCGACGGTAGTGTTGCTGAGGCTCAAGGA |
| 417 | PRKAA2_2685 | CGAGAAATTCAAAATCTAAAACTCTTTCGNNNNNNNNCTTCAGCT TCCCGATATCCGACGGTAGTGTTGGGCGTCGGCACCTT |
| 418 | PRKAA2_2688 | GCCAAGATAGCCGATTTCGGATTATCTAANNNNNNNNCTTCAGCT TCCCGATATCCGACGGTAGTGTAAGAGATGGAAGCCAG |
| 419 | PRKAA2_2691 | GCTGCAGGTTGACCCACTNNNNNNNNCTTCAGCTTCCCGATATCC GACGGTAGTGTTGTATGCTCTTCTTTGTGGCACCCTCC |
| 420 | PRKAA2_2695 | GCAGACAGCCCCAAAGCAAGATGNNNNNNNNCTTCAGCTTCCCGA TATCCGACGGTAGTGTGAATAATGAACCAAGCCAGTGA |
| 421 | PRKAA2_2699 | CCACAACTGCAGAGAGCCNNNNNNNNCTTCAGCTTCCCGATATCC GACGGTAGTGTGTTGATAACAGGAGCTATCTTTTGGAC |
| 422 | RPIA_3164 | CTACAATTGTCCATGCTGTGCAGCGNNNNNNNNCTTCAGCTTCCC GATATCCGACGGTAGTGTACTCCAACAGCATCTGCCC |
| 423 | RPIA_3166 | CTCAATCTCATCAAGGGTGGCNNNNNNNNCTTCAGCTTCCCGATA TCCGACGGTAGTGTCCTCGTCTGTATTCCCACTTCCTT |
| 424 | RPIA_3168 | GCTGTGAGCCAGAAGTTTGGGGNNNNNNNNCTTCAGCTTCCCGAT ATCCGACGGTAGTGTGTGGCTGGCTATGCTAGTCGCTT |
| 425 | RPIA_3169 | GTTTGACCGGGTACACAAATGGAGTGAANNNNNNNNCTTCAGCTT CCCGATATCCGACGGTAGTGTGGCCTATGTCCCAGTGA |
| 426 | RPIA_3171 | GGAGCAGAGTGTGTTCACCTTGAGTCTCCNNNNNNNNCTTCAGCT TCCCGATATCCGACGGTAGTGTATCAAAATGATCCCAG |
| 427 | SDHA_1569 | GGGCATCTGCTAAAGTTTCAGATTCCATNNNNNNNNCTTCAGCTT CCCGATATCCGACGGTAGTGTTGTCGGGGGTCCGGGG |
| 428 | SDHA_1570 | GCATTTGGCCTTTCTGAGGCAGGGTTTNNNNNNNNCTTCAGCTTC CCGATATCCGACGGTAGTGTCACTGTTGATGGGAACAA |
| 429 | SDHA_1571 | GCACAGCTAGAAAATTATGGCATGCCGTNNNNNNNNCTTCAGCTT CCCGATATCCGACGGTAGTGTTTTGATGCAGTGGTGGT |
| 430 | SDHA_1572 | GCCTCAAGTTTGGAAAGGGCGGGCAGNNNNNNNNCTTCAGCTTCC CGATATCCGACGGTAGTGTAGCATGTGTTACCAAGCTG |
| 431 | SDHA_1573 | GCGATATGATACCAGCTATTTTGTGGAGNNNNNNNNCTTCAGCTT CCCGATATCCGACGGTAGTGTTTATCAGCGTGCATTTG |
| 432 | SDHA_1574 | GCATAGAGGACGGGTCCATCCATNNNNNNNNCTTCAGCTTCCCGA TATCCGACGGTAGTGTGACTGGCCACTCGCTATTGCA |
| 433 | SDHA_1575 | CTTCAGCTGCACGTCTGCCNNNNNNNNCTTCAGCTTCCCGATATC CGACGGTAGTGTTTTTGCCTTGGATCTCCTGATGGAGA |
| 434 | SDHA_1576 | GTTCAGTTCCACCCTACAGGCATATATGGNNNNNNNNCTTCAGCT TCCCGATATCCGACGGTAGTGTCTGTTGTTGCCACAGG |
| 435 | SDHA_1577 | GCGAAAGGTTTATGGAGCGATACGCCCNNNNNNNNCTTCAGCTTC CCGATATCCGACGGTAGTGTAGGGCAGGCCTTCCTTG |
| 436 | SDHA_1578 | CGAGAAGGAAGAGGCTGTGGCCCTGAGANNNNNNNNCTTCAGCTT CCCGATATCCGACGGTAGTGTTGTCGTGGAGAGGGAGG |
| 437 | SDHA_1579 | GCCTGGCATTTCAGAGACAGCNNNNNNNNCTTCAGCTTCCCGATA TCCGACGGTAGTGTTGGCGTCTAGAGATGTGGTGTCTC |
| 438 | SDHA_1580 | GCGGCATTCCCACCAACTACAANNNNNNNNCTTCAGCTTCCCGAT ATCCGACGGTAGTGTTGCACCACCTACCTCCAGAGCA |
| 439 | SDHA_1581 | GCCTCGGTACATGGTGCCAANNNNNNNNCTTCAGCTTCCCGATAT CCGACGGTAGTGTTCCCTGTCCTCCCCACCGTGCATTA |
| 440 | SDHA_1582 | GCCTGGAGATAAAGTCCCTCCAATTAANNNNNNNNCTTCAGCTTC CCGATATCCGACGGTAGTGTTGTACGCCTGTGGGGAGG |
| 441 | SDHA_1583 | GCTGATGGAAGCATAAGAACATCGGNNNNNNNNCTTCAGCTTCCC GATATCCGACGGTAGTGTCTGGTTGTCTTTGGTCGGGC |
| 442 | SDHA_1584 | GCGTGTTGCAAGAAGGTTGTGGGANNNNNNNNCTTCAGCTTCCCG ATATCCGACGGTAGTGTGCTGGGGAAGAATCTGTCATG |
| 443 | SDHA_1585 | GTCTGGAACACGGACCTGGTGGAGANNNNNNNNCTTCAGCTTCCC GATATCCGACGGTAGTGTATGCAGAAGTCAATGCAAAA |
| 444 | SDHA_1586 | CAGAGGCACGGAAGGAGTCACNNNNNNNNCTTCAGCTTCCCGATA TCCGACGGTAGTGTATCAGCAAGCTCTATGGAGACCTA |
| 445 | SDHA_1587 | GCCCATCCAGGGGCAACAGAAGAANNNNNNNNCTTCAGCTTCCCG ATATCCGACGGTAGTGTTGGAGCTGCAGAACCTGATGC |
| 446 | SDHA_1588 | GGAAGGTCACTCTGGAATATAGACCCGTGNNNNNNNNCTTCAGCT TCCCGATATCCGACGGTAGTGTGGAAGACTACAAGGTG |
| 447 | SDHA_1589 | GTGGTGATGACAGAATCAGCTTTTGNNNNNNNNCTTCAGCTTCCC GATATCCGACGGTAGTGTGTCCTATGTGGACGTTGGCA |
| 448 | SDHB_2193 | CCCAGACAAGGCTGGAGACAAACCTCANNNNNNNNCTTCAGCTTC CCGATATCCGACGGTAGTGTTCTCCTTGAGGCGCCGGT |
| 449 | SDHB_2195 | TGACTCTACTTTGACCTTCCGAAGATCNNNNNNNNCTTCAGCTTC CCGATATCCGACGGTAGTGTTTGCCATCTATCGATGGG |
| 450 | SDHB_2196 | CACTCTAGCTTGCACCCGAAGGATTGNNNNNNNNCTTCAGCTTCC CGATATCCGACGGTAGTGTTGTGGCCCCATGGTATTGG |
| 451 | SDHB_2197 | GATCTTGTTCCCGATTTGAGCAACTTCTANNNNNNNNCTTCAGCT TCCCGATATCCGACGGTAGTGTTCTGTGGCTCTTGTGC |
| 452 | SDHB_2198 | GCAGCAGTATCTGCAGTCCATAGANNNNNNNNCTTCAGCTTCCCG ATATCCGACGGTAGTGTCAAAAATCTACCCTCTTCCAC |
| 453 | SDHB_2199 | GCTACTGGTGGAACGGAGACAAATATCTGNNNNNNNNCTTCAGCT TCCCGATATCCGACGGTAGTGTGAAGAAGAAGGATGAA |
| 454 | SDHB_2200 | AGCGCCTGGCCAAGCTGCANNNNNNNNCTTCAGCTTCCCGATATC CGACGGTAGTGTAGTGCATTCTCTGTGCCTGCTGTAGC |
| 455 | SDHB_2201 | GCAGAGATCAAGAAAATGATGGCAACCTNNNNNNNNCTTCAGCTT CCCGATATCCGACGGTAGTGTAGAGATGACTTCACAG |
| 456 | SDHB_2202 | CCAGCTCAGAGCTGAACANNNNNNNNCTTCAGCTTCCCGATATCC GACGGTAGTGTATGAACTGCACAAGGACCTGTCCTAAG |
| 457 | SDHC_2206 | GCTTTGAGTGCAGGGGTCTCTCTTTTTGNNNNNNNNCTTCAGCTT CCCGATATCCGACGGTAGTGTGAAGAGATGGAGCGGT |
| 458 | SDHC_2207 | GCACTGATCCACACAGCTAANNNNNNNNCTTCAGCTTCCCGATAT CCGACGGTAGTGTTGTCCATCTGCCACCGTGGCACTGG |
| 459 | SDHC_2208 | GCCTGAAGATTCCCCAGCTATACNNNNNNNNCTTCAGCTTCCCGA TATCCGACGGTAGTGTTTTGGAACTTGTGAAGTCCCTG |
| 460 | SDHC_2209 | CCCAGCATCATCTTCCTACACANNNNNNNNCTTCAGCTTCCCGAT ATCCGACGGTAGTGTATCCGACACTTGATGTGGGACCT |
| 461 | SDHD_2214 | CACTTGTCACCGAGCCACCATTCNNNNNNNNCTTCAGCTTCCCGA TATCCGACGGTAGTGTTGAGTGCCGTTTGCGGTGCCCT |
| 462 | SDHD_2215 | GTCTGCTTCCGGCTGCTTATTTGAATCCNNNNNNNNCTTCAGCTT CCCGATATCCGACGGTAGTGTACCGACCTATCCCAGAA |
| 463 | SDHD_2216 | GTTGTTACTGACTATGTTCATGGGGATGNNNNNNNNCTTCAGCTT CCCGATATCCGACGGTAGTGTGAGAGGGTTGTCAGTGT |
| 464 | SDHD_2217 | GCTATTTCAACTATCACGATGTGGGCNNNNNNNNCTTCAGCTTCC CGATATCCGACGGTAGTGTCCTCACTCTTCATGGTCAC |
| 465 | SDHD_2218 | GTATGCCTCTTTGCCTCTGCTTTGTNNNNNNNNCTTCAGCTTCCC GATATCCGACGGTAGTGTTGGCACTTTCAGCTTTAACC |
| 466 | SLC16A1_0891 | CGGCTTCTCTTATGCATTTCCCNNNNNNNNCTTCAGCTTCCCGAT ATCCGACGGTAGTGTTTGGATTTGACCTGCATTTTGG |
| 467 | SLC16A1_0894 | CGTCTGTATTGGAGTCATTGGAGGTNNNNNNNNCTTCAGCTTCCC GATATCCGACGGTAGTGTTGGAGGTCCTATCAGCAGTA |
| 468 | SLC16A1_0898 | CAGATCTTATTGGAAGACACCCTAAACNNNNNNNNCTTCAGCTTC CCGATATCCGACGGTAGTGTGTTGCTGGAGCCCTCAT |
| 469 | SLC16A1_0902 | GTTGGATTCTGTGTCTATGCGGGATTCTTNNNNNNNNCTTCAGCT TCCCGATATCCGACGGTAGTGTGACCATCTATGGGACT |
| 470 | SLC16A1_0906 | GGAGGGCCCAAGGAGGAGGAAAGNNNNNNNNCTTCAGCTTCCCGA TATCCGACGGTAGTGTTGGCAAAAGAACAGAAA |
| 471 | SLC16A3_1117 | GCGGCTTTGTGCTTTACGNNNNNNNNCTTCAGCTTCCCGATATCC GACGGTAGTGTATCCTGGGCGGCCTGCTGCTCAACTGC |
| 472 | SLC16A3_1120 | GCTCTGCAGTGTGTGCGTGAACNNNNNNNNCTTCAGCTTCCCGAT ATCCGACGGTAGTGTCTTCTCCTACGCCTTCCCCAA |
| 473 | SLC16A3_1124 | CGACACCAAGGCCGCCTTCCTNNNNNNNNCTTCAGCTTCCCGATA TCCGACGGTAGTGTGCCTGCTAGACCTGAGCGTCTTCC |
| 474 | SLC16A3_1128 | CGACCCACGTCTACATGTACGTGTTNNNNNNNNCTTCAGCTTCCC GATATCCGACGGTAGTGTTGCAGTTCGAGGTGCTCATG |
| 475 | SLC16A3_1130 | GCATTTCCTGAAGGCTGAGCCTGAGAAAANNNNNNNNCTTCAGCT TCCCGATATCCGACGGTAGTGTCTGGGCAACTTCTTCT |
| 476 | SLC16A7_1390 | CCTATGCATTCCCCAAAGCNNNNNNNNCTTCAGCTTCCCGATATC CGACGGTAGTGTCTCTTGGTGCCAACAGAGTTACTCT |
| 477 | SLC16A7_1393 | GCAACCCGCCTTAACCATAATTGGCAAANNNNNNNNCTTCAGCTT CCCGATATCCGACGGTAGTGTGGTGGTGATAGCAGGAG |
| 478 | SLC16A7_1397 | CCCTTTTTAAGCATAGAGGATTTCTGATANNNNNNNNCTTCAGCT TCCCGATATCCGACGGTAGTGTACCCAATCAAACCACT |
| 479 | SLC16A7_1401 | GTGTTAGCAGTGTTCTCTTTGNNNNNNNNCTTCAGCTTCCCGATA TCCGACGGTAGTGTCGACCTCGAATTCAGTACTTCTTC |
| 480 | SLC16A7_1404 | CCTTGAGCAAATCTAAACATTCGGANNNNNNNNCTTCAGCTTCCC GATATCCGACGGTAGTGTTGTCCTGTGGGGCTATTGTG |
| 481 | SLC2A1_2721 | GCTCTGGTCCCTCTCAGTGGCCATCTTTTNNNNNNNNCTTCAGCT TCCCGATATCCGACGGTAGTGTTCCCTGCAGTTTGGCT |
| 482 | SLC2A1_2724 | TCACCCACAGCCCTTCGTNNNNNNNNCTTCAGCTTCCCGATATCC GACGGTAGTGTCTTCGTGTCCGCCGTGCTCATGGGCTT |
| 483 | SLC2A1_2727 | GCATCTTCGAGAAGGCGGGGGTGNNNNNNNNCTTCAGCTTCCCGA TATCCGACGGTAGTGTGGAGAAGAAGGTCACCA |
| 484 | SLC2A1_2730 | GCCCCATCCCATGGTTCATCGTGGNNNNNNNNCTTCAGCTTCCCG ATATCCGACGGTAGTGTGCTGGCATGGCGGGTTGTG |
| 485 | SLC2A1_2733 | TTCCATCCCCTGGGGGCTNNNNNNNNCTTCAGCTTCCCGATATCC GACGGTAGTGTTGTGCTCCTGGTTCTGTTCTTCATCTT |
| 486 | SLC2A3_2804 | CACTGGGGTCATCAATGCTCCTGAGAANNNNNNNNCTTCAGCTTC CCGATATCCGACGGTAGTGTATCACCCCTAGATCTTTC |
| 487 | SLC2A3_2808 | GCCCTGCGGGGTGCCTTTGNNNNNNNNCTTCAGCTTCCCGATATC CGACGGTAGTGTGGCTGCTTTATGGGACTGTGT |
| 488 | SLC2A3_2812 | CCATTGTGCTCCAGCTCTCTCAGCAGNNNNNNNNCTTCAGCTTCC CGATATCCGACGGTAGTGTCACCCAGGATGTATCCCAA |
| 489 | SLC2A3_2816 | ACTCTTCAGCCAGGGCCCNNNNNNNNCTTCAGCTTCCCGATATCC GACGGTAGTGTCGCTCATGACTGTTTCTTTGTTATTAA |
| 490 | SLC2A3 2819 | GCCTGCTAAGGAGACCACCACCAANNNNNNNNCTTCAGCTTCCCG ATATCCGACGGTAGTGTTTACCTTCTTCAAAGTCCCTG |
| 491 | SLC5A1_1305 | CATTTTCACCAAGATCTCGGCNNNNNNNNCTTCAGCTTCCCGATA TCCGACGGTAGTGTTTGTGCTGGGCTGGCTGTTTGTCC |
| 492 | SLC5A1_1309 | CATCTTCCGAGATCCCCTCACNNNNNNNNCTTCAGCTTCCCGATA TCCGACGGTAGTGTTTGCTTTTCACGAAGTGGGAGGCT |
| 493 | SLC5A1_1313 | GTCATGCTGGCCTCCCTCATGAGCTNNNNNNNNCTTCAGCTTCCC GATATCCGACGGTAGTGTATTGCCTGTGTCGTCCCTTC |
| 494 | SLC5A1_1317 | AGCCCAGCAACTGTCCCACNNNNNNNNCTTCAGCTTCCCGATATC CGACGGTAGTGTCTGTCTTCCTGCTTGCTATTTTCTGG |
| 495 | SLC5A1_1321 | CATCCTGGTGACCGTGGCTGTCTTTTNNNNNNNNCTTCAGCTTCC CGATATCCGACGGTAGTGTTTTTGTGGGCTAGAGCAGC |
| 496 | SLC5A5_0875 | GCCAGCAAGCAGATCACTGCAGNNNNNNNNCTTCAGCTTCCCGAT ATCCGACGGTAGTGTTCTTTCAACATGACAGATGCCGC |
| 497 | SLC5A5_0877 | CCCAGTTTTGGCTCTACTTTGCAGGGNNNNNNNNCTTCAGCTTCC CGATATCCGACGGTAGTGTGGGAGTTCTGTCCTTCTGG |
| 498 | SLC5A5_0879 | GCTTTAACGTGTCTGTGCAGGGTNNNNNNNNCTTCAGCTTCCCGA TATCCGACGGTAGTGTTCTTGATTTTGCCCGTACCCGT |
| 499 | SLC5A5_0881 | GCATGATGATGCAGTCAGGGCGCAAAGNNNNNNNNCTTCAGCTTC CCGATATCCGACGGTAGTGTGACACTGCAAAGGGAA |
| 500 | SLC5A5_0883 | CATAAGTTATTTCCTAGGATTTTTCCCCCNNNNNNNNCTTCAGCT TCCCGATATCCGACGGTAGTGTCTGGCGGAAGATTGCT |
| 501 | SLC7A1_2222 | CACTTTTGATCTGGTGGCCCTCNNNNNNNNCTTCAGCTTCCCGAT ATCCGACGGTAGTGTTTCCCGTCATATTCCAGCTCTG |
| 502 | SLC7A1_2226 | CCCCGGCGTGCTGGCTGAAAACNNNNNNNNCTTCAGCTTCCCGAT ATCCGACGGTAGTGTCGGCTGGAACTTAATCCTCTCCT |
| 503 | SLC7A1_2232 | GCTGGGAAGGTGCCAAGTACGNNNNNNNNCTTCAGCTTCCCGATA TCCGACGGTAGTGTGTGGGGATCGTGGCGTCCCTCTTG |
| 504 | SLC7A1_2236 | GGCAAGCACCAATGATTCCCAGCTNNNNNNNNCTTCAGCTTCCCG ATATCCGACGGTAGTGTCTCTCCTGGCTTACTCGTTG |
| 505 | SLC7A1_2240 | CGTGAACGTCTATCTCATGATGCNNNNNNNNCTTCAGCTTCCCGA TATCCGACGGTAGTGTTTTCTGCTCGCAGGGTCTGCCC |
| 506 | SLC9A1_2249 | TCCCCTCACAGACTCTTCCACCANNNNNNNNCTTCAGCTTCCCGA TATCCGACGGTAGTGTCAGTGACAGCCCCAGCTCCCA |
| 507 | SLC9A1_2255 | GCCTCATGAAGATAGGTTTCCATGTGNNNNNNNNCTTCAGCTTCC CGATATCCGACGGTAGTGTCCGCCCTGTTAATCATTCC |
| 508 | SLC9A1_2261 | GCGGGGTGCTTGTGGGCGTGGTNNNNNNNNCTTCAGCTTCCCGAT ATCCGACGGTAGTGTGGGAGTCCTTGCTCAATGA |
| 509 | SLC9A1_2267 | GCCCCTGGTAGACCTGTTGGCTGTNNNNNNNNCTTCAGCTTCCCG ATATCCGACGGTAGTGTGAGGGGCCATCGCCTTCTCT |
| 510 | SLC9A1_2273 | GCAGCTGGAGCAGAAGATCAACAACTACCNNNNNNNNCTTCAGCT TCCCGATATCCGACGGTAGTGTATCCTGAGGAACAACT |
| 511 | SLCA12_1015 | GCCTGTGTGACAAGCTGGGGAAGAATNNNNNNNNCTTCAGCTTCC CGATATCCGACGGTAGTGTGGGAGGAGAGGTTAGATG |
| 512 | SLCA12_1019 | GCTGGGGCCTGGGAAGAAGAATGATNNNNNNNNCTTCAGCTTCCC GATATCCGACGGTAGTGTTAAGGCTAGTGGCCGCTTGG |
| 513 | SLCA12_1023 | GCTGATGGTGGATTTCTTCAACATTTTGNNNNNNNNCTTCAGCTT CCCGATATCCGACGGTAGTGTGGAGGAGACTAAGATGG |
| 514 | SLCA12_1027 | CGTCCTTCCTGTTGGAGCNNNNNNNNCTTCAGCTTCCCGATATCC GACGGTAGTGTCCTTCTCCTTTTTTGCTGGCATTTTCC |
| 515 | SLCA12_1031 | CGAGTGCATGAAGATATTGAAATGACCAANNNNNNNNCTTCAGCT TCCCGATATCCGACGGTAGTGTTGGCTGTGGACTGGCT |
| 516 | SOD_0414 | GGACTGACTGAAGGCCTGCATGGATNNNNNNNNCTTCAGCTTCCC GATATCCGACGGTAGTGTGTGGCCTAGCGAGTTATG |
| 517 | SOD_0415 | GTGGGCCAAAGGATGAAGAGAGGCATNNNNNNNNCTTCAGCTTCC CGATATCCGACGGTAGTGTGGTGTGGGGAAGCATTA |
| 518 | SOD_0416 | TCTCACTCTCAGGAGACCATTGCNNNNNNNNCTTCAGCTTCCCGA TATCCGACGGTAGTGTTCCTCACTTTAATCCTCTATCC |
| 519 | SOD_0417 | GTACAAAGACAGGAAACGCTGGAAGTCNNNNNNNNCTTCAGCTTC CCGATATCCGACGGTAGTGTGGTGTGGCCGATGTGTCT |
| 520 | SOD_0418 | CCCTTGGATGTAGTCTGAGGCCCCTTANNNNNNNNCTTCAGCTTC CCGATATCCGACGGTAGTGTGCACACTGGTGGTCCATG |
| 521 | SOD2_0438 | GCCCTGGAACCTCACATCAACGCGNNNNNNNNCTTCAGCTTCCCG ATATCCGACGGTAGTGTTAGCACCAGCACTAGCAGCA |
| 522 | SOD2_0439 | GCGTTGGCCAAGGGAGATGTTACAGCNNNNNNNNCTTCAGCTTCC CGATATCCGACGGTAGTGTACCTGCCCTACGACTAC |
| 523 | SOD2_0441 | GCTCAGGTTGGGGTTGGCTTGGTTTNNNNNNNNCTTCAGCTTCCC GATATCCGACGGTAGTGTGGACAAACCTCAGCCCTAA |
| 524 | SOD2_0443 | GGGAGAATGTAACTGAAAGATACATGGCTNNNNNNNNCTTCAGCT TCCCGATATCCGACGGTAGTGTACTGCAAGGAACAACA |
| 525 | SOD2_0444 | GCTGAGTATGTTAAGCTCTTTATGACTGNNNNNNNNCTTCAGCTT CCCGATATCCGACGGTAGTGTCGCTTACTACCTTCAGT |
| 526 | TAL_2770 | GAAGATTCCGGGCCGAGTATCCNNNNNNNNCTTCAGCTTCCCGAT ATCCGACGGTAGTGTGATGCCCGCTTACCAGGA |
| 527 | TAL_2772 | TCGAGGAGCAGCACGGCATCCACNNNNNNNNCTTCAGCTTCCCGA TATCCGACGGTAGTGTTGATAAAGATGCGATGGTGGCC |
| 528 | TAL_2774 | GTTTAGCTACAAAACCATTGTCATGGGCNNNNNNNNCTTCAGCTT CCCGATATCCGACGGTAGTGTCATCTCCCCATTTGTT |
| 529 | TAL_2776 | CCACCTGGATGAGAAGTCTTTCCGNNNNNNNNCTTCAGCTTCCCG ATATCCGACGGTAGTGTAAGCACTGGCCGGCTGTGAC |
| 530 | TAL_2778 | GAGGCTGGACTCCAGATCTGCACCGNNNNNNNNCTTCAGCTTCCC GATATCCGACGGTAGTGTGAGGACCAGATGGCTGTGG |
| 531 | TIGAR_3037 | CATGAGGACAAAGCAGACCATGCATGGNNNNNNNNCTTCAGCTTC CCGATATCCGACGGTAGTGTAGGAGAAAATAATCCAAG |
| 532 | TIGAR_3039 | CGGAGGAGAGACGCTGGACCAGGTGAAANNNNNNNNCTTCAGCTT CCCGATATCCGACGGTAGTGTGACGGTAAAGTATGAC |
| 533 | TIGAR_3041 | GGATTAGCAGCCAGTGTCTTAGTTGTGNNNNNNNNCTTCAGCTTC CCGATATCCGACGGTAGTGTAGCGGATCAAAAAGAACA |
| 534 | TIGAR_3043 | GAGGAAGGAAGAGAAGTTAAACCAACGGTNNNNNNNNCTTCAGCT TCCCGATATCCGACGGTAGTGTTGAGAAGTCTGTTTGA |
| 535 | TP53I3_2466 | GTGAAGTCCTCCTGAAGGTGGCNNNNNNNNCTTCAGCTTCCCGAT ATCCGACGGTAGTGTCTGCCCTGTCCTGTCCTGCCCT |
| 536 | TP53I3_2467 | GCAACATTTTGGGACTTGAGGCATCTGGNNNNNNNNCTTCAGCTT CCCGATATCCGACGGTAGTGTTGAAGGAGGTGGCCAA |
| 537 | TP53I3_2468 | GCCATGGCTCTGCTCCCCGGTGGNNNNNNNNCTTCAGCTTCCCGA TATCCGACGGTAGTGTACTTAATGCAGAGACAAGGCCA |
| 538 | TP53I3_2470 | GCTAATCCATGCAGGACTGAGTGGTGTNNNNNNNNCTTCAGCTTC CCGATATCCGACGGTAGTGTAAGGGCTCCTCATGCCTA |
| 539 | TP53I3_2471 | GCTTCAAATGGCAGAAAAGCTTGGAGNNNNNNNNCTTCAGCTTCC CGATATCCGACGGTAGTGTGGAAATGTTCAGGCTGGAG |
| 540 | TP53I3_2472 | GCTGGAGTTAATCTTATTCTAGACTGCNNNNNNNNCTTCAGCTTC CCGATATCCGACGGTAGTGTTATTCCTCTGGTCACAGC |
| 541 | TP53I3_2473 | GTCGATGGGTTCTCTATGGTCTGATGGGNNNNNNNNCTTCAGCTT CCCGATATCCGACGGTAGTGTACAAAAAAGAGGATTTC |
| 542 | TP53I3_2474 | GCTGAGGTCTAGGGACAATAAGTACANNNNNNNNCTTCAGCTTCC CGATATCCGACGGTAGTGTAAGAACGTCAACTGCCTGG |
| 543 | TP53I3_2475 | AGGGCCCCCAACGTCTGCTNNNNNNNNCTTCAGCTTCCCGATATC CGACGGTAGTGTGGCCCCTGTTTTCAAAGCTACTTTTT |
| 544 | TP53I3_2476 | TCGTCCTGGAACTGCCCCAGTGAANNNNNNNNCTTCAGCTTCCCG ATATCCGACGGTAGTGTCAAATTCTGCCTCACTTCTCC |
| 545 | TRX_1250 | CTTCTCAGCCACGTGGTGTNNNNNNNNCTTCAGCTTCCCGATATC CGACGGTAGTGTTTTGGATCCATTTCCATCGGTCCTTA |
| 546 | TRX_1251 | GTTTTTTAAGAAGGGACAAAAGGTGGGTGNNNNNNNNCTTCAGCT TCCCGATATCCGACGGTAGTGTGCAGGTGATAAACTTG |
| 547 | TRX_1252 | GTTTTCTGAAAATATAACCAGCCATTGGCNNNNNNNNCTTCAGCT TCCCGATATCCGACGGTAGTGTAGAGTGTGAAGTCAAA |
| 548 | FGFR2_4 | GCCGTGATCAGTTGGACTAAGGATGNNNNNNNNCTTCAGCTTCCC GATATCCGACGGTAGTGTGTTTAGTTGAGGATACCACA |
| 549 | FGFR2_6 | CGATGGTGCGGAAGATTTTGTCAGTGAGANNNNNNNNCTTCAGCT TCCCGATATCCGACGGTAGTGTCCACGCCTAGAGACTC |
| 550 | FGFR2_8 | GCCGGTGTTAACACCACGGACAAANNNNNNNNCTTCAGCTTCCCG ATATCCGACGGTAGTGTGACGTAGAGTTTGTCTGCAAG |
| 551 | FGFR2_10 | ACTACCTGGAGATAGCCATTTACTGCATNNNNNNNNCTTCAGCTT CCCGATATCCGACGGTAGTGTTTTTGAGGACGCTGGGG |
| 552 | FGFR2_12 | GCAGTGTTAAAACATGAATGACTGTGTCNNNNNNNNCTTCAGCTT CCCGATATCCGACGGTAGTGTCGAACAGTATTCACCTA |
| 553 | VHL_20 | CGTGCTGCCCGTATGGCTCAACTNNNNNNNNCTTCAGCTTCCCGA TATCCGACGGTAGTGTAAGAGTCCGGCCCGGAGGAACT |
| 554 | VHL_21 | GCTCTTCAGAGATGCAGGGACACNNNNNNNNCTTCAGCTTCCCGA TATCCGACGGTAGTGTTCTTCTGCAATCGCAGTCCGCG |
| 555 | VHL_22 | GCGCCGAGGAGGAGATGGAGGNNNNNNNNCTTCAGCTTCCCGATA TCCGACGGTAGTGTAGAACTGGGACGAGGCCGAGG |
| 556 | VHL_24 | AGTCGGGCGCCGAGGAGTCCGNNNNNNNNCTTCAGCTTCCCGATA TCCGACGGTAGTGTGGCGTCCGGCCCGGGTGGTCTGG |
| 557 | VHL_25 | CTCAATGTTGACGGACAGCCTATTNNNNNNNNCTTCAGCTTCCCG ATATCCGACGGTAGTGTTACCCAACGCTGCCGCCTGG |
| 558 | VHL_26 | GTCCGGAGCCTAGTCAAGCCTGAGAATTANNNNNNNNCTTCAGCT TCCCGATATCCGACGGTAGTGTCGATGGGCTTCTGGTT |
| 559 | VHL_27 | GCGGCTGACACAGGAGCGCATTGCACATNNNNNNNNCTTCAGCTT CCCGATATCCGACGGTAGTGTAAAGAGCGATGCCTCCA |
| 560 | VHL_28 | GCTTTTGATGGTACTGATGAGTCTTGNNNNNNNNCTTCAGCTTCC CGATATCCGACGGTAGTGTTACGAAGATCTGGAAGACC |
| 561 | NTRK1 (TRKa)_33 | GATGTGCACGCCCGGCTGCAANNNNNNNNCTTCAGCTTCCCGATA TCCGACGGTAGTGTAACACGGAGGCAATCGACTGCATC |
| 562 | NTRK1 (TRKa)_36 | GATGGTGTACCTGGCGGGTCTGCATTTTNNNNNNNNCTTCAGCTT CCCGATATCCGACGGTAGTGTCTCAACCGCTTCCTCC |
| 563 | NTRK1 (TRKa)_50 | CATCGTGAAGAGTGGTCTCCGTTTCGTGGNNNNNNNNCTTCAGCT TCCCGATATCCGACGGTAGTGTATAGCCTCCACCACCT |
| 564 | NTRK1 (TRKa)_56 | GCAAAGGCTCTGGGCTCCAAGGCCANNNNNNNNCTTCAGCTTCCC GATATCCGACGGTAGTGTCAACAAATGTGGACGGAGAA |
| 565 | NTRK1 (TRKa)_59 | GCAGGGATATCTACAGCACCGACTNNNNNNNNCTTCAGCTTCCCG ATATCCGACGGTAGTGTTGGCTAGCCAGGTCGCTGCGG |
| 566 | PDGFRB_69 | AGTCAACACCTCCTCAACCNNNNNNNNCTTCAGCTTCCCGATATC CGACGGTAGTGTCTCTATACTGCCGTGCAGCCCAATG |
| 567 | PDGFRB_74 | CGGTGGTGTGGGAACGGATGTNNNNNNNNCTTCAGCTTCCCGATA TCCGACGGTAGTGTTGCTGTTGCTGTCTCTCCTGT |
| 568 | PDGFRB_92 | CGTGGCTTTTCTGGTATCTTTGAGGACAGNNNNNNNNCTTCAGCT TCCCGATATCCGACGGTAGTGTCATCTTTCTCACGGAA |
| 569 | PDGFRB_97 | GTCCGAGTGCTGGAGCTAAGTGAGAGCNNNNNNNNCTTCAGCTTC CCGATATCCGACGGTAGTGTCGGTATGTGTCAGAGCTG |
| 570 | PDGFRB_101 | GCCAATGGCATGGAGTTTCTGGCCTNNNNNNNNCTTCAGCTTCCC GATATCCGACGGTAGTGTCCAACTACATGGCCCCTTAC |
| 571 | ERBB2 (HER2)_118 | TTCGGCCCCAGCCCCCTTNNNNNNNNCTTCAGCTTCCCGATATCC GACGGTAGTGTTCCCCACACATGACCCCAGCCCTCTAC |
| 572 | ERBB2 (HER2)_123 | GCCCTGGGACCAGCTCTTTCNNNNNNNNCTTCAGCTTCCCGATAT CCGACGGTAGTGTACAATGGCGCCTACTCGCTGACCCT |
| 573 | ERBB2 (HER2)_131 | CACGATTTTGTGGAAGGACATCTTCNNNNNNNNCTTCAGCTTCCC GATATCCGACGGTAGTGTAACAATACCACCCCTGTCAC |
| 574 | ERBB2 (HER2)_136 | GCAAGAAGATCTTTGGGAGCCTGGCATNNNNNNNNCTTCAGCTTC CCGATATCCGACGGTAGTGTATGGAACACAGCGGTGTG |
| 575 | ERBB2 (HER2)_157 | GCTGGCTCCGATGTATTTGATGGTGNNNNNNNNCTTCAGCTTCCC GATATCCGACGGTAGTGTCTGGGGGCATGGTCCA |
| 576 | NTRK2 (TRKb)_172 | GCGAGAGCCCCACATGAGGAAGAACATCNNNNNNNNCTTCAGCTT CCCGATATCCGACGGTAGTGTAAACAGCCCTGGTACCA |
| 577 | NTRK2 (TRKb)_179 | GCAACCTGCAGCACATCAATTTTACCCNNNNNNNNCTTCAGCTTC CCGATATCCGACGGTAGTGTCAAACCAGAAAAGGTTAG |
| 578 | NTRK2 (TRKb)_184 | GCAGATCTCTTGTGTGGCGGAAAATCTNNNNNNNNCTTCAGCTTC CCGATATCCGACGGTAGTGTAGGTGATCCGGTTCCTAA |
| 579 | NTRK2 (TRKb)_189 | CGGGGACACCACGAACAGAAGTAATNNNNNNNNCTTCAGCTTCCC GATATCCGACGGTAGTGTGAGTATGGGAAGGATGAGAA |
| 580 | NTRK2 (TRKb)_194 | GCTGAATGCTATAACCTCTGTCCTGNNNNNNNNCTTCAGCTTCCC GATATCCGACGGTAGTGTATCCCCAGTACTTTGGCATC |
| 581 | PDGFRA_216 | GCACAACTGATCCCGAGACTCCTGTNNNNNNNNCTTCAGCTTCCC GATATCCGACGGTAGTGTGAATGAGCTTGAAGGCAGGC |
| 582 | PDGFRA_226 | GTAATAATGAAACTTCCTGGACTATTTNNNNNNNNCTTCAGCTTC CCGATATCCGACGGTAGTGTCATCCATTCTGGACTTG |
| 583 | PDGFRA_236 | CGACATCCAGAGATCACTCTATGATCGTCNNNNNNNNCTTCAGCT TCCCGATATCCGACGGTAGTGTAAAGCACACGGAGCTA |
| 584 | PDGFRA_241 | GTGGGTACCGGATGGCCAAGCCTGNNNNNNNNCTTCAGCTTCCCG ATATCCGACGGTAGTGTACAACCTCTACACCACA |
| 585 | PDGFRA_246 | CATCAAGAGAGAGGACGAGACCATNNNNNNNNCTTCAGCTTCCCG ATATCCGACGGTAGTGTCTACATCATTCCTCTGCCTGA |
| 586 | FGFR1_258 | CGTCAATGTTTCAGATGCTCTCCCCTCCNNNNNNNNCTTCAGCTT CCCGATATCCGACGGTAGTGTGAAAGCAACCGCACCC |
| 587 | FGFR1_259 | GCATCACAGGGGAGGAGGTGGANNNNNNNNCTTCAGCTTCCCGAT ATCCGACGGTAGTGTTGTGGAAGTGGAGTCCTTCCTGG |
| 588 | FGFR1_261 | TGGCAAAGAATTCAAACCNNNNNNNNCTTCAGCTTCCCGATATCC GACGGTAGTGTGCCCGTAGCTCCATATTGGACATCCCC |
| 589 | FGFR1_264 | CAGATAACACCAAACCAAACCNNNNNNNNCTTCAGCTTCCCGATA TCCGACGGTAGTGTTCTATGCTTGCGTAACCAGCAGCC |
| 590 | FGFR1_265 | CATCCCTCTGCGCAGACAGGTAANNNNNNNNCTTCAGCTTCCCGA TATCCGACGGTAGTGTTCATCTATTGCACAGGGGCCTT |
| 591 | VEGFA 121 | TGTGACAAGCCGAGGCGGTGAGCCNNNNNNNNCTTCAGCTTCCCG ATATCCGACGGTAGTGTTCCAACATCACCATGCAGATT |
| 592 | VEGFA 121b | TCTCTCACCAGGAAAGACTGATACNNNNNNNNCTTCAGCTTCCCG ATATCCGACGGTAGTGTTCCAACATCACCATGCAGATT |
| 593 | VEGFA 165 | GAGGCGGTGAGCCGGGCAGGAGGANNNNNNNNCTTCAGCTTCCCG ATATCCGACGGTAGTGTAGAGCGGAGAAAGCAT |
| 594 | VEGFA 165_165b | CCTGTGGGCCTTGCTCAGAGCNNNNNNNNCTTCAGCTTCCCGATA TCCGACGGTAGTGTAGTCCAACATCACCATGCAGATT |
| 595 | VEGFA 165b | CCACGCTGCCGCCACCACACCANNNNNNNNCTTCAGCTTCCCGAT ATCCGACGGTAGTGTTCCGCAGACGTGTAAATGTTCCT |
| 596 | VEGF 189_189b | GTTCGAGGAAAGGGAAAGGGGCAAAAACNNNNNNNNCTTCAGCTT CCCGATATCCGACGGTAGTGTCCATGCAGATTATGCG |
| 597 | VEGFA_ex1_5 (6) | GGCGTGAGCCCTCCCCCTTGGGANNNNNNNNCTTCAGCTTCCCGA TATCCGACGGTAGTGTAGCAAGAGCTCCAGAGAG |
| 598 | VEGFA_ex1_5 (7) | CGAAGTGGTGAAGTTCATGGATGTCTNNNNNNNNCTTCAGCTTCC CGATATCCGACGGTAGTGTCCTCCGAAACCATGAAC |
| 599 | VEGFA_ex1_5 (13) | CGTTTTAATTTATTTTTGCTTGCCNNNNNNNNCTTCAGCTTCCCG ATATCCGACGGTAGTGTGTTAGGTGGACCGGTCAGCGG |
| 600 | VEGFA_ex1_5 (14) | CACTGTGGATTTTGGAAACCAGCNNNNNNNNCTTCAGCTTCCCGA TATCCGACGGTAGTGTCCCTCTTCTTTTTTCTTAAACA |
| 601 | VEGFA_ex1_5 (15) | GGCGCTCGGAAGCCGGGCTCATGGANNNNNNNNCTTCAGCTTCCC GATATCCGACGGTAGTGTGCGCGGGGGAAGCCGAG |
| 602 | VEGFA_ex1_5 (16) | GCCTGGAGTGTGTGCCCACTGAGGAGNNNNNNNNCTTCAGCTTCC CGATATCCGACGGTAGTGTAGCTACTGCCATCCAA |
| 603 | VEGFA_ex1_5 (17) | CCTACAGCACAACAAATGTGAATGCAGACNNNNNNNNCTTCAGCT TCCCGATATCCGACGGTAGTGTGATGCGGGGGCTGCTG |
| 604 | VEGFA_ex1_5 (18) | CTGTGGGCCTTGCTCAGAGCGGANNNNNNNNCTTCAGCTTCCCGA TATCCGACGGTAGTGTCCAACATCACCATGCAGATTA |
| 605 | ADPGK_0002 | GCCAGAGCTGCCAGGCTCGGNNNNNNNNCTTCAGCTTCCCGATAT CCGACGGTAGTGTCGGAAGAGGCGCGGGCTAGG |
| 606 | ADPGK_0004 | CACCAGCCGAGTGTCTCTGAGGNNNNNNNNCTTCAGCTTCCCGAT ATCCGACGGTAGTGTATCCATTTCCACACGCTGGTCT |
| 607 | ADPGK _0011 | GTGGGGCCAGTTAAAAGCTCCNNNNNNNNCTTCAGCTTCCCGATA TCCGACGGTAGTGTGGTTCTTCTTTGCGGTCCAGTTGG |
| 608 | ADPGK_0015 | TTCTCACCCAGTCAGCCTCNNNNNNNNCTTCAGCTTCCCGATATC CGACGGTAGTGTTGACATCCCCACTGGTATTCCAGTTC |
| 609 | ADPGK_0017 | GCAACTGTGGATGGACACTGGGCCNNNNNNNNCTTCAGCTTCCCG ATATCCGACGGTAGTGTGGTGTTCCTGATGTGGG |
| 610 | AR_0041 | AGTCGGCCCTGGAGTGCCACCCCGAGANNNNNNNNCTTCAGCTTC CCGATATCCGACGGTAGTGTAGCAAGAGACTAGCCCCA |
| 611 | AR_0062 | TGGCGGCGGCGGCGGCGGCNNNNNNNNCTTCAGCTTCCCGATATC CGACGGTAGTGTAGCCTGCATGGCGCGGGTGCAGCGGG |
| 612 | AR_0068 | GCTTGTACACGTGGTCAAGTGGGNNNNNNNNCTTCAGCTTCCCGA TATCCGACGGTAGTGTGTCAGCCCATCTTTCTGAATGT |
| 613 | AR_0069 | GCTCATGGTGTTTGCCATGGGCTGGNNNNNNNNCTTCAGCTTCCC GATATCCGACGGTAGTGTTCTAGCCTCAATGAACTGGG |
| 614 | AR_0074 | CATGGTGAGCGTGGACTTTCCGGAAATGNNNNNNNNCTTCAGCTT CCCGATATCCGACGGTAGTGTAAAAGAAAAAATCCCAC |
| 615 | AR_0075 | CCACACCCAGTGAAGCATNNNNNNNNCTTCAGCTTCCCGATATCC GACGGTAGTGTGCTGCATCAGTTCACTTTTGACCTGCT |
| 616 | ARV7_ARV_0009 | GCATCTCAAAATGACCAGACCCTGNNNNNNNNCTTCAGCTTCCCG ATATCCGACGGTAGTGTGTGCGCCAGCAGAAATGAT |
| 617 | ARV12_ARV_0002 | GCAGAGATCATCTCTGTGCAAGTGNNNNNNNNCTTCAGCTTCCCG ATATCCGACGGTAGTGTAGAGACAGCTTGTACACGTGG |
| 618 | BRAF_0106 | CCCCAAATTCTCACCAGTCCNNNNNNNNCTTCAGCTTCCCGATAT CCGACGGTAGTGTCCAACTTGATTTGCTGTTTGTCTCC |
| 619 | BRAF_0112 | GACATGTGAATATCCTACTCTTCATGGGCNNNNNNNNCTTCAGCT TCCCGATATCCGACGGTAGTGTGGAACAGTCTACAAGG |
| 620 | BRAF_0115 | GCTACAGTGAAATCTCGATGGAGTGGGTCNNNNNNNNCTTCAGCT TCCCGATATCCGACGGTAGTGTACGACAGACTGCACAG |
| 621 | BRAF_0116 | CATACAGCTTTCAGTCAGATGTATATGCANNNNNNNNCTTCAGCT TCCCGATATCCGACGGTAGTGTATAGGTGATTTTGGTC |
| 622 | BRAF_0117 | CAAACATCAACAACAGGGACCAGNNNNNNNNCTTCAGCTTCCCGA TATCCGACGGTAGTGTGGATCCATTTTGTGGATGGCAC |
| 623 | CAT_0151 | CGGACATGGTCTGGGACTTCTGGAGCCTANNNNNNNNCTTCAGCT TCCCGATATCCGACGGTAGTGTGAAGATGGTAACTGGG |
| 624 | CAT_0153 | CCAGGGCATCAAAAACCTNNNNNNNNCTTCAGCTTCCCGATATCC GACGGTAGTGTGGTTTCTTTCTTGTTCAGTGATCGGGG |
| 625 | CAT_0155 | CACCAAGGTTTGGCCTCACAAGGACTANNNNNNNNCTTCAGCTTC CCGATATCCGACGGTAGTGTTGACTATGGCATCCGGG |
| 626 | CAT_0159 | CCTGAAGGATGCACAAATTTTCATCCAGNNNNNNNNCTTCAGCTT CCCGATATCCGACGGTAGTGTTCTGGAGAAGTGCGGAG |
| 627 | CAT_0161 | GCGGCAAGGGAGAAGGCAAATCTGTGAGGNNNNNNNNCTTCAGCT TCCCGATATCCGACGGTAGTGTCAAGAACTTCACTGAG |
| 628 | CD274_0182 | GAGGGCCCGGCTGTTGAAGGACCAGCTNNNNNNNNCTTCAGCTTC CCGATATCCGACGGTAGTGTCCCAGTAGAAAAACAA |
| 629 | CD274_0184 | TGGTTGTGGATCCAGTCACCTCTGNNNNNNNNCTTCAGCTTCCCG ATATCCGACGGTAGTGTAGATCACAGATGTGAAATTGC |
| 630 | CD274_0186 | GCACTTTTAGGAGATTAGATCCTGAGGANNNNNNNNCTTCAGCTT CCCGATATCCGACGGTAGTGTAAGCAGTGACCATCAAG |
| 631 | CD274_0188 | GGCATCCAAGATACAAACTCAAAGAAGCNNNNNNNNCTTCAGCTT CCCGATATCCGACGGTAGTGTCACATCCTCCAAATGAA |
| 632 | CD274_0189 | CTTCTGATCTTCAAGCAGGGATTCTCNNNNNNNNCTTCAGCTTCC CGATATCCGACGGTAGTGTCTGACATTCATCTTCCGTT |
| 633 | CTLA4_0199 | GCAAAGCAATGCACGTGGCCCAGCCTGNNNNNNNNCTTCAGCTTC CCGATATCCGACGGTAGTGTAACACCGCTCCCATAAA |
| 634 | CTLA4_0203 | CTTCCTAGATGATTCCATCTGCACGGNNNNNNNNCTTCAGCTTCC CGATATCCGACGGTAGTGTGCTTTGTGTGTGAGTATGC |
| 635 | CTLA4_0205 | TTGATCCAGAACCGTGCCCAGATTCNNNNNNNNCTTCAGCTTCCC GATATCCGACGGTAGTGTTCAAGTGAACCTCACTATCC |
| 636 | CTLA4_0207 | CCCCCAACAGAGCCAGAANNNNNNNNCTTCAGCTTCCCGATATCC GACGGTAGTGTGACTTCCTCCTCTGGATCCTTGCAGC |
| 637 | FBP1_0214 | CCCAGCTGCTCAACTCGCTCNNNNNNNNCTTCAGCTTCCCGATAT CCGACGGTAGTGTTTGCACCTGCAGCCCCGCGCTCT |
| 638 | FBP1_0222 | GATCCCCTTGATGGATCTTCCNNNNNNNNCTTCAGCTTCCCGATA TCCGACGGTAGTGTGTCCTCTCCAACGACCTGGTTATG |
| 639 | FBP1_0224 | GTCCTTGCCATGGACTGTGGGGTNNNNNNNNCTTCAGCTTCCCGA TATCCGACGGTAGTGTCGTTGGAACCATTTTTGGCATC |
| 640 | FBP1_0226 | GCTCCTTATGGGGCCCGGTATGTGGGNNNNNNNNCTTCAGCTTCC CGATATCCGACGGTAGTGTGGACAAGGATGTGAAGATA |
| 641 | FBP1_0228 | CCACTGGGAAGGAGGCCGTGTTAGACGTNNNNNNNNCTTCAGCTT CCCGATATCCGACGGTAGTGTTCTGGTCTACGGAGGG |
| 642 | FOLH1_0243 | CCACCTCCTCCAGGATATGAANNNNNNNNCTTCAGCTTCCCGATA TCCGACGGTAGTGTTTTGGCCTGGATTCTGTTGAGCT |
| 643 | FOLH1_0247 | CCTCTCACACCAGGTTACCCAGCAANNNNNNNNCTTCAGCTTCCC GATATCCGACGGTAGTGTTCATTCTCTACTCCGACCCT |
| 644 | FOLH1_0251 | GTGGAGCAGCTGTTGTTCATGAAATTGTGNNNNNNNNCTTCAGCT TCCCGATATCCGACGGTAGTGTAATGAAGTGACAAGAA |
| 645 | FOLH1_0255 | GGGATCTGGAAATGATTTTGAGGTGTTNNNNNNNNCTTCAGCTTC CCGATATCCGACGGTAGTGTACACAACCTAACAAAAGA |
| 646 | FOLH1_0259 | GTTCAGTGAGAGACTCCAGGACTTTGACNNNNNNNNCTTCAGCTT CCCGATATCCGACGGTAGTGTAGAAAGTATGCTGACAA |
| 647 | HP16-E7_0296 | GGTTCTAAAACGAAAGTATTTGGGTAGTCNNNNNNNNCTTCAGCT TCCCGATATCCGACGGTAGTGTCAGGTGAAGATTTGGT |
| 648 | HP16-E2_0316 | TATTAACCACCAGGTGGTGCCAACACTGGNNNNNNNNCTTCAGCT TCCCGATATCCGACGGTAGTGTACAAAATACTAACACA |
| 649 | HP16-E2_0318 | GGATATACAGTGGAAGTGCAGTTTGATGGNNNNNNNNCTTCAGCT TCCCGATATCCGACGGTAGTGTTGAACTGCAACTAACG |
| 650 | HP16-E2_0320 | GTAAAAATAAAGTATGGGAAGTTCATGCGNNNNNNNNCTTCAGCT TCCCGATATCCGACGGTAGTGTATTTGTGAAGAAGCAT |
| 651 | HP16-E2_0322 | AGCCAGACACCGGAAACCCCNNNNNNNNCTTCAGCTTCCCGATAT CCGACGGTAGTGTAGCAGCAACGAAGTATCCTCTCCTG |
| 652 | HP16-E2_0324 | GCATTGTACATTGTATACTGCAGTGTCGTNNNNNNNNCTTCAGCT TCCCGATATCCGACGGTAGTGTTCCTCACTGCATTTAA |
| 653 | HP16-E6_0369 | GTTACTGCGACGTGAGGTATATGACTNNNNNNNNCTTCAGCTTCC CGATATCCGACGGTAGTGTACCAAAAGAGAACTGCAA |
| 654 | HP16-E6_0370 | GTTTTATTCTAAAATTAGTGAGTATAGACNNNNNNNNCTTCAGCT TCCCGATATCCGACGGTAGTGTTAGAATGTGTGTACTG |
| 655 | HP16-E6_0371 | CGTTGTGTGATTTGTTAATTAGGTGTATTNNNNNNNNCTTCAGCT TCCCGATATCCGACGGTAGTGTAGAGATGGGAATCCAT |
| 656 | HP16-E6_0372 | GTGGACCGGTCGATGTATGTCTNNNNNNNNCTTCAGCTTCCCGAT ATCCGACGGTAGTGTGAACAGCAATACAACAAAC |
| 657 | HP16-E7_0375 | CGTAGACATTCGTACTTTGGAAGACCTGNNNNNNNNCTTCAGCTT CCCGATATCCGACGGTAGTGTGAGGAGGAGGATGAAA |
| 658 | IGF1R_0464 | GCGGGGTGGGGGGGGAGAGAGAGTTTTNNNNNNNNCTTCAGCTTC CCGATATCCGACGGTAGTGTACAGCCTTACGCCCACA |
| 659 | IGF1R_0467 | CATTACTCGGGGGGCCATCAGGATTGNNNNNNNNCTTCAGCTTCC CGATATCCGACGGTAGTGTAGAGCCTCGGAGACCT |
| 660 | IGF1R_0489 | GCTCAGATGCTCCAAGGATGCACCANNNNNNNNCTTCAGCTTCCC GATATCCGACGGTAGTGTAGGAGTGCCCCTCGGGCTT |
| 661 | IGF1R_0505 | TCTCTCTCTGGGAATGGGTCGTGGACNNNNNNNNCTTCAGCTTCC CGATATCCGACGGTAGTGTAAAATACGGATCACAAG |
| 662 | IGF1R_0513 | GGTATGACGCGAGATATCTATGAGACAGNNNNNNNNCTTCAGCTT CCCGATATCCGACGGTAGTGTAGATGGCCGGAGAGAT |
| 663 | KDR_0549 | GCCCAATAATCAGAGTGGCAGTGNNNNNNNNCTTCAGCTTCCCGA TATCCGACGGTAGTGTCTGTGGGTTTGCCTAGTGTTTC |
| 664 | KDR_0557 | CCTGTGCAGCATCCAGTGGGCTGATGANNNNNNNNCTTCAGCTTC CCGATATCCGACGGTAGTGTCGAAGCATCAGCATAAGA |
| 665 | KDR_0565 | GCAGGAGAGCGTGTCTTTGTGGTGNNNNNNNNCTTCAGCTTCCCG ATATCCGACGGTAGTGTGGCAAATGTGTCAGCTTTG |
| 666 | KDR_0581 | GTGACTTTGGCTTGGCCCGGGATNNNNNNNNCTTCAGCTTCCCGA TATCCGACGGTAGTGTGAGCATCTCATCTGTTACAGCT |
| 667 | KDR_0589 | GCAGGGAGTCTGTGGCATCTGAAGGCTCNNNNNNNNCTTCAGCTT CCCGATATCCGACGGTAGTGTAAAAGTAATCCCAGATG |
| 668 | KLK3_0638 | CAGTGTGTGGACCTCCATGTTATTTCCNNNNNNNNCTTCAGCTTC CCGATATCCGACGGTAGTGTGAGCTCACGGATGCTGTG |
| 669 | KLK3_0643 | CCTGAAGAATCGATTCCTCAGGCCAGGTGNNNNNNNNCTTCAGCT TCCCGATATCCGACGGTAGTGTACTGCATCAGGAACAA |
| 670 | KLK3_0645 | GCTTCAAGGTATCACGTCATGGGGCAGNNNNNNNNCTTCAGCTTC CCGATATCCGACGGTAGTGTTGACGTGTGTGCGCAAGT |
| 671 | KLK3_0646 | GTGGATCAAGGACACCATCGTGGCCAANNNNNNNNCTTCAGCTTC CCGATATCCGACGGTAGTGTGGGTGATTCTGGGGGC |
| 672 | KLK3_0647 | CTCAAGCCTCCCCAGTTCTACNNNNNNNNCTTCAGCTTCCCGATA TCCGACGGTAGTGTCCTTCCCTGTACACCAAGGTGGTG |
| 673 | KRAS_0653 | GATCCAACAATAGAGGATTCCTACAGGANNNNNNNNCTTCAGCTT CCCGATATCCGACGGTAGTGTAGGTGCGGGAGAGAGG |
| 674 | KRAS_0654 | GCAGGTCAAGAGGAGTACAGTGCNNNNNNNNCTTCAGCTTCCCGA TATCCGACGGTAGTGTGAGTGCCTTGACGATACAG |
| 675 | KRAS_0655 | CATTTGAAGATATTCACCATTATAGAGNNNNNNNNCTTCAGCTTC CCGATATCCGACGGTAGTGTGAGAAACCTGTCTCTTGG |
| 676 | KRAS_0656 | GTGATTTGCCTTCTAGAACAGNNNNNNNNCTTCAGCTTCCCGATA TCCGACGGTAGTGTGGGAGGGCTTTCTTTGTGTATTTG |
| 677 | KRAS_0657 | GTGGAGGATGCTTTTTATACATTGGTGAGNNNNNNNNCTTCAGCT TCCCGATATCCGACGGTAGTGTGGTCCTAGTAGGAAAT |
| 678 | KRAS_0658 | GCATTATAATGTAATCTGGGTGTTGATGNNNNNNNNCTTCAGCTT CCCGATATCCGACGGTAGTGTAGCAAAGACAAGACAGA |
| 679 | KRAS_0659 | GCAAAGATGGTAAAAAGAAGAAAAAGAAGNNNNNNNNCTTCAGCT TCCCGATATCCGACGGTAGTGTGCAAAGAAGAAAAGAC |
| 680 | PDCD1_0668 | CAAAGAGAGCCTGCGGGCAGANNNNNNNNCTTCAGCTTCCCGATA TCCGACGGTAGTGTGGACTGCCGCTTCCGTGTC |
| 681 | PDCD1_0670 | GGACACTGCTCTTGGCCCCTCTNNNNNNNNCTTCAGCTTCCCGAT ATCCGACGGTAGTGTTGCCCTGTGTCCCTGAGCAGAC |
| 682 | PDCD1_0671 | TACCGCATGAGCCCCAGCNNNNNNNNCTTCAGCTTCCCGATATCC GACGGTAGTGTTTCTTAGACTCCCCAGACAGGCCCT |
| 683 | PDCD1_0673 | GGAGGACCCCTCAGCCGTGCCTGTGTNNNNNNNNCTTCAGCTTCC CGATATCCGACGGTAGTGTTGGTGGTTGGTGTCGTGG |
| 684 | PDCD1_0682 | CCAGCCGGCCAGTTCCAAACCNNNNNNNNCTTCAGCTTCCCGATA TCCGACGGTAGTGTGGCACCTACCTCTGTGGGGC |
| 685 | TP53_0689 | TCTGGCCCCTCCTCAGCATCTTNNNNNNNNCTTCAGCTTCCCGAT ATCCGACGGTAGTGTCTGTGGGTTGATTCCACACCCCC |
| 686 | TP53_0690 | CCCCTGCACCAGCCCCCTNNNNNNNNCTTCAGCTTCCCGATATCC GACGGTAGTGTTGGATGATTTGATGCTGTCCCCGGACG |
| 687 | TP53_0697 | GCCTGAGGTTGGCTCTGACTGTNNNNNNNNCTTCAGCTTCCCGAT ATCCGACGGTAGTGTGAGCGCTGCTCAGATAGCGATGG |
| 688 | TP53_0699 | CGGCGCACAGAGGAAGAGAATCTCNNNNNNNNCTTCAGCTTCCCG ATATCCGACGGTAGTGTAGTTCCTGCATGGGCGGCATG |
| 689 | TP53_0703 | TCCCACCCCCATCTCTCCCNNNNNNNNCTTCAGCTTCCCGATATC CGACGGTAGTGTAGCAGGGCTCACTCCAGCCACCT |
| 690 | CS_2331 | GCTTCCTCCACGAATTTGAAAGACNNNNNNNNCTTCAGCTTCCCG ATATCCGACGGTAGTGTTCTCTCCCTTTCTTACCTCCC |
| 691 | CS_2332 | GCAACATGGCAAGACGGTGGTGGGCCAANNNNNNNNCTTCAGCTT CCCGATATCCGACGGTAGTGTAACCAAGAATGCATCT |
| 692 | CS_2333 | GTTCTTGATCCTGATGAGGGCATCCGTTNNNNNNNNCTTCAGCTT CCCGATATCCGACGGTAGTGTAAGGAGCAGGCCAGAA |
| 693 | CS_2334 | GCCTGAGGGCTTATTTTGGCTGCTGGNNNNNNNNCTTCAGCTTCC CGATATCCGACGGTAGTGTGGCATGAAGGGATTGGT |
| 694 | CS_2335 | CCCATGTGGTCACCATGCTGGACAACNNNNNNNNCTTCAGCTTCC CGATATCCGACGGTAGTGTGGCTAAGGGTGGGGAAG |
| 695 | CS_2336 | GCCCGAGCATATGCACAGGGTATNNNNNNNNCTTCAGCTTCCCGA TATCCGACGGTAGTGTAAGAGTGGGCAAAGAGGG |
| 696 | CS_2337 | GCTACCTTGTGTTGCAGCAAAGNNNNNNNNCTTCAGCTTCCCGAT ATCCGACGGTAGTGTGTCTCAGCTCAGTGCAGCTGTT |
| 697 | CS_2338 | GGACTGGTCTCACAATTTCACCAACATGNNNNNNNNCTTCAGCTT CCCGATATCCGACGGTAGTGTGTACTGGGAGTTGATTT |
| 698 | CS_2339 | GTGACCATGAGGGTGGCAATGTAAGTGNNNNNNNNCTTCAGCTTC CCGATATCCGACGGTAGTGTAGAGAAGGCAGCGGTA |
| 699 | CS_2340 | GCCTCTCCATGGACTGGCAAATCAGGAAGNNNNNNNNCTTCAGCT TCCCGATATCCGACGGTAGTGTTACCTCACCATCCACA |
| 700 | CS_2341 | GTTACGAGACTACATCTGGAACACACTCANNNNNNNNCTTCAGCT TCCCGATATCCGACGGTAGTGTCTGTCCTTTGCAGCAG |
| 701 | CS_2342 | GCGATATACCTGTCAGCGAGAGTTTGCNNNNNNNNCTTCAGCTTC CCGATATCCGACGGTAGTGTAGCTGCAGAAGGAAGTTG |
| 702 | CS_2343 | GTGCCCAATGTCCTCTTAGAGCNNNNNNNNCTTCAGCTTCCCGAT ATCCGACGGTAGTGTGTTGTTCCAGGCTATGGCCATGC |
| 703 | CS_2344 | GATGAATTACTACACGGTCCTGTTTGGGNNNNNNNNCTTCAGCTT CCCGATATCCGACGGTAGTGTGTTGGTTGCTCAGCTGT |
| 704 | CS_2345 | GAAAGGCCCAAGTCCATGAGCACAGANNNNNNNNCTTCAGCTTCC CGATATCCGACGGTAGTGTGTGGGGTGCTGCTCCAGT |
| 705 | CS_2346 | GAGACTGGGTGAAAGTGACTACCAGAAAGNNNNNNNNCTTCAGCT TCCCGATATCCGACGGTAGTGTGCATTGGGTGTACTGG |
| 706 | D2HGDH_3222 | GTCCCCGTCTTTGACGAGATCATCCTNNNNNNNNCTTCAGCTTCC CGATATCCGACGGTAGTGTGCTGTAGCAAGGTGCTGCT |
| 707 | D2HGDH_3223 | GCTGAGCCGGTATGTGGAGGAANNNNNNNNCTTCAGCTTCCCGAT ATCCGACGGTAGTGTAGGCATGGTGGGTGGCAG |
| 708 | D2HGDH_3224 | GCTGGAGGCCTGCGGTTTCTTNNNNNNNNCTTCAGCTTCCCGATA TCCGACGGTAGTGTAATTCTGGTTTGCCAGGCGGGCTG |
| 709 | D2HGDH_3225 | GGAAGGACAACACGGGCTATGACCTGAAGNNNNNNNNCTTCAGCT TCCCGATATCCGACGGTAGTGTAAACGTGGCAACCAAC |
| 710 | D2HGDH_3226 | GCTGTGAACGTGGCTTTCCTCNNNNNNNNCTTCAGCTTCCCGATA TCCGACGGTAGTGTTGTCCTGGACTGCCTGACCTCCCT |
| 711 | D2HGDH_3227 | GCATTCGAGTTCATGGATGCTGTGTGNNNNNNNNCTTCAGCTTCC CGATATCCGACGGTAGTGTCGGTGTCCATCTTGTGTCC |
| 712 | D2HGDH_3228 | GCTCCAACGCAGGCCATGACNNNNNNNNCTTCAGCTTCCCGATAT CCGACGGTAGTGTGGGGATGCTGGGTGAGATCCTGTCT |
| 713 | D2HGDH_3229 | GCCACCGACCAGAGGAAAGTCAANNNNNNNNCTTCAGCTTCCCGA TATCCGACGGTAGTGTCGGTGCAAGAGAGTCCGTTTT |
| 714 | D2HGDH_3230 | TACAAGTACGACCTCTCCCTCCNNNNNNNNCTTCAGCTTCCCGAT ATCCGACGGTAGTGTTTCCTGGAGCACGCGCTGGGCTC |
| 715 | D2HGDH_3231 | GCCAAGCACGTGGTGGGCTATGNNNNNNNNCTTCAGCTTCCCGAT ATCCGACGGTAGTGTTGGGCCCTGAGGGAAAGGATCA |
| 716 | D2HGDH_3218 | GCACGGAGTGGGCTTCAGGAAGAGGGANNNNNNNNCTTCAGCTTC CCGATATCCGACGGTAGTGTACCTTGGAGATGGTAACC |
| 717 | FH_0120 | GTATTATGGCGCCCAGACCGTGNNNNNNNNCTTCAGCTTCCCGAT ATCCGACGGTAGTGTTTCGGCTCCCGGCTTGGGTG |
| 718 | FH_0121 | GAAGCGAGCGGCCGCTGAAGTAAACCNNNNNNNNCTTCAGCTTCC CGATATCCGACGGTAGTGTGGTGAACTAAAGGTGCC |
| 719 | FH_0122 | CATTTTCCTCTCGTGGTATGGCAGACTGGNNNNNNNNCTTCAGCT TCCCGATATCCGACGGTAGTGTAAGCTTTTGGCATCTT |
| 720 | FH_0123 | GTGAACTTGGCAGCAAGATACCTGTGCNNNNNNNNCTTCAGCTTC CCGATATCCGACGGTAGTGTGAGGTAGCTGAAGGTAAA |
| 721 | FH_0124 | GCTGCAATAGAAGTTCATGAAGTACTGTNNNNNNNNCTTCAGCTT CCCGATATCCGACGGTAGTGTAGCAATAGAGCAATTGA |
| 722 | FH_0125 | CGTACTCATACTCAGGATGCTGTTCCNNNNNNNNCTTCAGCTTCC CGATATCCGACGGTAGTGTTTCCCACAGCAATGCACAT |
| 723 | FH_0126 | GCCAAGAATCTATGAGCTCGCAGCTGGAGNNNNNNNNCTTCAGCT TCCCGATATCCGACGGTAGTGTGCACAGATCATCAAGA |
| 724 | FH_0127 | GTGGCTGCACTTACAGGCTTGCCTTTTGNNNNNNNNCTTCAGCTT CCCGATATCCGACGGTAGTGTAAATATGCAATGACAAG |
| 725 | FH_0128 | GCCTGCAGTCTGATGAAGATAGCAAATGNNNNNNNNCTTCAGCTT CCCGATATCCGACGGTAGTGTGAAAAGGTTGCTGCAAA |
| 726 | FH_0129 | ACCAGGAAGCAGTATCATGCCAGGCAAGNNNNNNNNCTTCAGCTT CCCGATATCCGACGGTAGTGTGGTTGAGCTCAGTGGAG |
| 727 | FH_0130 | GTTGCTGTCACTGTCGGAGGCAGCAANNNNNNNNCTTCAGCTTCC CGATATCCGACGGTAGTGTGGTCAGGTCTGGGAGA |
| 728 | FH_0131 | GCTGCTGGGGGATGCTTCAGTTTNNNNNNNNCTTCAGCTTCCCGA TATCCGACGGTAGTGTGCAATGACCATGGTTGCAG |
| 729 | FH_0132 | GCTGATGAATGAGTCTCTAATGTTGGTGANNNNNNNNCTTCAGCT TCCCGATATCCGACGGTAGTGTGTTTTCAAGCCAATGA |
| 730 | FH_0133 | GCACACAAAAATGGATCAACCTTAAAGGNNNNNNNNCTTCAGCTT CCCGATATCCGACGGTAGTGTTGCGTGGTGGGAATCC |
| 731 | FH_0134 | GGACATGCTGGGTCCAAAGTGATTTACNNNNNNNNCTTCAGCTTC CCGATATCCGACGGTAGTGTTAGGGTATGACAAGGCAG |
| 732 | IDH1_2593 | CTACGTGGAATTGGATCTACATAGCTATGNNNNNNNNCTTCAGCT TCCCGATATCCGACGGTAGTGTCTGTCAAGGTTTATTG |
| 733 | IDH1_2594 | GCATAATGTTGGCGTCAAATGTGCCACTNNNNNNNNCTTCAGCTT CCCGATATCCGACGGTAGTGTGATTAAAGAGAAACTCA |
| 734 | IDH1_2595 | ATTCTGGGTGGCACGGTCTTCAGAGAAGNNNNNNNNCTTCAGCTT CCCGATATCCGACGGTAGTGTGCAGAAGCTATAAAGAA |
| 735 | IDH1_2596 | GCTTATGGGGATCAATACAGAGCAACTGNNNNNNNNCTTCAGCTT CCCGATATCCGACGGTAGTGTAAATCACCAAATGGCAC |
| 736 | IDH1_2597 | GAACCCAAAAGGTGACATACCTGGTNNNNNNNNCTTCAGCTTCCC GATATCCGACGGTAGTGTGGCTTGTGAGTGGATGGGTA |
| 737 | IDH1_2598 | GTTCCTTCCAAATGGCTCTGTCTAAGGNNNNNNNNCTTCAGCTTC CCGATATCCGACGGTAGTGTATAACCTACACACCAAGT |
| 738 | IDH1_2599 | GCGTTTTAAAGACATCTTTCAGGAGNNNNNNNNCTTCAGCTTCCC GATATCCGACGGTAGTGTCATGGGGATGTATAATCAAG |
| 739 | IDH1_2600 | CGACGACATGGTGGCCCAAGCTATGAAANNNNNNNNCTTCAGCTT CCCGATATCCGACGGTAGTGTCACCAAAAACACTATTC |
| 740 | IDH1_2601 | GTCGGACTCTGTGGCCCAAGGGTATNNNNNNNNCTTCAGCTTCCC GATATCCGACGGTAGTGTCAAGTCCCAGTTTGAAGCTC |
| 741 | IDH1_2602 | GCAGAGGCTGCCCACGGGACTGTAANNNNNNNNCTTCAGCTTCCC GATATCCGACGGTAGTGTGGAGGCTTCATCTGGGCCTG |
| 742 | IDH1_2603 | GCTTCCATTTTTGCCTGGACCAGAGGGTNNNNNNNNCTTCAGCTT CCCGATATCCGACGGTAGTGTATGATGACCAGCGTGCT |
| 743 | IDH1_2604 | GAAGTCTCTATTGAGACAATTGAGGCTGGNNNNNNNNCTTCAGCT TCCCGATATCCGACGGTAGTGTAGAAAGGACAGGAGAC |
| 744 | IDH1 2605 | GCAACGTTCTGACTACTTGNNNNNNNNCTTCAGCTTCCCGATATC CGACGGTAGTGTCAGAGCAAAGCTTGATAACAATAAAG |
| 745 | IDH1_2606 | GTTCATACCTGAGCTAAGAAGGATAATNNNNNNNNCTTCAGCTTC CCGATATCCGACGGTAGTGTGGACTTGGCTGCTTGCAT |
| 746 | IDH2_2059 | ACCCCTGATGAGGCCCGTGTGGAAGAGTTNNNNNNNNCTTCAGCT TCCCGATATCCGACGGTAGTGTACATCCAGCTAAAGTA |
| 747 | IDH2_2060 | GAGCCCATCATCTGCAAAAACATCCCNNNNNNNNCTTCAGCTTCC CGATATCCGACGGTAGTGTGTGTGGCTGTCAAGTGTGC |
| 748 | IDH2_2061 | GCGACCAGTACAAGGCCACAGANNNNNNNNCTTCAGCTTCCCGAT ATCCGACGGTAGTGTAAAGTCCCAATGGAACTATCCGG |
| 749 | IDH2_2062 | GAGTGGGAAGTGTACAACTTCCCCGCAGNNNNNNNNCTTCAGCTT CCCGATATCCGACGGTAGTGTACCAAGCCCATCACCAT |
| 750 | IDH2_2063 | GTATGCCATCCAGAAGAAATGGCCGCTGTNNNNNNNNCTTCAGCT TCCCGATATCCGACGGTAGTGTACCCCAAAAGATGGCA |
| 751 | IDH2_2064 | GCACTATAAGACCGACTTCGACAAGNNNNNNNNCTTCAGCTTCCC GATATCCGACGGTAGTGTTTTTGCGCACAGCTGCTTCC |
| 752 | IDH2_2065 | GCTTTGTGTGGGCCTGCAAGAACTATGNNNNNNNNCTTCAGCTTC CCGATATCCGACGGTAGTGTTTCAAGGACATCTTCCAG |
| 753 | IDH2_2066 | GCCCTGATGGGAAGACGATTGANNNNNNNNCTTCAGCTTCCCGAT ATCCGACGGTAGTGTTGGTGGCTCAGGTCCTCAAGTCT |
| 754 | IDH2_2067 | CCACCAGCACCAACCCCANNNNNNNNCTTCAGCTTCCCGATATCC GACGGTAGTGTATCCTGGCCCAGGGCTTTGGCTCCCTT |
| 755 | IDH2_ 2068 | GCTGGAGAAGGTGTGCGTGGAGACGGTNNNNNNNNCTTCAGCTTC CCGATATCCGACGGTAGTGTGGGAGCACCAGAAGGG |
| 756 | IDH2_2069 | GCAATGTGAAGCTGAACGAGCACTTCCTGNNNNNNNNCTTCAGCT TCCCGATATCCGACGGTAGTGTGGGGAAGCTGGATGGG |
| 757 | MDH1_1041 | GTGACTGGAGCAGCTGGTCAAATTGCATNNNNNNNNCTTCAGCTT CCCGATATCCGACGGTAGTGTTGGGAGAGGAGCGATCT |
| 758 | MDH1_1042 | GCTGTTGGATATCACCCCCATGATGGGNNNNNNNNCTTCAGCTTC CCGATATCCGACGGTAGTGTAGGACGATAAGTCTGAAC |
| 759 | MDH1_1043 | GCAACAGATAAAGAAGACGTTGCCTTNNNNNNNNCTTCAGCTTCC CGATATCCGACGGTAGTGTCTGTCTTTGGTAAAGATCA |
| 760 | MDH1_1044 | GCAAATGTGAAAATCTTCAAATCCCAGGGNNNNNNNNCTTCAGCT TCCCGATATCCGACGGTAGTGTCCCTCCTGAAAGATGT |
| 761 | MDH1_1045 | GCCAATACCAACTGCCTGACTGCTTCCAANNNNNNNNCTTCAGCT TCCCGATATCCGACGGTAGTGTGAAGGGAAGGCATGGA |
| 762 | MDH1_1046 | GATCACAACCGAGCTAAAGCTCAAATTGNNNNNNNNCTTCAGCTT CCCGATATCCGACGGTAGTGTAAATACGCCAAGAAGTC |
| 763 | MDH1_1047 | GAAACCATTCCTCGACTCAGTATCCAGANNNNNNNNCTTCAGCTT CCCGATATCCGACGGTAGTGTCTCCATCCATCCCCAA |
| 764 | MDH1_1048 | GCTCTGAAAGATGACAGCTGGCTCAAGGNNNNNNNNCTTCAGCTT CCCGATATCCGACGGTAGTGTCTTGGTGTGACTGCTAA |
| 765 | MDH1_1049 | GCCATGTCTGCTGCAAAAGCCATCTGTGANNNNNNNNCTTCAGCT TCCCGATATCCGACGGTAGTGTTGCAAGGAAAGGAAGT |
| 766 | MDH1_1050 | GCAACTCCTATGGTGTTCCTGATGATCTGNNNNNNNNCTTCAGCT TCCCGATATCCGACGGTAGTGTCATCAAGGCTCGAAAA |
| 767 | MDH1_1051 | GATTTCTCACGTGAGAAGATGGATCTTNNNNNNNNCTTCAGCTTC CCGATATCCGACGGTAGTGTGTTTGTGTCCATGGGTGT |
| 768 | MDH1_1052 | GCCTGACTAGACAATGATGTTACTAAATGNNNNNNNNCTTCAGCT TCCCGATATCCGACGGTAGTGTAGAATAAGACCTGGAA |
| 769 | MDH1_1053 | GCTATACTTAAATTACTTGTGAAAAACAANNNNNNNNCTTCAGCT TCCCGATATCCGACGGTAGTGTGAAGAAAAAGAAAGTG |
| 770 | MDH2_1470 | GCCACTTTCACTTCTCCTGAAGAACNNNNNNNNCTTCAGCTTCCC GATATCCGACGGTAGTGTTCCCGCTCCAGCCATGCTCT |
| 771 | MDH2_1471 | CCACATCGAGACCAAAGCCNNNNNNNNCTTCAGCTTCCCGATATC CGACGGTAGTGTTAAAGTAGCTGTGCTAGGGGCCTCTG |
| 772 | MDH2_1472 | GTGGTAGTTATTCCGGCTGGAGTCCNNNNNNNNCTTCAGCTTCCC GATATCCGACGGTAGTGTCTGACCCTCTATGATATCGC |
| 773 | MDH2_1473 | GCTGCCTGTGCCCAGCACTGCNNNNNNNNCTTCAGCTTCCCGATA TCCGACGGTAGTGTACCTGAACAGCTGCCTGACTGCCT |
| 774 | MDH2_1474 | CATTGGTGGCCATGCTGGGAAGACCATCANNNNNNNNCTTCAGCT TCCCGATATCCGACGGTAGTGTTTCAACACCAATGCCA |
| 775 | MDH2_1475 | CAGCTGACAGCACTCACTGGNNNNNNNNCTTCAGCTTCCCGATAT CCGACGGTAGTGTTGATCTGCGTCATTGCCAATCCGGG |
| 776 | MDH2_1476 | GCTTTGTCTTCTCCCTTGTGGATGNNNNNNNNCTTCAGCTTCCCG ATATCCGACGGTAGTGTAAGGTGGACTTTCCCCAGGAC |
| 777 | MDH2_1477 | GCTGCTGCTTGGAAAAGGGCATCGAGANNNNNNNCTTCAGCT TCCCGATATCCGACGGTAGTGTATGGCGTATGCCGGCG |
| 778 | MDH2_1478 | GCTGAAGGCCTCCATCAAGAAGGGGGANNNNNNNNCTTCAGCTTC CCGATATCCGACGGTAGTGTAGGAAACGGAATGTACC |
| 779 | MDH2_1479 | GCATCATGTCACTGCAAAGCCGTTGCAGANNNNNNNNCTTCAGCT TCCCGATATCCGACGGTAGTGTAGGAGAAGATGATCTC |
| 780 | VHL_3329 | AAAGACCTGGAGCGGCTGACACAGNNNNNNNNCTTCAGCTTCCCG ATATCCGACGGTAGTGTCCGAGTGTATACTCTGAAAGA |
| 781 | VHL_3330 | GCTTTTGATGGTACTGATGAGTCTTGATNNNNNNNNCTTCAGCTT CCCGATATCCGACGGTAGTGTACGAAGATCTGGAAGAC |
| 782 | MET_control_in tron_1_0002 | GTGACTTCTGCCACATTACCTGACNNNNNNNNCTTCAGCTTCCCG ATATCCGACGGTAGTGTCCTGTAGCAAGTATTTTCGCC |
| 783 | MET_control_in tron_19_0025 | CACACACACACACACACACACACCAGCNNNNNNNNCTTCAGCTTCCC GATATCCGACGGTAGTGTGATAGCGCTCTCATGGCTTG |
| 784 | MET_control_in tron_2_0057 | GCATTTGAAGGATCAAACAATCAACATCNNNNNNNNCTTCAGCTT CCCGATATCCGACGGTAGTGTGGAAAGATACCTGATAA |
| 785 | EGFR_0403 | GCCCTGGGGATCGGCCTCTTCATNNNNNNNNCTTCAGCTTCCCGA TATCCGACGGTAGTGTACCTGTGCCATCCAAACTGCAC |
| 786 | EGFR_0405 | GCACGGTGTATAAGGGACTCTGGATNNNNNNNNCTTCAGCTTCCC GATATCCGACGGTAGTGTTGCTGCAGGAGAGGGAGCTT |
| 787 | EGFR_0406 | GCCAACAAGGAAATCCTCGATGAAGCNNNNNNNNCTTCAGCTTCC CGATATCCGACGGTAGTGTAAAAGATCAAAGTGCTGGG |
| 788 | EGFR_0407 | TCTGCCTCACCTCCACCGTGCANNNNNNNNCTTCAGCTTCCCGAT ATCCGACGGTAGTGTAAGTTAAAATTCCCGTCGCTATC |
| 789 | EGFR_0408 | GCTCCCAGTACCTGCTCAACTGGTGTGTNNNNNNNNCTTCAGCTT CCCGATATCCGACGGTAGTGTTGGACAACCCCCACGT |
| 790 | EGFR_0410 | GCAGAAGGAGGCAAAGTGCCTATCAANNNNNNNNCTTCAGCTTCC CGATATCCGACGGTAGTGTAGGACCGTCGCTTGGTGCA |
| 791 | EGFR_0411 | GAGTTGATGACCTTTGGATCCAAGCCNNNNNNNNCTTCAGCTTCC CGATATCCGACGGTAGTGTCGGAAGAGAAAGAATACCA |
| 792 | EGFR_0414 | GCCAAGTCCTACAGACTCCAACNNNNNNNNCTTCAGCTTCCCGAT ATCCGACGGTAGTGTCATGGTCAAGTGCTGGATGATAG |
| 793 | EGFR_0417 | GACAGCATAGACGACACCTTCCTCCCAGNNNNNNNNCTTCAGCTT CCCGATATCCGACGGTAGTGTAGTGCAACCAGCAACAA |
| 794 | EGFR_0420 | GCCACCAAATTAGCCTGGACAACCCTGNNNNNNNNCTTCAGCTTC CCGATATCCGACGGTAGTGTAGAGACCCACACTACCA |
| 795 | EGFR_0383 | GGAAATTACCTATGTGCAGAGGAATTATGNNNNNNNNCTTCAGCT TCCCGATATCCGACGGTAGTGTCTGGAGGAAAAGAAAG |
| 796 | EGFR_0385 | GCAAATAAAACCGGACTGAAGGAGCNNNNNNNNCTTCAGCTTCCC GATATCCGACGGTAGTGTGTGGCTGGTTATGTCCTCAT |
| 797 | EGFR_0386 | GCCCTGTGCAACGTGGAGAGCATCNNNNNNNNCTTCAGCTTCCCG ATATCCGACGGTAGTGTCTACGAAAATTCCTATGCCTT |
| 798 | EGFR_0389 | GCCTGGTCTGCCGCAAATTCCGAGACGAANNNNNNNNCTTCAGCT TCCCGATATCCGACGGTAGTGTCCAGAAACTGACCAAA |
| 799 | ERBB4_0067 | GTCACTGGTATTCATGGGGACCCTTNNNNNNNNCTTCAGCTTCCC GATATCCGACGGTAGTGTCACTGACATTTGCCCAAAA |
| 800 | ERBB4_0071 | ACAACACTCTTCAGCACAATCAACCAGANNNNNNNNCTTCAGCTT CCCGATATCCGACGGTAGTGTTGCTTATCCTCAAGCAA |
| 801 | ERBB4_0077 | GTGGGCTCTTCATTCTGGTCATTGTGGGNNNNNNNNCTTCAGCTT CCCGATATCCGACGGTAGTGTACCCATGCCATCCAAA |
| 802 | ERBB4_0085 | GCCAATTAAATGGATGGCTCTGGAGTGNNNNNNNNCTTCAGCTTC CCGATATCCGACGGTAGTGTGCAGCCCGTAATGTCTTA |
| 803 | ERBB4_0087 | TGCCTCAGCCTCCCATCTGCACTANNNNNNNNCTTCAGCTTCCCG ATATCCGACGGTAGTGTGAGTGACGTTTGGAGCTATGG |
| 804 | ERBB4_0088 | GCTGAGTTTTCAAGGATGGCTCGAGNNNNNNNNCTTCAGCTTCCC GATATCCGACGGTAGTGTAGAGAAAGGAGAACGTT |
| 805 | ERBB4_0089 | GCTTCCCAGTCCAAATGACAGCAAGNNNNNNNNCTTCAGCTTCCC GATATCCGACGGTAGTGTGTTGGATGATTGATGCTGAC |
| 806 | ERBB4_0058 | GGGTGCTACTGCTGAGATTTTTGATGACTNNNNNNNNCTTCAGCT TCCCGATATCCGACGGTAGTGTCCATGTCAGGAAACCA |
| 807 | ERBB4_0094 | CAGTAGCACCCAGAGGTACAGTGCTGACCNNNNNNNNCTTCAGCT TCCCGATATCCGACGGTAGTGTCCAACTAGCACAATTC |
| 808 | ERBB4_0060 | GCAAGATATTGTTCGGAACCCATGGCCTTNNNNNNNNCTTCAGCT TCCCGATATCCGACGGTAGTGTACAGAAAAGATGGAAA |
| 809 | MERTK_0533 | GCTGAGTAATGGCTCAGTCATGATTTTTNNNNNNNNCTTCAGCTT CCCGATATCCGACGGTAGTGTCCCACCAACTGAAGTCA |
| 810 | MERTK_0537 | GTCCACAATGCTACGTGCACAGTGAGGANNNNNNNNCTTCAGCTT CCCGATATCCGACGGTAGTGTAAGCAGCAGGATGGAG |
| 811 | MERTK_0540 | AATCCTTCTGTCGGCGAGCCATNNNNNNNNCTTCAGCTTCCCGAT ATCCGACGGTAGTGTGTGGATTTATTTTGATTGGGTTG |
| 812 | MERTK_0546 | GCGAGATGACATGACTGTCTGTGTTGNNNNNNNNCTTCAGCTTCC CGATATCCGACGGTAGTGTACAGGACCAAAGCATA |
| 813 | MERTK_0547 | GCCTGTTAAATGGATCGCCATAGNNNNNNNNCTTCAGCTTCCCGA TATCCGACGGTAGTGTCTTCATCGAGATTTAGCTGCTC |
| 814 | MERTK_0549 | GAAGACTGCCTGGATGAACTGTATGANNNNNNNNCTTCAGCTTCC CGATATCCGACGGTAGTGTGTGATGTGTGGGCATTTGG |
| 815 | MERTK_0550 | CTCTTAGAAAGTTTGCCTGACGTTCGGNNNNNNNNCTTCAGCTTC CCGATATCCGACGGTAGTGTTGGCCACAGGTTGAAGCA |
| 816 | MERTK_0554 | GCTGACGACTCCTCAGAAGGCTCAGNNNNNNNNCTTCAGCTTCCC GATATCCGACGGTAGTGTTCTGCTGCAGTCACAGCTG |
| 817 | MERTK_0527 | TCGCTTCCTTCAGCATAACCAGTGTGNNNNNNNNCTTCAGCTTCC CGATATCCGACGGTAGTGTCTCAATCAGTGTACCTAAT |
| 818 | MERTK_0530 | CGAACAGCCTGAAAAATCCCCCNNNNNNNNCTTCAGCTTCCCGAT ATCCGACGGTAGTGTTCCTCACTTTACTAAGCAGCCTG |
| 819 | PLXND1_0604 | TTCCGCCCTTCCCCCCCAACNNNNNNNNCTTCAGCTTCCCGATAT CCGACGGTAGTGTCAGCCTGCCCAGCCTCAGTGGCAT |
| 820 | PLXND1_0605 | TCACCATCTACGACTGCAGCCNNNNNNNNCTTCAGCTTCCCGATA TCCGACGGTAGTGTTTTGGTCACCAGATTGCCTACTGC |
| 821 | PLXND1_0589 | CAGACCCCTGCACGGAGCTGANNNNNNNNCTTCAGCTTCCCGATA TCCGACGGTAGTGTTTCTCCTACGTGCTGCCCCTGGTC |
| 822 | PLXND1_0612 | CCGGGGAGCCTCTCACCCTCGTTANNNNNNNNCTTCAGCTTCCCG ATATCCGACGGTAGTGTGAGGTGGCTGTGGCTGAGG |
| 823 | PLXND1_0613 | GCTGCGACATCCAGATTGTCTCTGACAGANNNNNNNNCTTCAGCT TCCCGATATCCGACGGTAGTGTGGCCAAGAGGGAGAAG |
| 824 | PLXND1_0614 | TTCAACCAGACCATCGCCACACNNNNNNNNCTTCAGCTTCCCGAT ATCCGACGGTAGTGTTACCGGGTCAAGATAGGCCAAGT |
| 825 | PLXND1_0616 | GCAAAGGCTTCGCTGAGCTGCAGANNNNNNNNCTTCAGCTTCCCG ATATCCGACGGTAGTGTGTCCTGCTGCTGCTCTCCGTG |
| 826 | PLXND1_0617 | GGAGTATAAGCACTTCGTGACCCGCACNNNNNNNNCTTCAGCTTC CCGATATCCGACGGTAGTGTTGCTGCAGATGGAGGAG |
| 827 | PLXND1_0618 | TCCCAGACCCTCAACTCCCAGGGNNNNNNNNCTTCAGCTTCCCGA TATCCGACGGTAGTGTACATGACAGATCTCACCAAGGA |
| 828 | PLXND1_0622 | GTCCATCTGCATGTACAGCTGTCTGCGGGNNNNNNNNCTTCAGCT TCCCGATATCCGACGGTAGTGTTACTACACCAGCATCA |
| 829 | PLXND1_0626 | TTCGCCTCCAGCACACAGANNNNNNNNCTTCAGCTTCCCGATATC CGACGGTAGTGTATGGACTCGCTGAGCGTGCGGGCCAT |
| 830 | PLXND1_0630 | CTTTTTCGACTTCCTGGAGGAGCAGGCNNNNNNNNCTTCAGCTTC CCGATATCCGACGGTAGTGTTGCTCCCGGAAATCTACC |
| 831 | PLXND1_0632 | TCATCGCGCAGGCCTTCATCGACGCNNNNNNNNCTTCAGCTTCCC GATATCCGACGGTAGTGTACCCCGACACCCTACACATC |
| 832 | PLXND1_0634 | CTGGCCGAGGAGTCGAGGAAATACCAGAANNNNNNNNCTTCAGCT TCCCGATATCCGACGGTAGTGTATTCGCCAACCAACAA |
| 833 | PLXND1_0636 | GGTGGTGGCTTTGATGGAGGACAACATCTNNNNNNNNCTTCAGCT TCCCGATATCCGACGGTAGTGTAACACCAATGTGGCCA |
| 834 | RET_0681 | TCTCCCAGCACCAAGACCNNNNNNNNCTTCAGCTTCCCGATATCC GACGGTAGTGTCTGCCCCCTGTCCTGTGCAGTCAGC |
| 835 | RET_0683 | GCTTCCCTGAGGAGGAGAAGTGCTTCTGNNNNNNNNCTTCAGCTT CCCGATATCCGACGGTAGTGTGCGATGTTGTGGAGAC |
| 836 | RET_0687 | ATTGTATGGGGCCTGCAGCCAGGNNNNNNNNCTTCAGCTTCCCGA TATCCGACGGTAGTGTAAAGGCAGAGCAGGGTA |
| 837 | RET_0690 | CGGCTTGTCCCGAGATGTTTATGNNNNNNNNCTTCAGCTTCCCGA TATCCGACGGTAGTGTCTCATCTCATTTGCCTGGCAG |
| 838 | RET_0691 | GATCATATCTACACCACGCAAAGTGATGNNNNNNNNCTTCAGCTT CCCGATATCCGACGGTAGTGTGAGGGGCGGAAGATGA |
| 839 | RET_0693 | GGAGATGTACCGCCTGATGCNNNNNNNNCTTCAGCTTCCCGATAT CCGACGGTAGTGTTGGTCTTTTGGTGTCCTGCTGTGGG |
| 840 | RET_0695 | CACCGCTGGTGGACTGTAATAATGCCNNNNNNNNCTTCAGCTTCC CGATATCCGACGGTAGTGTGGTGTTTGCGGACATCAG |
| 841 | RET_0697 | GGATGCTTTCACCCTCAGCGGCAAAATNNNNNNNNCTTCAGCTTC CCGATATCCGACGGTAGTGTGAAAACAAACTCTATGGC |
| 842 | RET_0698 | GTGAAAGGTAATGGACTCACAAGGGGAANNNNNNNNCTTCAGCTT CCCGATATCCGACGGTAGTGTACGAGAGCTGATGGCA |
| 843 | RET_0673 | GCTCCTGGGAGAAGCTCAGTNNNNNNNNCTTCAGCTTCCCGATAT CCGACGGTAGTGTTGAGGAGGTGCCCAGCTTCCG |
| 844 | AXL_0731 | TCGTCGGACCACTGAAGCTACCNNNNNNNNCTTCAGCTTCCCGAT ATCCGACGGTAGTGTCTGTGTCCTCATCTTGGCTCTCT |
| 845 | AXL_0735 | TCCTCCTCTATTCCCGGCTNNNNNNNNCTTCAGCTTCCCGATATC CGACGGTAGTGTTCTGCATGAAGGAATTTGACCATCCC |
| 846 | AXL_0737 | GTCCGTGTGTGTGGCGGACTTCNNNNNNNNCTTCAGCTTCCCGAT ATCCGACGGTAGTGTAGTGTACCTGCCCACTCAGATG |
| 847 | AXL_0738 | GCCATTGAGAGTCTAGCTGACCGTGTCTNNNNNNNNCTTCAGCTT CCCGATATCCGACGGTAGTGTAGGAACTGCATGCTGAA |
| 848 | AXL_0739 | CGGGCGTGGAGAACAGCGAGATTTATGNNNNNNNNCTTCAGCTTC CCGATATCCGACGGTAGTGTAAGATGCCAGTCAAGTGG |
| 849 | AXL_0740 | GGACTGTATGCCTTGATGTCGCGGTGCTNNNNNNNNCTTCAGCTT CCCGATATCCGACGGTAGTGTGGGGTGACAATGTGGG |
| 850 | AXL_0742 | AGCTGACCCCCCAACCCANNNNNNNNCTTCAGCTTCCCGATATCC GACGGTAGTGTGTTTTACAGAGCTGCGGGAAGATTTGG |
| 851 | AXL_0744 | TTCCCACCCCACGCCTTATCNNNNNNNNCTTCAGCTTCCCGATAT CCGACGGTAGTGTTCAGCCTGCTGATAGGGGCTCCCC |
| 852 | AXL_0727 | GCCCGAAGACAGGACTGTGGCNNNNNNNNCTTCAGCTTCCCGATA TCCGACGGTAGTGTAGCTCAGAATCACCTCCCTGCA |
| 853 | AXL_0717 | TCCCCCTGGCCACGGCTCCANNNNNNNNCTTCAGCTTCCCGATAT CCGACGGTAGTGTGCTGGAGGGCTTGCCTTACTTCCT |
| 854 | EGFR_delta_14_ 15_1808nt_0001 | GCCAGGTCTTGAAGGCTGTCCAANNNNNNNNCTTCAGCTTCCCGA TATCCGACGGTAGTGTGTCTGCCATGCCTTGTGCT |
| 855 | EGFR_delta_14_ 15_1808nt_0002 | GCCCTGGGGATCGGCCTCTTCATGCGAANNNNNNNNCTTCAGCTT CCCGATATCCGACGGTAGTGTGACAAGTGCAACCTTCT |
| 856 | EGFR_delta_2_7 265nt_0006 | GTGGTGACAGATCACGGCTCGTGNNNNNNNNCTTCAGCTTCCCGA TATCCGACGGTAGTGTGAGAGCCGGAGCGAGCTCTT |
| 857 | EGFR_delta_2_7 265nt_0007 | GCCGCAAAGTGTGTAACGGAATAGGTANNNNNNNNCTTCAGCTTC CCGATATCCGACGGTAGTGTCTGGAGGAAAAGAAAG |
| 858 | EGFR_delta_12_ 12_674nt_0011 | GCCAAGGGAGTTTGTGGAGAACTCTGAGTNNNNNNNNCTTCAGCT TCCCGATATCCGACGGTAGTGTATTCTGAAAACCGTAA |
| 859 | EGFR_delta_12_ 12_674nt_0012 | CGGGGACCAGACAACTGTATCCAGTGTGNNNNNNNNCTTCAGCTT CCCGATATCCGACGGTAGTGTAACCTAGAAATCATACG |
| 860 | EGFR_delta_25_ 27_3123nt_0017 | CACAATCAGCCTCTGAACCCNNNNNNNNCTTCAGCTTCCCGATAT CCGACGGTAGTGTCCAAAGTTCCGTGAGTTGATCATCG |
| 861 | MET_delta_14_3 128nt_0025 | GTTTCCTAATTCATCTCAGAACGGTTCANNNNNNNNCTTCAGCTT CCCGATATCCGACGGTAGTGTAATAGTTCAACCAGATC |
| 862 | MET_delta_14_3 128nt_0026 | CAGTCCATTACTGCAAAATACTGTCCACANNNNNNNNCTTCAGCT TCCCGATATCCGACGGTAGTGTAAAAAGAGAAAGCAAA |
| 863 | MET_delta_4_5_ 1579nt_0032 | GCAGGTTTTCCCAAATAGTGCACCCCTTGNNNNNNNNCTTCAGCT TCCCGATATCCGACGGTAGTGTGCTTTGCAGCGCGTTG |
| 864 | MET_delta_4_5_ 1579nt-0033 | ACTAGAGTTCTCCTTGGAAATGAGAGCNNNNNNNNCTTCAGCTTC CCGATATCCGACGGTAGTGTGACATCAGAGGGTCGCTT |
| 865 | MET_delta_7_8_ 2049nt_0037 | GCACGATGAATACTGTGTCAAACAGNNNNNNNNCTTCAGCTTCCC GATATCCGACGGTAGTGTTTGAAGGAGGGACAAGGCTG |
| 866 | MET_delta_7_8_ 2049nt_0038 | GCCAACCGAGAGACAAGCATCTTCANNNNNNNNCTTCAGCTTCCC GATATCCGACGGTAGTGTAATGAGAGCTGCACCTTGAC |
| 867 | MET_var_1_fusi on_9_10A_2638n t_0042 | ATTAGTACTTGGTGGAAAGAACCTCTCAANNNNNNNNCTTCAGCT TCCCGATATCCGACGGTAGTGTCCCAAACCATTTCAAC |
| 868 | MET_var_1_fusi on_9_10A_2638n t_0043 | CAGTTAGTGTCCCGAGAATGGTCATAAANNNNNNNNCTTCAGCTT CCCGATATCCGACGGTAGTGTAAATTCATCCAACCAAA |
| 869 | MET_var_2_fusi on_9_10B_2664n t_0047 | GTGGTGGGAGCACAATAACAGGTGTTGNNNNNNNNCTTCAGCTTC CCGATATCCGACGGTAGTGTCAAACCATTTCAACTGAG |
| 870 | MET_var_2_fusi on_9_10B_2664n t_0048 | GCATGTCAACATCGCTCTAATTCAGNNNNNNNNCTTCAGCTTCCC GATATCCGACGGTAGTGTATTCATCCAACCAAATCTTT |
| 871 | MET_var_2_fusi on_9_11_2464nt 0052 | GCATGTCAACATCGCTCTAATTCAGNNNNNNNNCTTCAGCTTCCC GATATCCGACGGTAGTGTCCAAACCATTTCAACTGAGT |
| 872 | MET_var_2_fusi on_9_11_2464nt 0053 | GCCTTTTTCATGTTAGATGGGATCCTTTNNNNNNNNCTTCAGCTT CCCGATATCCGACGGTAGTGTCTATGAAATTCATCCAA |
| 873 | KIT_0066 | GTCACAACAACCTTGGAAGTAGTAGANNNNNNNNCTTCAGCTTCC CGATATCCGACGGTAGTGTATAATAGCTGGCATCACGG |
| 874 | KIT_0069 | CCGAAGGAGGCACTTACACATTCCTAGNNNNNNNNCTTCAGCTTC CCGATATCCGACGGTAGTGTGAACACCAGCAGTGGATC |
| 875 | KIT_0072 | GCACAATGGCACGGTTGAATGTAAGGNNNNNNNNCTTCAGCTTCC CGATATCCGACGGTAGTGTTGTCCAGGAACTGAGCAGA |
| 876 | KIT_0075 | CAAATGGGAGTTTCCCAGAAACAGGCTGANNNNNNNNCTTCAGCT TCCCGATATCCGACGGTAGTGTGTGATGATTCTGACCT |
| 877 | KIT_0078 | GCTATGGTGATCTTTTGAATTTTTTGAGNNNNNNNNCTTCAGCTT CCCGATATCCGACGGTAGTGTAACGGGAAGCCCTCATG |
| 878 | KIT_0080 | GCCGACAAAAGGAGATCTGTGAGAATAGGNNNNNNNNCTTCAGCT TCCCGATATCCGACGGTAGTGTAAGATCATGCAGAAGC |
| 879 | KIT_0083 | GTGAAGTGGATGGCACCTGAAAGCANNNNNNNNCTTCAGCTTCCC GATATCCGACGGTAGTGTAGAGACTTGGCAGCCAGAAA |
| 880 | KIT_0058 | GCTGTTATGCACTGATCCGGGCTTNNNNNNNNCTTCAGCTTCCCG ATATCCGACGGTAGTGTTTCTGCTCCTACTGCTTCGCG |
| 881 | KIT_0060 | TGCCAAGCTTTTCCTTGTTGACCGCTCCNNNNNNNNCTTCAGCTT CCCGATATCCGACGGTAGTGTAACGAATGAGAATAAGC |
| 882 | KIT_0063 | GCTGTGCCTGTTGTGTCTGTGTNNNNNNNNCTTCAGCTTCCCGAT ATCCGACGGTAGTGTAAGGCGGGCATCATGATCAAAAG |
| 883 | PTEN_0098 | GGATTCAAAGCATAAAAACCATTACAAGANNNNNNNNCTTCAGCT TCCCGATATCCGACGGTAGTGTCAAGAGGATGGATTCG |
| 884 | PTEN_0100 | CATGTTGCAGCAATTCACTGTAAAGCTGGNNNNNNNNCTTCAGCT TCCCGATATCCGACGGTAGTGTACACCGCCAAATTTAA |
| 885 | PTEN_0101 | GCCCTAGATTTCTATGGGGAAGTAAGGNNNNNNNNCTTCAGCTTC CCGATATCCGACGGTAGTGTCCAATGGCTAAGTGAAGA |
| 886 | PTEN_0102 | GGATTATAGACCAGTGGCACTGTTGTTTNNNNNNNNCTTCAGCTT CCCGATATCCGACGGTAGTGTTTTAAAGGCACAAGAG |
| 887 | PTEN_0103 | CCTCAGTTTGTGGTCTGCCAGCTNNNNNNNNCTTCAGCTTCCCGA TATCCGACGGTAGTGTGTCAGAGGCGCTATGTGTATT |
| 888 | PTEN_0104 | GCCGTTACCTGTGTGTGGTGATATNNNNNNNNCTTCAGCTTCCCG ATATCCGACGGTAGTGTTTCCAATGTTCAGTGGCGG |
| 889 | PTEN_0105 | ACCAGGACCAGAGGAAACCTCANNNNNNNNCTTCAGCTTCCCGAT ATCCGACGGTAGTGTGTTCATGTACTTTGAGTTCCCTC |
| 890 | PTEN_0107 | AGCCAACCGATACTTTTCTCCAANNNNNNNNCTTCAGCTTCCCGA TATCCGACGGTAGTGTGTAGAAAATGGAAGTCTATGTG |
| 891 | PTEN_0109 | GATCAGCATACACAAATTACAAAAGTCTGNNNNNNNNCTTCAGCT TCCCGATATCCGACGGTAGTGTCGTCAAATCCAGAGGC |
| 892 | PTEN_0110 | GGACCTTTTTTTTTTTAATGGCAATAGGNNNNNNNNCTTCAGCTT CCCGATATCCGACGGTAGTGTTGACTCTGATCCAGAGA |
| 893 | ACTB_0129 | TTGCTCCTCCTGAGCGCAAGTACTCNNNNNNNNCTTCAGCTTCCC GATATCCGACGGTAGTGTACATCCGCAAAGACCTGTAC |
| 894 | ACTB_0123 | TCTGGCACCACACCTTCTACAATGNNNNNNNNCTTCAGCTTCCCG ATATCCGACGGTAGTGTGTGATGGTGGGCATGGGTC |
| 895 | ACTB_0124 | AACCCCAAGGCCAACCGCGANNNNNNNNCTTCAGCTTCCCGATAT CCGACGGTAGTGTTGAAGTACCCCATCGAGCACGGCAT |
| 896 | ACTB_0126 | GGTCATCACCATTGGCAATGAGCGGTNNNNNNNNCTTCAGCTTCC CGATATCCGACGGTAGTGTGAGCGGGAAATCGTGCGTG |
| 897 | ACTB_0127 | GGCATCCACGAAACTACCTTCAACNNNNNNNNCTTCAGCTTCCCG ATATCCGACGGTAGTGTTGCTTCCAGCTCCTCCCTGG |
| 898 | ACTB_0128 | CCACCATGTACCCTGGCATTGCCNNNNNNNNCTTCAGCTTCCCGA TATCCGACGGTAGTGTACTCTTCCAGCCTTCCTTCCT |
| 899 | MET_var_1_0147 | GTTCCATAAACTCTGGATTGCATTCCTACNNNNNNNNCTTCAGCT TCCCGATATCCGACGGTAGTGTCAGAGATTCTTACCCC |
| 900 | MET_var1_0150 | GTGAGATGTCTCCAGCATTTTTACGGNNNNNNNNCTTCAGCTTCC CGATATCCGACGGTAGTGTTTCTTTTCGGGGTGTTCGC |
| 901 | MET_var_1_0153 | ACTCCCATCCAGTGTCTCNNNNNNNNCTTCAGCTTCCCGATATCC GACGGTAGTGTCTCTTAACATCTATATCCACCTTCATT |
| 902 | MET_var_1_0156 | GCTGACCATATGTGGCTGGGACTTTGGATNNNNNNNNCTTCAGCT TCCCGATATCCGACGGTAGTGTTGGTGCCACGACAAAT |
| 903 | MET_var_1_0159 | GCTGGTGGCACTTTACTTACTTTNNNNNNNNCTTCAGCTTCCCGA TATCCGACGGTAGTGTGTCCTGCCATGAATAAGCATTT |
| 904 | MET_var_1_0162 | GCTTTGCCAGTGGTGGGAGCACAATANNNNNNNNCTTCAGCTTCC CGATATCCGACGGTAGTGTAGCCAACCGAGAGACAA |
| 905 | MET_var_1_0165 | GCCTTTTGAAAAGCCAGTGATGATCNNNNNNNNCTTCAGCTTCCC GATATCCGACGGTAGTGTTTGTACCACTCCTTCCCTGC |
| 906 | MET_var_1_0169 | GCAAATTAAAGATCTGGGCAGTGAATTAGNNNNNNNNCTTCAGCT TCCCGATATCCGACGGTAGTGTGTCCTTGGAAAAGTAA |
| 907 | MET_var_1_0170 | CCCAACTACAGAAATGGTTTCAAATGNNNNNNNNCTTCAGCTTCC CGATATCCGACGGTAGTGTCTTGGGTTTTTCCTGTGGC |
| 908 | MET_var_1_0171 | GCAGTATCCTCTGACAGACATGTCCNNNNNNNNCTTCAGCTTCCC GATATCCGACGGTAGTGTGCTTGTAAGTGCCCGAAGTG |
| 909 | MET_var_1_0174 | CAATTTCTGACCGAGGGAATCATCATGANNNNNNNNCTTCAGCTT CCCGATATCCGACGGTAGTGTGAAGTCATAGGAAGAGG |
| 910 | MET_var_1_0178 | GCAAACTCAAAAGTTTACCACCAAGTCNNNNNNNNCTTCAGCTTC CCGATATCCGACGGTAGTGTAAAAATTCACAGTCAAGG |
| 911 | MET_var_1_0181 | ACTTTCATTGGGGAGCACTATGTCCATNNNNNNNNCTTCAGCTTC CCGATATCCGACGGTAGTGTACTCCTACAACCCGAATA |
| 912 | MET_var_1_0184 | GTATTGTTATTTAAATTACTGGATTCTANNNNNNNNCTTCAGCTT CCCGATATCCGACGGTAGTGTCATAGTGCTAGTACTAT |
| 913 | MET_var_1_0144 | CGGTTCATCAACTTCTTTGTAGGNNNNNNNNCTTCAGCTTCCCGA TATCCGACGGTAGTGTACAATCATACTGCTGACATACA |
| 914 | TUBB_0211 | TCCGCCGGAAGGCCTTCCTCCACTNNNNNNNNCTTCAGCTTCCCG ATATCCGACGGTAGTGTAACAATGTCAAGACAGCCGTC |
| 915 | TUBB_0202 | CAGCTGACCCACTCACTGGNNNNNNNNCTTCAGCTTCCCGATATC CGACGGTAGTGTTTTGTATTTGGTCAGTCTGGGGCAGG |
| 916 | TUBB_0205 | CCACCTTGTCTCAGCCACCANNNNNNNNCTTCAGCTTCCCGATAT CCGACGGTAGTGTTACAATGCCACCCTCTCCGTCCATC |
| 917 | TUBB_0208 | TACCTCACCGTGGCTGCTNNNNNNNNCTTCAGCTTCCCGATATCC GACGGTAGTGTCTTTATGCCTGGCTTTGCCCCTCTCAC |
| 918 | ERBB3_0013 | CCACCACTCTTTGAACTGGACCAAGNNNNNNNNCTTCAGCTTCCC GATATCCGACGGTAGTGTCGGGGCTTCTCATTGTTGAT |
| 919 | ERBB3_0015 | GCCGAGGAGGTGTCTGTGTGANNNNNNNNCTTCAGCTTCCCGATA TCCGACGGTAGTGTAGACATCAAGCATAATCGGCCG |
| 920 | ERBB3_0019 | CCCATCTGACAATGGCTTTGACAGTGNNNNNNNNCTTCAGCTTCC CGATATCCGACGGTAGTGTCCCAATCTACAAGTACCCA |
| 921 | ERBB3_0025 | GCCAAGGGAATGTACTACCTTGAGGANNNNNNNNCTTCAGCTTCC CGATATCCGACGGTAGTGTGTCATCTCTGCAGCTTG |
| 922 | ERBB3_0027 | GTGGATGGCCCTTGAGAGTATCCACTTNNNNNNNNCTTCAGCTTC CCGATATCCGACGGTAGTGTAAACGTGCTACTCAAGTC |
| 923 | ERBB3_0034 | GCAGTTTCTGGGAGCAGTGAACGGTGCNNNNNNNNCTTCAGCTTC CCGATATCCGACGGTAGTGTAGCCTACCAGTTGGAA |
| 924 | ERBB3_0037 | TACTCCCTCCTCCCGGGAAGGCANNNNNNNNCTTCAGCTTCCCGA TATCCGACGGTAGTGTCGGAGATAGCGCCTACCA |
| 925 | ERBB3_0043 | CTCCTGCTCCCTGTGGCACTCNNNNNNNNCTTCAGCTTCCCGATA TCCGACGGTAGTGTCCCCCCATGTCCATTATGCCC |
| 926 | ERBB3_0002 | GCTTTGTCACATGGACACAATTGACTGNNNNNNNNCTTCAGCTTC CCGATATCCGACGGTAGTGTGGAAGTTTGCCATCTTC |
| 927 | ERBB3_0005 | GCCTGCCGGCACTTCAATGACAGTGGAGNNNNNNNNCTTCAGCTT CCCGATATCCGACGGTAGTGTACATTGACCAAGACCA |
| 928 | RON_0255 | TTTTGCCCCAACCCGCCTNNNNNNNNCTTCAGCTTCCCGATATCC GACGGTAGTGTCCCCAACTCTGTCGTCTGTGCCTTCCC |
| 929 | RON_0263 | AACTGGAGCCCTTGGGCACCCAGNNNNNNNNCTTCAGCTTCCCGA TATCCGACGGTAGTGTTTCTGGTCTGGTGCCTGAGGG |
| 930 | RON_0265 | GGCACCTGTCTCACTCTTGAAGGCNNNNNNNNCTTCAGCTTCCCG ATATCCGACGGTAGTGTCCTCACCGTGACTAACATGCC |
| 931 | RON_0270 | GGAGCTGCTGGCTTTACACTGCCTGGNNNNNNNNCTTCAGCTTCC CGATATCCGACGGTAGTGTGGCTTAGGGCAGTGGAAAG |
| 932 | RON_0274 | GCACTGGTCTTCAGCTACTGGTGGNNNNNNNNCTTCAGCTTCCCG ATATCCGACGGTAGTGTGGTCTGCGTAGATGGTG |
| 933 | RON_0279 | GTGGAGGCCTTCCTGCGAGAGGGGCTNNNNNNNNCTTCAGCTTCC CGATATCCGACGGTAGTGTACAGTGACCGAGTCATTGG |
| 934 | RON_0281 | CCTCATCAGCTTTGGCCTGCAGGTANNNNNNNNCTTCAGCTTCCC GATATCCGACGGTAGTGTTGTGCTGGCTCTCATTGGT |
| 935 | RON_0282 | CAGTCAAGGTGGCTGACTTTGGTTNNNNNNNNCTTCAGCTTCCCG ATATCCGACGGTAGTGTAACCCCACCGTGAAGGA |
| 936 | RON_0287 | CTCACCCATGCCAGGGAATGTACGNNNNNNNNCTTCAGCTTCCCG ATATCCGACGGTAGTGTTGGGGGAGGTGGAGCAG |
| 937 | RON_0252 | CCACACGGGAGCCTTCGTATACTNNNNNNNNCTTCAGCTTCCCGA TATCCGACGGTAGTGTTTCAGCCCACGCTCAGTGTCT |
| 938 | ALK_0320 | TGCCCAGAGGCTCCTTTCTCCNNNNNNNNCTTCAGCTTCCCGATA TCCGACGGTAGTGTTGTGAGCTGGAGTATTCCCCTCC |
| 939 | ALK_0323 | GTGGAAACCGCAGCTTGTCTGCAGTGGANNNNNNNNCTTCAGCTT CCCGATATCCGACGGTAGTGTACAACGAGGCTGCAAGA |
| 940 | ALK_0328 | CGTGTCCTTGGTGCTAGTGGNNNNNNNNCTTCAGCTTCCCGATAT CCGACGGTAGTGTTTGCTCAGTACCACTGATGTCCC |
| 941 | ALK_0329 | GCCTGTGGCAGTGGATGGTGTTGNNNNNNNNCTTCAGCTTCCCGA TATCCGACGGTAGTGTTGAGCTCCGAATGTCCTGG |
| 942 | ALK_0334 | GCGGGAAAGGCGGGAAGAACACCATGANNNNNNNNCTTCAGCTTC CCGATATCCGACGGTAGTGTAACAACGCCTACCAGAA |
| 943 | ALK_0335 | GCTGTACATCCTGGTTGGGCAGCAGGGANNNNNNNNCTTCAGCTT CCCGATATCCGACGGTAGTGTAGCCACCGACACCTACA |
| 944 | ALK_0347 | CTGCCCCGGTTCATCCTGCTGGANNNNNNNNCTTCAGCTTCCCGA TATCCGACGGTAGTGTTGGCTGTGAAGACGCTGCCTG |
| 945 | ALK_0349 | CAGACACATGGTCCTTTGGAGTGCTGNNNNNNNNCTTCAGCTTCC CGATATCCGACGGTAGTGTTGGAGACTTCGGGATG |
| 946 | ALK_0355 | CCCAACGTACGGCTCCTGGTTNNNNNNNNCTTCAGCTTCCCGATA TCCGACGGTAGTGTTCTCTGTTCGAGTCCCTAGAGGGC |
| 947 | ALK_0358 | GCCCCTGGAGCTGGTCATTACGANNNNNNNNCTTCAGCTTCCCGA TATCCGACGGTAGTGTTGCTCCTAGAGCCCTCTTCGCT |
| 948 | EGFR_0391 | GCCTGTGGGGCCGACAGCTATGAGATGGANNNNNNNNCTTCAGCT TCCCGATATCCGACGGTAGTGTTCTACAACCCCACCAC |
| 949 | EGFR_0394 | CGTAAAGGAAATCACAGGGTTTTTGCTGANNNNNNNNCTTCAGCT TCCCGATATCCGACGGTAGTGTAAACACTTCAAAAACT |
| 950 | forward primer HMBSFw | CTGGTAACGGCAATGCGGCT |
| 951 | reverse primer HMBSRv | TTCTTCTCCAGGGCATGTTC |

| | | |
|---|---|---|
| Sequences 1 - 949 are smMIPs | | |

### Examples

### Example 1 - targeted smMIP-based RNA sequencing yields relevant information on metabolism

In the past four decades an overwhelming amount of data has become available on the molecular events that underlie carcinogenesis. Research has mainly focused on molecular alterations and their consequences for among others the PI3K/pAKT/mTOR pathway(19-22) and cell cycle control, apoptosis (23, 24) and DNA repair pathways (25, 26). Currently, numerous FDA-approved drugs are available that target cancer cells based on these genetic defects with a level of specificity that is not attainable with conventional chemotherapies (27, 28), permitting personalized medicine. Whereas targeted cancer therapies may prolong survival, it is now widely recognized that inherent genetic instability ultimately leads to therapy resistance of most cancers (11-14).

For proliferation, cancer cells need to generate ATP to maintain energy balance and ion homeostasis, import carbon and nitrogen sources for synthesis of amino acids, nucleotides and lipids (29, 30) and maintain redox potential to protect cells against oxidative stress (31). Blocking one or more of these processes may prohibit proliferation and/or sensitize cells to toxic therapy in a synthetic lethality approach. As an example, increasing oxidative stress in a cancer with metabolic inhibitors may enhance the efficacy of radiotherapy (32) or chemotherapy (33). With the increasing knowledge of deranged metabolic pathways in cancer (34-37), (adjuvant) targeting of cancer-specific metabolic pathways may be a highly interesting addition to current treatment protocols. The best-known example of cancer-specific metabolic adaptation is aerobic glycolysis, also known as the Warburg effect (38). As glycolysis is inefficient in terms of ATP production, cancer cells characteristically upregulate glucose transporters GLUT1 and/or GLUT3. Besides glucose, glutamine and fatty acids are recognized as important fuels for cancer cells (39, 40) (41, 42).

While metabolic adaptations are mostly seen as a consequence of carcinogenesis, it has been unequivocally established that metabolic alterations can also cause cancer, examples being mutations in genes encoding mitochondrial (e.g. *IDH2, FH, SDH*) and cytosolic (e.g. *IDH1)* metabolic enzymes (43, 44) (45, 46) (1, 47, 48), the latter being prominent in among others low grade gliomas and secondary glioblastomas (1, 48). Clear cell renal cell carcinoma (ccRCC) is now considered a metabolic disease with metabolic alterations resulting indirectly from inactivating mutations in or epigenetic silencing of *VHL,* found in ~80% of clear cell renal cell cancers (ccRCC) (49). pVHL is a major regulator of ubiquitination and breakdown of transcription factor hypoxia inducible factors HIF-1α and HIF-2α (49). Mutations in the aforementioned metabolic enzymes and in *VHL* have been shown to induce epigenetic alterations that affect expression of other metabolic enzymes in an unpredictable fashion (17, 50-52).

To apply metabolic inhibitors as potential additions to the current anti-tumor armamentarium, it is of high importance to identify which metabolic pathways are active in a specific cancer in a personalized fashion. Here we applied a novel next generation-sequencing based method using single molecule molecular inversion probes (smMIPs(15)), to detect expression levels of 104 genes involved in metabolism, and concomitantly identify variants therein. As a proof of concept, we applied smMIPs to map part of the metabolic transcriptome of a VHL-defective ccRCC cell line and a corresponding VHL-rescued isogenic derivative, as well as in patient derived glioma xenograft models. We validated the technique by correlating results with whole transcriptome RNAseq data (as gold standard for transcriptome analysis) and protein expression. We further verified the ability of the assay to detect oncogenic mutations in cell lines and patient tumor tissue.

Our data show that targeted RNA sequencing of transcripts encoding metabolic enzymes using smMIPs predict the predominant metabolic pathways that are operational in cancer (53) and simultaneously allows variant detection in the targeted transcripts.

### Materials and Methods

### Cell lines -

The cell line SKRC7 is derived from a primary human ccRCC and has been described before (54). Cells were cultured in RPMI 1640 (Lonza Group, Switzerland) supplemented with 10% fetal calf serum (FCS) (Gibco, Thermo Fisher Scientific, Waltham, MA, USA) and 40 µg/ml gentamycin (Centrafarm, Etten-Leur, The Netherlands). An isogenic SKRC7 cell line expressing a functional haemagglutinine (HA)-tagged VHL (SKRC7-VHL^{HA}) was created by transfection with pcDNA3.1-VHL^{HA} followed by selection of stable transfectants in the same medium with 400 µg/ml geneticin (Gibco, Thermo Fisher Scientific, Waltham, MA, USA). The patient-derived glioma xenograft models E478 and E98 have been described before (55, 56).

### Patient material -

Use of patient material was according to the guidelines of the local ethical committee for use of patient material and was performed with informed consent. Surgically obtained tissue from a male patient with a grade III astrocytoma was snap frozen frozen in liquid nitrogen.

### RNA and cDNA preparation -

Total RNA was isolated from sections of snap-frozen E478 xenograft tissue, human tumor tissue and from 80% confluent SKRC7 and SKRC7-VHL^{HA} cells using TRIzol reagent (Life Technologies, ThermoFisher Scientific, Waltham, MA, USA) according to the manufacturers' instructions. RNA quality was estimated based on relative levels of 28S, 18S and 5S rRNA bands on agarose gel and with Bioanalyzer assays (Agilent Technologies, Amstelveen, The Netherlands). RNA was reverse transcribed to cDNA using Superscript II reverse transcriptase (Invitrogen, ThermoFisher Scientific, Waltham, MA, USA) and random hexamer primers (Promega, Madison, WI, USA) according to standard protocols. Next, cDNA was purified using the NucleoSpin Gel and PCR Clean-up kit (Macherey-Nagel, Düren, Germany). For quality control, cDNA was subjected to PCR for reference gene hydroxymethylbilane Synthase (HBMS) with forward primer HMBSFw (5'-CTGGTAACGGCAATGCGGCT-3') and reverse primer HMBSRv (5'-TTCTTCTCCAGGGCATGTTC-3') using AmpliTaq Gold 360 master mix (Applied Biosystems, ThermoFisher Scientific, Waltham, MA USA).

### Whole transcriptome RNAseq analysis

High quality RNA with RIN scores > 8 was subjected to whole transcriptome RNAseq according to standard protocols. Sequencing was performed on an Illumina Hiseq and yielded 30-50 million reads per sample (paired end sequencing protocol). The dataset was analyzed using the 'Tuxedo' protocol; reads were mapped against the RefSeq human genome (hg19) with TopHat and final transcript assembly was done with the Cufflinks package (57). Normalization was done with both Cuffquant and the calculation of fragments as transcript per million mapped reads (TPM) to obtain relative expression values. Occurrence of single nucleotide variants was visualized in the Integrated Genomics Viewer browser (lGV, the Broad institute).

### smMIP design

The technique of targeted RNAseq using smMIPs is depicted in Figure 1. It is based on the hybridization of an extension and ligation probe, joined by a 'constant' backbone sequence in an inverted manner to a cDNA of interest, followed by gap-filling/ligation and PCR. SmMIPs against the antisense strand of 104 predicted transcripts (UCSC human genome assembly hg19) were designed based on the MIPgen algorithm as described by Boyle et al. (18). Whenever possible, smMIPs were designed with ligation and extension probes located on adjacent exons to prevent contribution of smMIP probes that hybridize to potential contaminations of genomic DNA. Transcripts of interest were encoding enzymes and transporters functioning in various metabolic pathways, including lipid metabolism, glycolysis, oxidative phosphorylation (OXPHOS), tricarboxylic acid (TCA) cycle, pentose phosphate pathway (PPP), glutaminolysis and control of reductive potential (see Table I). The smMIP set also contained probes for detection of β-actin and β-tubulin as housekeeping genes, and a number of tyrosine kinases with relevance for cancer. SmMIPs were designed with extension probes of 16 to 20 nt in length and ligation probes of 20-24 nt in length, joined by a constant backbone sequence (40 nt) with a stretch of 8 random nucleotides incorporated adjacent to the ligation probe. The random 8N sequence is incorporated to reduce all amplicons originating from one individual smMIP to one unique MIP (see below). The length of gap-fill was set at 112 nt. Whereas the design was based on full coverage, for the majority of transcripts 5-10 smMIPS per transcript were included in the panel with the target regions distributed evenly over the reading frame. For 18 transcripts (CS, D-2HGDH, L-2HGDH, FH, IDH1-3A-G, MDH1-2, MYC, OGDH, SDHA-D, VHL) smMIP sets were chosen that covered the full coding sequences.

### Capture and library preparation

642 smMIPs (IDT, Leuven, Belgium) were pooled at 100 µM/smMIP. The smMIP pool was phosphorylated using T4 Polynucleotide Kinase (New England Biolabs, NEB, Ipswich, MA, USA) in T4 DNA ligase buffer (NEB) at 37°C for 45 min, followed by inactivation for 20 min at 65°C. The capture reaction was performed with 50 ng of cDNA and an estimated 8000-fold molar excess of the phosphorylated smMIP pool (16) in a 25 µL reaction mixture containing Ampligase buffer (Epicentre, Madison, WI, USA), dNTPs, Hemo KlenTaq enzyme (New England Biolabs, NEB, Ipswich, MA, USA) and thermostable DNA ligase (Ampligase, Epicentre). The capture mix was incubated for 10 min at 95 °C (denaturation), followed by incubation for 18 h at 60 °C, during which hybridization and concomitant primer extension and ligation occurs. Directly after this step non-circularized smMIPs, RNA and cDNA were removed by treatment with 10 U Exonuclease I and 50 U of Exonuclease III (both NEB) for 45 min at 37°C, followed by heat inactivation (95°C, 2 min). The circularized smMIP library was subjected to standard PCR with 2x iProof High-Fidelity DNA Polymerase master Mix (Bio-Rad, Hercules, CA) with a primer set containing a unique barcoded reverse primer for each sample. Generation of PCR products of correct size (266 bp) was validated on agarose gel electrophoresis, and PCR-libraries from different samples were pooled based on relative band intensity. The pool was then purified using AMPureXP beads (Beckman Coulter Genomics, High Wycombe, UK) according to manufacturers' instructions. The purified library was run on a TapeStation 2200 (Agilent Technologies, Santa Clara, CA, USA) and quantified via Qubit (Life Technologies, ThermoFisher Scientific, Waltham, MA USA) to assess quality of the library.

Reproducibility of the technique was tested by preparing biological replica libraries, using different RNA preparations from the same cell lines.

### Sequencing and annotation

Libraries were sequenced on the Illumina NextSeq platform (Illumina, San Diego, CA) at the Radboudumc sequencing facility to produce 2x 150 bp paired-end reads. Reads were mapped to the reference transcriptome (hg19) using the SeqNext module of JSI SequencePilot version 4.2.2 build 502 (JSI Medical Systems, Ettenheim, Germany). The random 8 nt sequence flanking the ligation probe was used to reduce PCR amplicates to one smMIP (unique reads).

### Single nucleotide variant (SNV) calling and expression analysis -

All single nucleotide variants (SNVs) called with a minimal variant percentage of 5% detected in at least 5 unique reads (forward and reverse) were selected for further analysis. Variants were annotated and classified into synonymous or non-synonymous. Next, they were validated in whole transcriptome RNAseq data, generated from different RNA isolations from the same cell lines.

Individual read counts for each smMIP were divided by the total read count within a sample and multiplied by 10⁶ resulting in a fragment per million (FPM) value for each smMIP in a sample. We choose for this normalization procedure instead of normalization against housekeeping genes because perfect housekeeping genes do not exist. E.g. expression of metabolic genes is subject to variation, dependent on cell cycle, and the same is true for expression of genes such as actin and tubulin.

### Western blotting -

Cell extracts were prepared from SKRC7 and SKRC7-VHL^{HA} cells by solubilizing in RIPA buffer (Cell Signaling) and protein concentrations were determined using BCA assays. 20 µg of protein was separated on 12% SDS-PAGE gels and electroblotted on nitrocellulose. After blocking in Odyssey blocking buffer (1:1 in PBS) membranes were incubated overnight in Odyssey blocking buffer containing antibodies against HK-2 (2867S, Cell signaling technology), CA9 (M75, Dr. Oosterwijk) or γ-tubulin (C20, Santa Cruz Biotechnology, Dallas, TX) as loading control. Antibodies were detected with secondary antibodies conjugated with Alexa680 or DyLight800, and signal was visualized with the Odyssey scanner (LI-COR).

### Statistics

FPM values for each transcript (mean FPM values from all smMIPs targeting one transcript) were correlated with TPM values (transcripts per million values) for the same transcript obtained from whole RNAseq data from the same cell lines. For three samples replicate assays were performed. Correlation analyses were performed using GraphPad Prism v.5.03 (GraphPad, San Diego, CA, USA).

### Results

SmMIP-based next generation sequencing (NGS) of genomic DNA was recently introduced in routine diagnostics in our institute to detect tumor-associated mutations in DNA (16). To investigate whether smMIPs can also be used for multiplex determination of gene expression levels, concomitant with variant detection, we designed a smMIP set for targeted detection and sequencing of transcripts encoding metabolic enzymes. To establish the strength of the technique we used the SKRC7 and SKRC7-VHL^{HA} isogenic cell line pair as prototypical cell lines in which different metabolic pathways prevail. Like 80% of ccRCCs, the SKRC7 cell line carries a defective VHL gene resulting in constitutive stabilization of HIF-1α and HIF-2α and a pseudohypoxic response (53, 54, 58). Re-introduction of VHL was expected to result in rapid HIF1/2 breakdown and repair of this metabolic aberration.

Whole RNAseq-derived gene expression data of SKRC7 cells confirmed the presence of a nonsense and functionally inactivating Q132-stop mutation in 100% of VHL transcripts (Figure 2A) whereas only wtVHL sequence was detected in the SKRC7VHL^{HA} (Figure 2B), and this was readily reproduced in the smMIP assay (Figure 2C,D). Introduction of functional VHL^{HA} in SKRC7 cells resulted in 100-fold increase in VHL expression (Figure 2E, see also western blot in figure 2F ).

### Optimization of library preparation

Using an initial set of 642 smMIPs, covering 104 transcripts of interest for this study (see Table I), we tested our protocol of library preparation with 50 ng of hexamer-primed cDNA generated from 13 different RNA samples (cell line -and xenograft derived) of which also whole RNAseq datesets were available. A 25-cycle PCR with barcoded primers on the circularized smMIP library yielded PCR fragments of the expected size of 266 bp (not shown).

Based on initial experiments we also tested the procedure on 10 and 25 ng cDNA. Both conditions yielded less PCR fragments and less unique reads compared to 50 ng. We therefore continued with 50 ng cDNA input in subsequent experiments. Illumina NextSeq sequencing of the libraries generated of SKRC7 and SKRC7-VHL cells, yielded 286,000 and 69,000 annotated unique reads respectively (corrected for PCR-amplicates based on the random 8N sequence in the smMIP), which is in the range of other samples run with the same smMIP panel (not shown). For most transcripts performance of individual smMIPs was variable (see example in Table II, showing FPM values for 10 different smMIPs designed against the VHL transcript in both cell lines), a known phenomenon also in DNA smMIP NGS (16)). This was a priori reason to include at least 5 smMIPs per gene transcript in our panel, allowing transcriptome analysis using mean normalized smMIP values for each transcript. This number was a trade-off between generating expensive, large panels which would yield in part futile and irrelevant data, and too small panels resulting in under- or overestimation of transcript levels.

First we compared the targeted smMIP RNAseq dataset, generated with a 864 smMIP panel, to a whole transcriptome RNAseq dataset (considered as gold standard), performed on different RNA isolates from the same cell lines. The whole RNAseq dataset consisted of 3.2x10⁷ and 3.4x10⁷ reads, assigned to 44,503 different transcripts for SKRC7 and SKRC7-VHL^{HA}, respectively. For each transcript of interest, TPM (transcripts per million) values from the whole RNAseq dataset were plotted against mean FPM values from smMIP analyses. Such analysis for metabolic transcripts and tyrosine kinase transcripts separately, gave correlation coefficients of 0.903 and 0.974, respectively, for SKRC7 (Figure 3A,B) and 0.784 and 0.903, respectively, for SKRC7-VHL^{HA} (Figure 3C,D), suggesting that, as expected, expression of metabolic genes is subject to more variation than of tyrosine kinases. Plotting whole transcriptome RNAseq data against unique reads obtained with the best performing smMIP per transcript, or the median of unique reads for each transcript (to prevent bias by non- or poor-performing smMIPs) did not improve this correlation (not shown).

One of the appealing characteristics of targeted RNAseq using smMIPs is that panels can be expanded to detect novel transcripts of interest. To test how this affects the outcome of the assay, we added 222 smMIPs for detection and targeted sequencing of other transcripts of interest to our initial panel and re-performed the assay using newly isolated RNA from the same cell line. Relative levels of transcripts within samples correlated well between assays with the initial and the expanded smMIP set (SKRC7: r=0.903, SKRC7-VHL^{HA}: r=0.876).

### Functional validation of targeted smMIP data

Having confirmed the validity of the smMIP dataset, we analyzed expression levels of genes involved in metabolism in SKRC7 and SKRC7-VHL^{HA} cells. Figure 4A and 4B show two biological duplicates of smMIP-based mean FPM values for a number of transcripts involved in glycolysis. Expression of HIF target genes glucose transporter 1 and 3 (SLC2A1 and SCL2A3), monocarboxylate transporter MCT4 (SLC16A3), carbonic anhydrases 9 and 12 (CA9, CA12), hexokinase 2 (HK2), lactate dehydrogenase A (LDH-A) and phosphoglycerate kinase (PGK1) were significantly and reproducibly reduced in SKRC7-VHL^{HA} cells relative to SKRC7 cells (Figure 4A,B), in line with data obtained from whole transcriptome RNA seq data (figure 4C). Relative expression levels of CA9 and HK2 transcript levels were further confirmed on the protein level (Figure 4D). The strong reduction of CA9, HK2 and LDHA, all target genes of HIF, was in line with expectations for a VHL-defective cell line.

### Variant detection

To investigate whether smMIP based RNAseq allows efficient detection of single nucleotide variants (SNVs), we performed variant calling of the smMIP library in SeqNext. Several heterozygous and homozygous variants were detected that could be validated in the whole RNAseq dataset (see VHL example in Figure 2C and D). We then further validated the sensitivity of the assay to detect SNVs (called a variant in relation to reference genome hg19) and performed smMIP analysis on RNA, isolated from the IDH1^{R132H} mutant oligodendroglioma line E478 (56) and the astrocytoma cell line E98, in which we previously identified a novel mutation in IDH1 (IDH1^{R314C})(1). Both mutations were identified (Figure 5A,B).

### Discussion

Here we present a novel approach of library generation for targeted RNA next generation sequencing using smMIPs and show that the technique yields reproducible and biologically relevant information which is qualitatively comparable to whole transcriptome RNAseq. Especially for the evaluation of relative contributions of metabolic pathways in cancer, that are amenable to epigenetic and transcription-factor based regulation, DNA sequencing may not always yield relevant information. Using an isogenic pair of cell lines differing only in VHL expression as a test case, we here show that targeted RNA seq of transcripts involved in metabolism with our smMIP panel yields relevant information on metabolic pathways with relative abundancies that are similar to that of whole transcriptome RNAseq.

Although the generation of smMIP libraries for targeted RNAseq obviously yields only a fraction of the data compared with whole transcriptome RNAseq, it has distinct advantages: 1) costs of the technique are approximately 5-10% of the cost of whole transcriptome RNAseq; 2) by designing smMIPs with ligation and extension probes localized on neighbouring exons, the library is expected not to be contaminated with heteronuclear RNA, transfer RNAs and ribosomal RNAs that may account for a large number of reads in whole transcriptome RNAseq, dependent on preprocessing; 3) the choice of extension and ligation target sequences allows the design of smMIPs that specifically detect splice variants; 4) the technique allows detection of SNVs and indels with high efficiency, once smMIPs are chosen to cover the mutated region; 5) coverage per target sequence of transcript of interest is higher than with whole transcriptome RNAseq; 6) smMIP sets may be extended with novel smMIPs of interest without affecting performance; 7) data sets are much smaller and easier to handle.

The technique was validated with an isogenic ccRCC cell line pair, differing only in expression of VHL. Our targeted smMIP analysis revealed that, as expected, expression levels of HIF target genes HK-2, CA9, CA12 and LDH-A were downregulated upon rescue of VHL, a known regulator of HIF proteolysis. This suggests that rescue of VHL function reduces flux of glucose into the glycolytic pathway.

Because altered metabolic activity in cancer can be a consequence of general oncogenic stress but also of mutations in genes encoding metabolic enzymes and conditions relating to the microenvironment (oxygen tension, access to stromal cell-derived metabolites (59)) cancer metabolism is in an extremely complex landscape (60). Nevertheless, targeting of cancer-specific metabolic pathways is gaining importance in cancer research. Since decades glycolysis has been considered the predominant metabolic pathway in cancer, but it is increasingly clear that tumors can also thrive using glutamine as carbon and nitrogen donor. Identification of the fuel-processing pathways that represent metabolic Achilles heels in cancer is important to apply metabolic inhibition in a personalized fashion. Approaches to identify metabolic pathways in clinical cancers currently include carbon tracing using *ex vivo* mass spectroscopy (59, 61, 62) and *in vivo* ¹H-, ¹³C carbon- or ³¹P-based magnetic resonance spectroscopic imaging (40, 63) but these approaches are not suitable for implementation in routine patient care. SmMIP-based transcript profiling may be a highly relevant alternative with added value in the field of cancer diagnostics as it can identify metabolic Achilles heels by simultaneously measuring smart combinations of relative gene expression levels and variants. When combined with smMIP sets that detect actionable mutations in oncogenes or tumor suppressor genes, personalized treatment protocols may be further optimized by including inhibitors of the most predominant metabolic pathways such as glycolysis (e.g. 3-bromopyruvate, dichloroacetate(64-66)), pentose phosphate pathway (e.g. 6-aminonicotinamide (33), glutaminolysis (e.g. epigallocathechin-3-gallate(67, 68)), mitochondrial oxidative phosphorylation (e.g. metformin(69-71)), fatty acid oxidation and lipid synthesis (e.g. cerulenin (72)).

### Example 2 - Glutaminolysis in cancers predicts enhanced sensitivity to a combination of epigallocatechin-3-galate (EGCG) and radiotherapy as compared to radiotherapy alone

Clear cell renal cell carcinoma (ccRCC) are relatively resistant to radiotherapy and chemotherapy. Due to dysfunctional von Hippel-Lindau (VHL) protein these tumors accumulate hypoxia-inducible factors HIF-1α and HIF-2α resulting in pseudohypoxic responses that accompanying aberrant metabolism (49, 73). This translates in expression of a set of transporters and enzymes that increase glucose uptake for use in aerobic glycolysis and lactate production, instead of oxidative phosphorylation (73). Increased uptake of glucose and its conversion to glucose-6-phosphate by the hexokinase family of enzymes leads to an increased flux through the pentose phosphate pathway (PPP), providing the cell with NADPH, the most important form of reducing power in cells, and ribose-5-phosphate (R5P), a precursor of nucleotide synthesis (33). Whereas in normal cells oxidative glucose metabolism yields mitochondrial citrate as a major carbon source for lipid biosynthesis, this pathway is blocked in VHL-deficient cells that process pyruvate towards lactate instead of acetyl-CoA for TCA cycle feeding (74). Cells with a VHL defect therefore use glutamine as a metabolic rescue pathway. During glutaminolysis, glutamine is converted to glutamate and α-KG via the sequential activities of glutaminase and glutamate dehydrogenase. Subsequently cells employ reductive carboxylation of α-ketoglutarate (α-KG) in the cytoplasm to produce isocitrate (reverse reaction of IDH1) that is converted to citrate (aconitase) and acetyl-CoA (ATP-citrate lyase) that, together with NADPH, is processed to fatty acids (75). In VHL-mutated cancers high expression levels of enzymes of the pentose phosphate pathway (PPP), combined with low levels of TCA enzymes correlates with poor survival (76).

In ccRCC differential expression of HIF-1α and HIF-2α is observed, with tumors expressing either both subtypes or exclusively HIF-2α. Part of the effects of HIF-1α and HIF-2α are overlapping, but they also have distinct effects on cell metabolism. HIF-1α causes glycolytic enzyme expression (77) and limits mitochondrial pyruvate consumption (78, 79), thereby blocking anabolic biosynthesis via this pathway. Furthermore HIF-1α inhibits cell cycle progression via inhibition of c-myc (80). HIF-2α however, does not regulate glycolysis and stimulates cell-cycle progression (81). The exact metabolic pathways may therefore differ between different VHL-mutated cancers. Unraveling the metabolic pathways of cancer cells that facilitate malignant behavior is of high importance, since these may be amenable for targeting with the aim to inhibit cell growth and tumor progression, or induce oxidative stress sensitizing cells to radiotherapy or chemotherapy.

Here we investigated the metabolic pathways in two VHL impaired ccRCC cell lines, SKRC-17 (expressing only HIF-2) and SKRC-7 (expressing both HIF-1 and HIF2) (54, 58). Expression of metabolic enzymes was explored with smMIP sequencing, and carbon sources that are essential for proliferation of these cells were identified. Results show that SKRC-7 cells use glucose for lactate production (high levels of enzymes for glucose-to-pyruvate-to-lactate). Gene expression profiles of SKRC-17 suggest that cells use glucose mainly for the pentose phosphate pathway. Both cell types have high levels of glutaminase and glutamate dehydrogenase, suggesting sensitivity to the glutamate dehydrogenase inhibitor EGCG. The high levels of PPP in SKRC17 suggest additional activity of 6-aminonicotinamide (6-AN).

### Materials and methods

### Cell culture

SKRC-7, derived from primary human RCC, and SKRC-17, derived from a soft tissue metastatic lesion of human RCC (82) both carry a non-sense mutation in *VHL* (Q132X in SKRC-7 and S65X in SKRC-17) and therefore lack functional pVHL. In SKRC-7 this leads to high levels of HIF-1α and HIF-2α, whereas SKRC-17 presents with high levels of HIF-2α only (54, 58). Unless stated otherwise, cells were cultured in RPMI 1640 (Lonza Group, Switzerland) supplemented with 10% fetal calf serum (FCS) (Gibco, Thermo Fisher Scientific, Waltham, MA, USA) and 40 µg/ml gentamycin (Centrafarm, Etten-Leur, The Netherlands).

### SmMIP sequencing

642 smMIPs (IDT, Leuven, Belgium) were pooled at 100 µM/smMIP. The smMIP pool was phosphorylated using T4 Polynucleotide Kinase (New England Biolabs, NEB, Ipswich, MA, USA) in T4 DNA ligase buffer (NEB) at 37°C for 45 min, followed by inactivation for 20 min at 65°C. The capture reaction was performed with 50 ng of cDNA and an estimated 8000-fold molar excess of the phosphorylated smMIP pool (16) in a 25 µL reaction mixture containing Ampligase buffer (Epicentre, Madison, WI, USA), dNTPs, Hemo KlenTaq enzyme (New England Biolabs, NEB, Ipswich, MA, USA) and thermostable DNA ligase (Ampligase, Epicentre). The capture mix was incubated for 10 min at 95 °C (denaturation), followed by incubation for 18 h at 60 °C, during which hybridization and concomitant primer extension and ligation occurs. Directly after this step, non-circularized smMIPs, RNA and cDNA were removed by treatment with 10 U Exonuclease I and 50 U of Exonuclease III (both NEB) for 45 min at 37°C, followed by heat inactivation (95°C, 2 min). The circularized smMIP library was subjected to standard PCR with 2x iProof High-Fidelity DNA Polymerase master Mix (Bio-Rad, Hercules, CA) with a primer set containing a unique barcoded reverse primer for each sample. Generation of PCR products of correct size (266 bp) was validated on agarose gel electrophoresis, and PCR-libraries from different samples were pooled based on relative band intensity. The pool was then purified using AMPureXP beads (Beckman Coulter Genomics, High Wycombe, UK) according to manufacturers' instructions. The purified library was run on a TapeStation 2200 (Agilent Technologies, Santa Clara, CA, USA) and quantified via Qubit (Life Technologies, ThermoFisher Scientific, Waltham, MA USA) to assess quality of the library.

Reproducibility of the technique was tested by preparing biological replica libraries, using different RNA preparations from the same cell lines.

### Western blotting

To verify transcript levels observed in the smMIP sequencing data on protein level, western blots for some of the interesting enzymes active in glycolysis (HK2, PKM2, GAPDH), glutaminolysis (GLUD) and reverse carboxylation (IDH1) were performed. Cells were cultured in 6 well plates till 80% confluency, then cells were harvested and processed to cell lysates by scraping in 100 µl 10 mM Tris-HCL pH 7.5 and 0.32 M sucrose and sonicating on ice (3 cycles of 30 sec max power and 30 sec off, Bioruptor, Diagenode). Lysates were centrifuged (14000 rpm, 10 min, 4°C) and supernatants were subjected to BCA assays (Pierce, Thermo Fisher Scientific, Waltham, MA, USA) for protein concentration measurements. 20 µg of total cytosolic protein was subjected to SDS-PAGE and electroblotted onto a nitrocellulose membrane (Whatman Optitran BA-S85, GE healthcare, Little Chalfont, UK). After blocking in Odyssey Blocking buffer (LI-COR biosciences, Licoln, NE, USA) in PBS (1:1) the membrane was incubated with mouse-anti-HA (1:500, Sc-7932, Santa cruz, Dallas, TX, USA), rabbit-anti-GLUD (1:400, GTX105765, GeneTex Inc, San Antonio, TX, USA), rabbit-anti-IDH1 (1:1000, HPA035248, Sigma Aldrich, St. Louis, MO, USA), mouse-anti-GAPDH (1:10,000, ab8245, Abcam, Cambridge, US), rabbit-anti-HK2 (1:1000, 2867S, Cell Signaling Technology, Danvers, MA, USA), rabbit-anti-PKM2 (1:1000, D78A4, Cell Signaling Technology, Danvers, MA, USA) and goat-anti-γtubulin (1:5000, sc-7396, Santa Cruz, Dallas, TX, USA) in blocking buffer, followed by incubation with goat-anti-mouseDyLight800 (1:10.000, Thermo Fisher Scientific, Waltham, MA, USA), goat-anti-rabbitAlexa680 (1:10.000, Invitrogen, Waltham, MA, USA), or donkey-anti-goatAlexa680 (1:10,000, Invitrogen, Waltham, MA, USA) in blocking buffer. After washing, blots were analyzed on the Odyssey scanner (LI-COR biotechnology, Lincoln, NE, USA). Signals were corrected for γ-tubulin and the mean of 3 independent experiments is plotted. Statistical significance was determined with an unpaired Student's T-test.

### Cell proliferation assays

Cellular protein content was determined as a measure of cell proliferation, using suforhodamine B (SRB) assays as described in (83). Sensitivity to EGCG was determined by adding a concentration range of EGCG (0-50 µM) or DMSO solvent one day after seeding 1,000 cells per well in 96-wells plates (Nunc, Roskilde, Denmark). An SRB assay was performed after 3 days, and IC50 values were determined in GraphPad Prism using the sigmoidal dose response with variable slope nonlinear regression analysis.

Furthermore cell proliferation over 8 days in presence or absence of EGCG was determined. Cells were seeded at 1,000 cells per well and at day 1 and day 5 after seeding the medium was changed for medium with or without 10 µM EGCG. Controls were incubated with DMSO. Protein content was determined every 2 days. Experiments were performed in triplicate and statistical significance was determined using one-way ANOVA with bonferroni correction.

To determine the sensitivity of the cells to glutamine or glucose deprivation, the regular medium was changed for either D-glucose depleted (0 g/L D-glucose and 4 g/L L-glutamine), L-glutamine depleted (1 g/L L-glutamine and 5 g/L D-glucose) or regular RPMI medium supplemented with 10% FCS and antibiotics with or without 10 µM EGCG one day after seeding the cells. Again protein content was determined every 2 days. Experiments were performed in triplicate and statistical significance was determined using one-way ANOVA with bonferroni correction.

### Cellular and mitochondrial respiration

Cells were grown till 80% confluency in culture flasks, and after trypsinization 1.5*10⁶ cells were resuspended in culture medium and transferred to the thermostated (37°C) chamber of an Oxygraph-2k equipped with Datlab recording and analysis software (Oroboros Instruments, Innsbruck, Austria). The basal respiration was measured and then the remaining mitochondrial respiration was inhibited with 2.5 µM complex V inhibitor oligomycin. Then maximal respiration was measured by sequential addition of 0.5 µM mitochondrial uncoupler FCCP. Subsequently 0.5 µM complex I inhibitor Rotenone and 2.5 µM complex III inhibitor Antimycin A were added to completely shut down the electron transport chain. The remaining oxygen consumption is due to non-mitochondrial respiration. Two separate experiments were performed and significance was determined with an unpaired Student's T test.

### Radiotherapy experiments

Since SKRC-7 and SKRC-17 cells are unable to grow as colonies, sensitivity to radiotherapy was analyzed by monitoring cell proliferation with the xCELLigence. This method has been shown to measure effects of radiotherapy that correlate with the conventional clonogenic assays (84). Cells were plated at 1,000 cells per well and left to adhere overnight. Then cells were treated with 10 µM EGCG for 24 hrs, after which they were irradiated with 4 Gy (IR, 3.1Gy/min;XRAD 320 ix, Precision XRT; N. Brandford, CT, USA). The cell index was measured from the moment of seeding for 200 hrs in real time with intervals of 15 min, fresh medium supplemented with 10 µM EGCG was added every 72 hours. Cell index was normalized to the moment of applying radiotherapy, and cell growth was calculated by performing linear regression in GraphPad Prism. The experiment was performed with two internal duplicates, and statistical significance was determined with an unpaired Student's T test.

### Results

### VHL rescue causes differential changes in metabolism of SKRC-7 and SKRC-17

SKRC7 and SKRC17 present with different expression profiles (Figure 6A). Levels of PGK1 and PDK1 transcripts were 3-fold lower in SKRC17 than in SKRC7, suggesting relatively inefficient processing of glucose to pyruvate in SKRC17. On the other hand, in this cell line enzymes of the PPP (G6PD and RPIA) were upregulated compared SKRC7. To test whether this difference is reflected in altered sensitivity to the PPP inhibitor 6-AN, we tested this compound in proliferation assays. Of note, whereas SKRC-7 cells surprisingly responded with an increase of cell proliferation, SKRC-17 cells responded by significantly decreased cell proliferation. The high levels of glutamine and glutamate-processing enzymes suggest sensitivity to the GLUD1 inhibitor EGCG. A combination of EGCG and 6-AN was able to completely block cell growth (Figure 6B).

### Example 3 - smMIP-based targeting sequencing allows the distinction of splice variants

The melanoma cell line Mel57 expresses low levels of vascular endothelial growth factor (VEGF-A). VEGF-A consists of different splice variants, consisting of exons 1-5,8 (VEGF-A121), exons 1-5,7,8 (VEGF-165) and exons 1-8 (VEGF-189). These variants have differential activities, ranging from vessel dilatation to full neo-angiogenesis (85). We designed smMIP165 that has its ligation and extension probes in exon 5 and 7, smMIP121 that has its ligation and extension probes in exon 5 and 8, and smMIP189, that has its ligation and extension probes in exon 5 and 6. We performed the smMIP assay with a panel including smMIP121, smMIP165, smMIP189 and 5 smMIPs located in exons 1-5, recognizing all isoforms of VEGF on RNA isolated from the Mel57 cell line and from cell lines expressing the different VEGF isoforms, as described in (85). Figure 7 and the accompanying table show that the different isoform-specific smMIPs specifically recognize the splice variants.

Cancer cells can induce changes in RNA splicing events if these give the cells a growth advantage.

These changes may be an inherent characteristic of a cancer, but may also be selected under pressure of treatment. An example is EGFR variant III that results from an intragenic deletion in the EGFR gene that results in loss of exons 2 to 7 in the mature transcript. Whereas 50% of glioblastomas are characterized by amplification of the EGFR oncogene, in 50% of this population expression of EGFRvIII is found. By placing extension and ligation probes of an individual smMIP in exons 1 and 8, respectively, only the exon 1-8 fusion product is detected, because the backbone sequence of 40 nucleotides is physically not able to bridge exons 2-7 in the wild-type transcript (Figure 9 shows that in the group of gliomas there is elevated expression of EGFR in 39/75 brain tumors (52%; mean FPM 738 in positives vs mean FPM 35 in negatives, using an arbitrary cut off FPM value of 100) and expression of EGFRvIII in 12/75 brain tumors (16%; mean FPM 642 in positives vs mean FPM 0.27 in negatives, using an arbitrary cut-off value of 6). Thus, smMIP based detection of EGFRvIII is highly specific and highly sensitive.

Another example is androgen receptor in prostate cancer. Patients with castration-resistant prostate cancer are treated with enzalutamide. However, a change in splicing that results in loss of exon 7 (ARv7) results in resistance to enzalutamide. By designing a smMIP with extension and ligation probe arms in exons 6 and 8, respectively, Arv7 is readily detected in cell lines derived from enzalutamide- resistant cancers, while it is not detected in any other cancer type (FPM=277 and 640 in VCAP and DuCAP prostate cancer lines, respectively vs mean FPM=0.01 in 130 other cancers).

### Example 4 - smMIP based targeting sequencing can be used for accurate diagnosis

A sample of brain tumor tissue, obtained from patient N16-10 who signed informed consent for the study, was snap-frozen directly after surgery. RNA was isolated from the tissue via the Trizol protocol, followed by cDNA synthesis and preparation of the smMIP library. After Illumina next generation sequencing a mutation in the IDH1 gene was identified that corresponds to the hotspot IDH1R132H mutation. The same analysis revealed low levels of carbonic anhydrase 9 and hexokinase 2, indicating lack of hypoxic responses. Furthermore the sample shows low ratios of glutaminase/glutamate dehydrogenase, suggesting that the tumor was using glutamate for metabolism, and therefore suggesting sensitivity to glutamate dehydrogenase inhibitors such as EGCG and chloroquine. Furthermore, the data show high expression levels of TrkB and, to a lesser extent, PDGFRα. The absence of hypoxia suggests that the tumor was not of a World Health Organization guidelines-defined grade IV type. The presence of high levels of tyrosine kinases suggests astrocytoma, and based on the data a diagnosis of IDH1-mutated grade III astrocytoma was made, concordant with the original diagnosis that was set on histopathology.

### Example 5 - smMIP based targeting sequencing can be used for accurate diagnosis and prognosis

The data of example 4 were expanded with 74 additional samples of brain tumor tissues, obtained from patients who all signed informed consent for the study. The samples were snap-frozen directly after surgery and treated similarly to what has been described in example 4: RNA was isolated from the tissues via the Trizol protocol, followed by cDNA synthesis, preparation of the smMIP libraries and barcoded PCR. After Illumina next generation sequencing FASTQ files were processed by SeqNext software (JSI SequencePilot version 4.2.2 build 502 [JSI Medical Systems, Ettenheim, Germany]). All reads were mapped against the human genome (version hg19) and against manually added variant transcripts (e.g. EGFRvIII, Arv7, METd7/8, METd14). Thus, for every tumor sample a list of targeted transcript levels was generated and a list of all detected mutations/variations. From all 75 patients fully documented clinical follow-up was available.

In a first step, we performed unsupervised agglomerative clustering of log-transformed expression levels of the targeted genes of interest. Agglomerative clustering was performed according to Ward's method by calculating Manhattan distance between individual profiles using bio-informatic R-software scripts. The profiles were translated in a heat map which is represented in Figure 10a. As is shown the computer generates two main groups A and B, that are subdivided in a number of subgroups.

In a next step, potential associations of the clusters with overall survival was investigated by now including survival data for the patients (overall survival, counted from first diagnosis) and generating a Kaplan-Meyer curve. The results in Fig. 10b show that the computer-generated groups have different survival with high significance (Fisher's exact test; p<0.0001). This shows that for gliomas this test has high prognostic value.

In a third step, associations between groups and mutations were analyzed by including the list of all detected mutations in a sample. Groups A and B were distinguished by mutations in the isocitrate dehydrogenase genes (IDH1R132 and IDH2R172) with high significance (p< 10E-11).

Fig. 10c shows an example of the heterozygous IDH1R132H detection in one of the samples, in this case with 38% of transcripts being from the mutant allele and 62% of transcripts from the wt allele. The difference in transcript frequency is attributable to genetically normal stromal cells with only wild type IDH transcripts.

In a fourth step we performed a subgroup analysis to further refine prognosis. Analyzing IDH-wild-type patients with very poor survival (OS<12 months) versus IDH-wild-type patients with better prognosis (group B in the Kaplan-Meyer curves) in such subgroup analysis showed that high expression levels of carbonic anhydrase 12 are associated with extremely poor prognosis (p<0.001; Fisher's exact test, Fig. 10d).

In a fifth step we retrospectively analyzed all data with respect to molecular information that was obtained during routine patient care. All mutations that we observed on the RNA level and that are routinely tested for in glioma patient care, were confirmed with DNA sequencing technology (Table III)

The profiles also reveal expression of genes in brain tumors that are associated with other cancers. An example is the androgen receptor that is often expressed at high levels in prostate carcinoma, and prostate specific membrane protein (PSMA). Other groups have described expression of this target on blood vessels in malignant tumors, including glioma (87). To investigate this further we analyzed tumors with high and low PSMA transcript levels using immunhistochemisty. Results indeed revealed blood vessel expression of PSMA protein in blood vessels from tumors with high transcript levels, and not in tumors with low transcript levels (see three examples in Figure 11).

Table III: Clinical characteristics. Diagnosis, histological type, and percentage tumor cells were confirmed by a trained pathologist (BK). Annotations as marked in this table are used in the heatmap of Fig. 10a.

Abbreviations M, male; F, female; IDH, isocitrate dehydrogenase; WT, IDH-wild type.
* data not available

| **Sample name** | **Sex** | **Age (at time of surgery)** | **Histological type** | **IDH mutation** | **% tumor cells** |
|---|---|---|---|---|---|
| **13-02** | M | 40 | Astrocytoma | IDH1-R132H | 70 |
| **13-03** | M | 58 | Oligodendroglioma | IDH1-R132H | 70 |
| **13-04** | F | 62 | Glioblastoma | WT | 60 |
| **13-06** | M | 53 | Oligodendroglioma | IDH2-R172K | 60 |
| **13-08** | M | 67 | Glioblastoma | WT | 70 |
| **13-09** | F | 58 | Glioblastoma | WT | 70 |
| **13-10** | M | 45 | Oligodendroglioma | IDH1-R132H | 65 |
| **13-11** | F | 67 | Glioblastoma | WT | 70 |
| **13-13** | M | 52 | Glioblastoma | IDH1-V178I | 70 |
| **13-14** | F | 64 | Glioblastoma | WT | 70 |
| **13-15** | F | 44 | Oligodendroglioma | IDH1-R132H | 50 |
| **13-16** | M | 60 | Glioblastoma | WT | 70 |
| **13-17** | M | 45 | Oligodendroglioma | IDH1-R132H | 50 |
| **13-18** | F | 49 | Oligodendroglioma | IDH1-R132H | 50 |
| **14-01** | F | 52 | Glioblastoma | WT | 80 |
| **14-02** | M | 43 | Oligodendroglioma | IDH1-R132H | 50 |
| **14-03** | F | 62 | Glioblastoma | WT | 70 |
| **14-04** | M | 72 | Glioblastoma | WT | 60 |
| **14-05** | M | 21 | Oligodendroglioma | IDH1-R132H | 70 |
| **14-06** | M | 43 | Oligodendroglioma | IDH1-R132H | 50 |
| **14-07** | M | 65 | Oligodendroglioma | IDH1-R132H | 50 |
| **14-08** | M | 50 | Astrocytoma | IDH1-R132H | 50 |
| **14-09** | F | 43 | Astrocytoma | IDH1-R132H | 60 |
| **14-10** | F | 45 | Glioblastoma | IDH1-R132H | 50 |
| **14-11** | M | 50 | Glioblastoma | WT | 60 |
| **14-12** | M | 59 | Oligodendroglioma | IDH1-R132H | 50 |
| **15-01** | M | 66 | Glioblastoma | WT | 50 |
| **15-02** | F | 61 | Glioblastoma | WT | 70 |
| **15-03** | F | 76 | Glioblastoma | WT | 40 |
| **15-04** | F | 59 | Glioblastoma | WT | 40 |
| **15-05** | M | 31 | Astrocytoma | IDH1-R132H | 70 |
| **15-06** | F | 49 | Astrocytoma | IDH1-R132H/V178I | 70 |
| **15-07** | M | 63 | Glioblastoma | IDH1-V178I | 65 |
| **15-08** | M | 55 | Astrocytoma | IDH1-R132H | 60 |
| **15-09** | M | 70 | Glioblastoma | WT | 70 |
| **15-10** | F | 68 | Oligodendroglioma | IDH1-R132H | 70 |
| **15-12** | M | 46 | Glioblastoma | WT | 70 |
| **15-13** | F | 78 | Glioblastoma | WT | 80 |
| **15-14** | M | 79 | Glioblastoma | WT | 70 |
| **15-15** | F | 58 | Glioblastoma | WT | 70 |
| **15-16** | M | 25 | Astrocytoma | IDH1-R132H/V178I | 50 |
| **15-17** | M | 68 | Glioblastoma | WT | 60 |
| **15-18** | F | 64 | Glioblastoma | WT | 70 |
| **16-01** | M | 61 | Glioblastoma | WT | 70 |
| **16-02** | M | 47 | Glioblastoma | WT | 70 |
| **16-03** | F | 46 | Astrocytoma | IDH1-R132H | 25 |
| **16-04** | M | 59 | Oligodendroglioma | IDH1-R132H | 60 |
| **16-05** | M | 51 | Glioblastoma | WT | 50 |
| **16-06** | F | * | Astrocytoma | IDH1-R132H | 50 |
| **16-07** | M | 74 | Glioblastoma | IDH1-Y183C | 60 |
| **16-08** | F | 69 | Glioblastoma | WT | 50 |
| **16-09** | F | 49 | Glioblastoma | WT | 70 |
| **16-10** | M | * | Astrocytoma | IDH1-R132H | 60 |
| **16-11** | M | 67 | Glioblastoma | WT | 50 |
| **16-12** | M | 23 | Astrocytoma | IDH1-R132H | 60 |
| **16-13** | M | 60 | Glioblastoma | WT | 70 |
| **16-14** | F | 60 | Oligodendroglioma | IDH2-R172M | 70 |
| **16-15** | F | 61 | Oligodendroglioma | IDH1-R132H | 70 |
| **16-16** | M | 58 | Glioblastoma | IDH1-V178I | 40 |
| **16-17** | F | 18 | Oligodendroglioma | IDH2-R172K | 40 |
| **16-18** | * | 30 | Oligodendroglioma | IDH2-R172W | 70 |
| **16-19** | M | 48 | Glioblastoma | WT | 70 |
| **17-01** | M | 58 | Oligodendroglioma | IDH1-R132H | 50 |
| **17-02** | M | 40 | Astrocytoma | IDH1-R132H/V178I | 70 |
| **17-03** | F | 76 | Glioblastoma | WT | 65 |
| **17-04** | M | 42 | Oligodendroglioma | IDH1-R132H | 70 |
| **17-05** | M | 59 | Astrocytoma | WT | 70 |
| **17-06** | M | 65 | Glioblastoma | WT | 70 |
| **17-07** | M | 63 | Glioblastoma | WT | 70 |

### Example 6 - metabolism in IDH-mutated glioma

Detailed analysis of expression of metabolic genes revealed that in the group of long survivors low levels of glucose importers, carbonic anhydrase and hexokinase 2 were expressed, indicating lack of hypoxia. Furthermore in this group high levels of glutamate dehydrogenase and aminobutyric acid aminotranferase (ABAT) RNA were observed, suggesting that these tumors use neurotransmitters (glutamate and GABA) for their catabolism, inducing sensitivity to glutamate dehydrogenase- and ABAT inhibitors (epigallocatechin-3-gallate, vigabatrine).

### Example 7 - Tyrosine kinases in glioma

In the group of IDH-mutated gliomas high expression levels of TrkB (mean FPM 15833 vs 4708 in IDHmut vs IDHwt, p=2x10E-11) were detected, suggesting sensitivity to the TrkB inhibitor entrectinib. EGFR expression was higher in IDHwt tumor (609 vs 116 in IDHwt vs IDHmut cancers, p=0.004), whereas EGFRvIII was exclusively expressed in this group (201 vs 0.16 in IDHwt vs IDHmut, p=0.0005, see also Figure 9).

Whereas in the group of IDH wild type cancers EGFR/EGFRvIII expression was observed in 52% of samples, the tyrosine kinase MET is observed in 9/75 brain tumors (12%). Of interest, profiles showed that tumors that expressed relatively high levels of MET, were low in EGFR and vice versa (correlation coefficient r= - 0.95). An interesting further finding was occurrence of a mutation in BRAF (BRAF-V600E) in one glioma. Wild-type BRAF is a crucial signaling intermediate that processes signals from activated membrane tyrosine kinases (e.g. EGFR, MET) to the nucleus, thereby inducing cell proliferation. BRAF-V600E is an auto-active molecule that signals to the nucleus without input from receptor tyroinse kinases in an uncontrolled fashion. This BRAF mutation occurs in 50% of melanoma cancers and can be inhibited by the targeted drug vemurafenib. The glioma containing this mutation did not express MET nor EGFR. Although anecdotal, this case suggests susceptibility to vemurafenib and unsusceptibility to EGFR or MET inhibitors.

### Example 7- Tyrosine kinase profiles predict sensitivity and non-sensitivity to targeted therapies in vitro.

The astrocytoma cell line E98 carries an auto-activating mutation in c-MET (22) and is highly sensitive to the multispecific VEGFR2/MET kinase inhibitor cabozantinib (86) and the MET-selective inhibitor Compound A (88). This sensitivity is reflected in decreased MET phosphorylation on western blot and decreased proliferation rates in vitro and delayed tumor development in vivo. The renal cancer cell line SKRC17 expresses similar levels of MET, phosphorylation of which is effectively inhibited by Compound A (Figure 12). Yet, SKRC17 cells do not respond to compound A with decreased proliferation rates. Profiling of membrane tyrosine kinases reveals that within the selected group of membrane tyrosine kinases that are measured in the assay, MET is the only one expressed by E98, whereas SKRC17 cells express an additional number of other tyrosine kinase inhibitors, including AXL, EGFRs, FGFRs. These results suggest that targeted inhibition of one receptor tyrosine kinase is only effective in the absence of rescue kinases, and that effective treatment of a cancer requires concomitant blockade of all membrane receptor tyrosine kinases on a cell.

### Example 8 - HPV RNA profiling reduces false positive outcomes of HPV-DNA -based population based screening

The Netherlands is one of the first countries implementing detection of high risk human papilloma viruses (hrHPV) in cervical swabs in a population-wide screening program, to allow early detection and preventive treatment of cervical cancers. The life-time risk of an HPV infection is 80%, and in the group of participating women 8% will test positive in this assay. It is known on forehand that 90% of these women are overtreated because HPV infections may resolve spontaneously. Furthermore, sex with an HPV positive partner will result in positivity in the sensitive PCR-based HPV DNA detection tests, but does not mean that the virus will actually infects cervical epithelial cells.

To discriminate between contamination and actual cellular infection, we designed smMIPs for detection of hrHPV transcripts E2, E6 and E7, based on available knowledge that loss of E2 gene expression is associated with chromosomal integration in infected cells and overexpression of the HPV-E6 and E7 oncoproteins. With the entire panel of smMIP probes, supplemented with hrHPV-detecting smMIP probes, we profiled a series of 29 gynecological tissues, ranging from normal uterus extirpations to ovarian cancer, endometrial cancers and cervix carcinomas (Figure 13). Samples were profiled blinded to pathology. HPV16 E6/E7 RNA expression was observed in 12 samples. In retrospect, these all were squamous cell carcinomas of the cervix. In a next step we analyzed all samples using the HPV-DNA PCR test. All HPV16-positive samples were confirmed on the DNA level, but 5 tissues that were negative in the HPV-RNA test, were positive in the HPV-DNA test (arrow heads). In retrospect, these samples consisted of two normal uteri and two endometrium carcinomas (that indeed are known not to be HPV-induced). These data clearly show that HPV-RNA screening is capable of reducing the number of false positive testing of HPV-DNA screening.

In a second step we investigated the sensitivity of HPV-testing by profiling 1, 10, 100, 1000, and 10,000 Hela cells, derived 490 years ago from a woman with an HPV-18 positive cervix carcinoma. Profiling of only 1 cell already detected 69 unique HPV18 reads, increasing to 168, 1419, 36767 reads in 10, 100 and 1000 cells respectively.

### REFERENCES

1. van Lith SA, Navis AC, Lenting K, Verrijp K, Schepens JT, Hendriks WJ, et al. Identification of a novel inactivating mutation in Isocitrate Dehydrogenase 1 (IDH1-R314C) in a high grade astrocytoma. Sci Rep. 2016;6:30486.
2. Bardella C, Pollard PJ, Tomlinson I. SDH mutations in cancer. Biochim Biophys Acta. 2011;1807(11):1432-43.
3. Linehan WM, Rouault TA. Molecular pathways: Fumarate hydratase-deficient kidney cancer--targeting the Warburg effect in cancer. Clin Cancer Res. 2013;19(13):3345-52.
4. Chuang JH, Chou MH, Tai MH, Lin TK, Liou CW, Chen T, et al. 2-Deoxyglucose treatment complements the cisplatin- or BH3-only mimetic-induced suppression of neuroblastoma cell growth. Int J Biochem Cell Biol. 2013;45(5):944-51.
5. Lis P, Dylag M, Niedzwiecka K, Ko YH, Pedersen PL, Goffeau A, et al. The HK2 Dependent "Warburg Effect" and Mitochondrial Oxidative Phosphorylation in Cancer: Targets for Effective Therapy with 3-Bromopyruvate. Molecules. 2016;21(12).
6. Pedersen PL. 3-Bromopyruvate (3BP) a fast acting, promising, powerful, specific, and effective "small molecule" anti-cancer agent taken from labside to bedside: introduction to a special issue. J Bioenerg Biomembr. 2012;44(1):1-6.
7. Haghighat N, McCandless DW. Effect of 6-aminonicotinamide on metabolism of astrocytes and C6-glioma cells. Metab Brain Dis. 1997;12(1):29-45.
8. Song IS, Han J, Lee HK. Metformin as an anticancer drug: A Commentary on the metabolic determinants of cancer cell sensitivity to glucose limitation and biguanides. J Diabetes Investig. 2015;6(5):516-8.
9. Flavin R, Peluso S, Nguyen PL, Loda M. Fatty acid synthase as a potential therapeutic target in cancer. Future Oncol. 2010;6(4):551-62.
10. Burchardt P, Rzezniczak J, Dudziak J, Dzumak A, Marchlewski T, Ganowicz-Kaatz T, et al. Evaluation of plasma PCSK9 concentrations, transcript of LDL receptor, as well as the total number of monocyte LDL receptors in acute coronary syndrome patients. Cardiol J. 2016;23(6):604-9.
11. Lord CJ, Ashworth A. Mechanisms of resistance to therapies targeting BRCA-mutant cancers. Nat Med. 2013;19(11):1381-8.
12. Hata AN, Niederst MJ, Archibald HL, Gomez-Caraballo M, Siddiqui FM, Mulvey HE, et al. Tumor cells can follow distinct evolutionary paths to become resistant to epidermal growth factor receptor inhibition. Nat Med. 2016;22(3):262-9.
13. Chong CR, Janne PA. The quest to overcome resistance to EGFR-targeted therapies in cancer. Nat Med. 2013;19(11):1389-400.
14. Bouwman P, Jonkers J. Molecular pathways: how can BRCA-mutated tumors become resistant to PARP inhibitors? Clin Cancer Res. 2014;20(3):540-7.
15. Hiatt JB, Pritchard CC, Salipante SJ, O'Roak BJ, Shendure J. Single molecule molecular inversion probes for targeted, high-accuracy detection of low-frequency variation. Genome Res. 2013;23(5):843-54.
16. Eijkelenboom A, Kamping EJ, Kastner-van Raaij AW, Hendriks-Cornelissen SJ, Neveling K, Kuiper RP, et al. Reliable Next-Generation Sequencing of Formalin-Fixed, Paraffin-Embedded Tissue Using Single Molecule Tags. J Mol Diagn. 2016;18(6):851-63.
17. Tonjes M, Barbus S, Park YJ, Wang W, Schlotter M, Lindroth AM, et al. BCAT1 promotes cell proliferation through amino acid catabolism in gliomas carrying wild-type IDH1. Nat Med. 2013;19(7):901-8.
18. Boyle EA, O'Roak BJ, Martin BK, Kumar A, Shendure J. MIPgen: optimized modeling and design of molecular inversion probes for targeted resequencing. Bioinformatics. 2014;30(18):2670-2.
19. Luo J, Manning BD, Cantley LC. Targeting the PI3K-Akt pathway in human cancer: rationale and promise. Cancer Cell. 2003;4(4):257-62.
20. Carracedo A, Pandolfi PP. The PTEN-PI3K pathway: of feedbacks and cross-talks. Oncogene. 2008;27(41):5527-41.
21. Yin Y, Shen WH. PTEN: a new guardian of the genome. Oncogene. 2008;27(41):5443-53.
22. Navis AC, van Lith SA, van Duijnhoven SM, de Pooter M, Yetkin-Arik B, Wesseling P, et al. Identification of a novel MET mutation in high-grade glioma resulting in an auto-active intracellular protein. Acta Neuropathol. 2015;130(1):131-44.
23. Soussi T, Wiman KG. Shaping genetic alterations in human cancer: the p53 mutation paradigm. Cancer Cell. 2007;12(4):303-12.
24. Sherr CJ, McCormick F. The RB and p53 pathways in cancer. Cancer Cell. 2002;2(2):103-12.
25. Boulton SJ. Cellular functions of the BRCA tumour-suppressor proteins. Biochem Soc Trans. 2006;34(Pt 5):633-45.
26. Burgess DJ. Chromosome instability: Tumorigenesis via satellite link. Nat Rev Cancer. 2011;11(3):158.
27. Casaletto JB, McClatchey Al. Spatial regulation of receptor tyrosine kinases in development and cancer. Nat Rev Cancer. 2012;12(6):387-400.
28. Rocca A, Farolfi A, Bravaccini S, Schirone A, Amadori D. Palbociclib (PD 0332991) : targeting the cell cycle machinery in breast cancer. Expert Opin Pharmacother. 2014;15(3):407-20.
29. Payen VL, Porporato PE, Baselet B, Sonveaux P. Metabolic changes associated with tumor metastasis, part 1: tumor pH, glycolysis and the pentose phosphate pathway. Cell Mol Life Sci. 2016;73(7):1333-48.
30. Patra KC, Hay N. The pentose phosphate pathway and cancer. Trends Biochem Sci. 2014;39(8):347-54.
31. Acharya A, Das I, Chandhok D, Saha T. Redox regulation in cancer: a double-edged sword with therapeutic potential. Oxid Med Cell Longev. 2010;3(1):23-34.
32. Hao J, Graham P, Chang L, Ni J, Wasinger V, Beretov J, et al. Proteomic identification of the lactate dehydrogenase A in a radioresistant prostate cancer xenograft mouse model for improving radiotherapy. Oncotarget. 2016.
33. Lucarelli G, Galleggiante V, Rutigliano M, Sanguedolce F, Cagiano S, Bufo P, et al. Metabolomic profile of glycolysis and the pentose phosphate pathway identifies the central role of glucose-6-phosphate dehydrogenase in clear cell-renal cell carcinoma. Oncotarget. 2015;6(15):13371-86.
34. Cairns RA, Harris IS, Mak TW. Regulation of cancer cell metabolism. Nat Rev Cancer. 2011;11(2):85-95.
35. Yang C, Ko B, Hensley CT, Jiang L, Wasti AT, Kim J, et al. Glutamine oxidation maintains the TCA cycle and cell survival during impaired mitochondrial pyruvate transport. Mol Cell. 2014;56(3):414-24.
36. Shanware NP, Mullen AR, DeBerardinis RJ, Abraham RT. Glutamine: pleiotropic roles in tumor growth and stress resistance. J Mol Med (Berl). 2011;89(3):229-36.
37. Wise DR, DeBerardinis RJ, Mancuso A, Sayed N, Zhang XY, Pfeiffer HK, et al. Myc regulates a transcriptional program that stimulates mitochondrial glutaminolysis and leads to glutamine addiction. Proc Natl Acad Sci U S A. 2008;105(48):18782-7.
38. Lunt SY, Vander Heiden MG. Aerobic glycolysis: meeting the metabolic requirements of cell proliferation. Annu Rev Cell Dev Biol. 2011;27:441-64.
39. Roodink I, van der Laak J, Kusters B, Wesseling P, Verrijp K, de Waal R, et al. Development of the tumor vascular bed in response to hypoxia-induced VEGF-A differs from that in tumors with constitutive VEGF-A expression. Int J Cancer. 2006;119(9):2054-62.
40. Hamans B, Navis AC, Wright A, Wesseling P, Heerschap A, Leenders W. Multivoxel (1)H MR spectroscopy is superior to contrast-enhanced MRI for response assessment after anti-angiogenic treatment of orthotopic human glioma xenografts and provides handles for metabolic targeting. Neuro Oncol. 2013;15(12):1615-24.
41. Altman BJ, Stine ZE, Dang CV. From Krebs to clinic: glutamine metabolism to cancer therapy. Nat Rev Cancer. 2016.
42. Bensaad K, Favaro E, Lewis CA, Peck B, Lord S, Collins JM, et al. Fatty acid uptake and lipid storage induced by HIF-1alpha contribute to cell growth and survival after hypoxia-reoxygenation. Cell Rep. 2014;9(1):349-65.
43. Saxena N, Maio N, Crooks DR, Ricketts CJ, Yang Y, Wei MH, et al. SDHB-Deficient Cancers: The Role of Mutations That Impair Iron Sulfur Cluster Delivery. J Natl Cancer Inst. 2016;108(1).
44. Lussey-Lepoutre C, Hollinshead KE, Ludwig C, Menara M, Morin A, Castro-Vega LJ, et al. Loss of succinate dehydrogenase activity results in dependency on pyruvate carboxylation for cellular anabolism. Nat Commun. 2015;6:8784.
45. Cancer Genome Atlas Research N, Linehan WM, Spellman PT, Ricketts CJ, Creighton CJ, Fei SS, et al. Comprehensive Molecular Characterization of Papillary Renal-Cell Carcinoma. N Engl J Med. 2016;374(2):135-45.
46. Kampjarvi K, Makinen N, Mehine M, Valipakka S, Uimari O, Pitkanen E, et al. MED12 mutations and FH inactivation are mutually exclusive in uterine leiomyomas. Br J Cancer. 2016;114(12):1405-11.
47. Molenaar RJ, Botman D, Smits MA, Hira VV, van Lith SA, Stap J, et al. Radioprotection of IDH1-Mutated Cancer Cells by the IDH1-Mutant Inhibitor AGI-5198. Cancer Res. 2015;75(22):4790-802.
48. van Lith SA, Navis AC, Verrijp K, Niclou SP, Bjerkvig R, Wesseling P, et al. Glutamate as chemotactic fuel for diffuse glioma cells: are they glutamate suckers? Biochim Biophys Acta. 2014;1846(1):66-74.
49. Kim WY, Kaelin WG. Role of VHL gene mutation in human cancer. J Clin Oncol. 2004;22(24):4991-5004.
50. Laukka T, Mariani CJ, Ihantola T, Cao JZ, Hokkanen J, Kaelin WG, Jr., et al. Fumarate and Succinate Regulate Expression of Hypoxia-inducible Genes via TET Enzymes. J Biol Chem. 2016;291(8):4256-65.
51. Losman JA, Kaelin WG, Jr. What a difference a hydroxyl makes: mutant IDH, (R)-2-hydroxyglutarate, and cancer. Genes Dev. 2013;27(8):836-52.
52. Robinson CM, Ohh M. The multifaceted von Hippel-Lindau tumour suppressor protein. FEBS Lett. 2014;588(16):2704-11.
53. Hakimi AA, Reznik E, Lee CH, Creighton CJ, Brannon AR, Luna A, et al. An Integrated Metabolic Atlas of Clear Cell Renal Cell Carcinoma. Cancer Cell. 2016;29(1):104-16.
54. Grabmaier K, MC AdW, Verhaegh GW, Schalken JA, Oosterwijk E. Strict regulation of CAIX(G250/MN) by HIF-1alpha in clear cell renal cell carcinoma. Oncogene. 2004;23(33):5624-31.
55. Claes A, Schuuring J, Boots-Sprenger S, Hendriks-Cornelissen S, Dekkers M, van der Kogel AJ, et al. Phenotypic and genotypic characterization of orthotopic human glioma models and its relevance for the study of anti-glioma therapy. Brain Pathol. 2008;18(3):423-33.
56. Navis AC, Niclou SP, Fack F, Stieber D, van Lith S, Verrijp K, et al. Increased mitochondrial activity in a novel IDH1-R132H mutant human oligodendroglioma xenograft model: in situ detection of 2-HG and alpha-KG. Acta Neuropathol Commun. 2013;1:18.
57. Trapnell C, Roberts A, Goff L, Pertea G, Kim D, Kelley DR, et al. Differential gene and transcript expression analysis of RNA-seq experiments with TopHat and Cufflinks. Nat Protoc. 2012;7(3):562-78.
58. Sjolund J, Johansson M, Manna S, Norin C, Pietras A, Beckman S, et al. Suppression of renal cell carcinoma growth by inhibition of Notch signaling in vitro and in vivo. The Journal of clinical investigation. 2008;118(1):217-28.
59. Tardito S, Oudin A, Ahmed SU, Fack F, Keunen O, Zheng L, et al. Glutamine synthetase activity fuels nucleotide biosynthesis and supports growth of glutamine-restricted glioblastoma. Nat Cell Biol. 2015;17(12):1556-68.
60. Martinez-Outschoorn UE, Peiris-Pages M, Pestell RG, Sotgia F, Lisanti MP. Cancer metabolism: a therapeutic perspective. Nat Rev Clin Oncol. 2016.
61. Chaumeil MM, Radoul M, Najac C, Eriksson P, Viswanath P, Blough MD, et al. Hyperpolarized (13)C MR imaging detects no lactate production in mutant IDH1 gliomas: Implications for diagnosis and response monitoring. Neuroimage Clin. 2016;12:180-9.
62. Yizhak K, Chaneton B, Gottlieb E, Ruppin E. Modeling cancer metabolism on a genome scale. Mol Syst Biol. 2015;11(6):817.
63. Esmaeili M, Hamans BC, Navis AC, van Horssen R, Bathen TF, Gribbestad IS, et al. IDH1 R132H mutation generates a distinct phospholipid metabolite profile in glioma. Cancer Res. 2014;74(17):4898-907.
64. Ganapathy-Kanniappan S, Vali M, Kunjithapatham R, Buijs M, Syed LH, Rao PP, et al. 3-bromopyruvate: a new targeted antiglycolytic agent and a promise for cancer therapy. Curr Pharm Biotechnol. 2010;11(5):510-7.
65. Ganapathy-Kanniappan S, Kunjithapatham R, Geschwind JF. Anticancer efficacy of the metabolic blocker 3-bromopyruvate: specific molecular targeting. Anticancer Res. 2013;33(1):13-20.
66. Kankotia S, Stacpoole PW. Dichloroacetate and cancer: new home for an orphan drug? Biochim Biophys Acta. 2014;1846(2):617-29.
67. Singh BN, Shankar S, Srivastava RK. Green tea catechin, epigallocatechin-3-gallate (EGCG): mechanisms, perspectives and clinical applications. Biochem Pharmacol. 2011;82(12):1807-21.
68. Altman BJ, Stine ZE, Dang CV. From Krebs to clinic: glutamine metabolism to cancer therapy. Nat Rev Cancer. 2016;16(10):619-34.
69. Sosnicki S, Kapral M, Weglarz L. Molecular targets of metformin antitumor action. Pharmacol Rep. 2016;68(5):918-25.
70. Zhang HH, Guo XL. Combinational strategies of metformin and chemotherapy in cancers. Cancer Chemother Pharmacol. 2016;78(1):13-26.
71. Gong J, Kelekar G, Shen J, Shen J, Kaur S, Mita M. The expanding role of metformin in cancer: an update on antitumor mechanisms and clinical development. Target Oncol. 2016;11(4):447-67.
72. Thupari JN, Pinn ML, Kuhajda FP. Fatty acid synthase inhibition in human breast cancer cells leads to malonyl-CoA-induced inhibition of fatty acid oxidation and cytotoxicity. Biochem Biophys Res Commun. 2001;285(2):217-23.
73. Baldewijns MM, van Vlodrop IJ, Vermeulen PB, Soetekouw PM, van Engeland M, de Bruine AP. VHL and HIF signalling in renal cell carcinogenesis. J Pathol. 2010;221(2):125-38.
74. Metallo CM, Gameiro PA, Bell EL, Mattaini KR, Yang J, Hiller K, et al. Reductive glutamine metabolism by IDH1 mediates lipogenesis under hypoxia. Nature. 2012;481(7381):380-4.
75. Gameiro PA, Yang J, Metelo AM, Perez-Carro R, Baker R, Wang Z, et al. In vivo HIF-mediated reductive carboxylation is regulated by citrate levels and sensitizes VHL-deficient cells to glutamine deprivation. Cell metabolism. 2013;17(3):372-85.
76. Cancer Genome Atlas Research N. Comprehensive molecular characterization of clear cell renal cell carcinoma. Nature. 2013;499(7456):43-9.
77. Hu CJ, Wang LY, Chodosh LA, Keith B, Simon MC. Differential roles of hypoxia-inducible factor 1alpha (HIF-1alpha) and HIF-2alpha in hypoxic gene regulation. Molecular and cellular biology. 2003;23(24):9361-74.
78. Kim JW, Tchernyshyov I, Semenza GL, Dang CV. HIF-1-mediated expression of pyruvate dehydrogenase kinase: a metabolic switch required for cellular adaptation to hypoxia. Cell metabolism. 2006;3(3):177-85.
79. Papandreou I, Cairns RA, Fontana L, Lim AL, Denko NC. HIF-1 mediates adaptation to hypoxia by actively downregulating mitochondrial oxygen consumption. Cell metabolism. 2006;3(3):187-97.
80. Koshiji M, Kageyama Y, Pete EA, Horikawa I, Barrett JC, Huang LE. HIF-1alpha induces cell cycle arrest by functionally counteracting Myc. EMBO J. 2004;23(9):1949-56.
81. Gordan JD, Bertout JA, Hu CJ, Diehl JA, Simon MC. HIF-2alpha promotes hypoxic cell proliferation by enhancing c-myc transcriptional activity. Cancer Cell. 2007;11(4):335-47.
82. Ebert T, Bander NH, Finstad CL, Ramsawak RD, Old LJ. Establishment and characterization of human renal cancer and normal kidney cell lines. Cancer Res. 1990;50(17):5531-6.
83. Skehan P, Storeng R, Scudiero D, Monks A, McMahon J, Vistica D, et al. New colorimetric cytotoxicity assay for anticancer-drug screening. Journal of the National Cancer Institute. 1990;82(13):1107-12.
84. Ibahim MJ, Crosbie JC, Paiva P, Yang Y, Zaitseva M, Rogers PA. An evaluation of novel real-time technology as a tool for measurement of radiobiological and radiation-induced bystander effects. Radiation and environmental biophysics. 2016;55(2):185-94.
85. Kusters B, de Waal R, Wesseling P, Verrijp K, Maass C, Heerschap A, et al. Differential effects of vascular endothelial growth factor a isoforms in a mouse brain metastasis model of human melanoma. Cancer Research. 2003;63(17):5408-13.
86. Navis, A.C., Bourgonje, A., Wesseling, P., Wright, A., Hendriks, W., Verrijp, K., van der Laak, J.A., Heerschap, A., and Leenders, W.P. (2013). Effects of dual targeting of tumor cells and stroma in human glioblastoma xenografts with a tyrosine kinase inhibitor against c-MET and VEGFR2. PLoS One 8, e58262.
87. Nomura, N., Pastorino, S., Jiang, P., Lambert, G., Crawford, J.R., Gymnopoulos, M., Piccioni, D., Juarez, T., Pingle, S.C., Makale, M., et al. (2014). Prostate specific membrane antigen (PSMA) expression in primary gliomas and breast cancer brain metastases. Cancer Cell Int 14, 26.
88. Van den Heuvel, C., Navis, A.C., de Bitter, T., Amiri, H., Verrijp, K., Heerschap, A., Rex, K., Dussault, I., Caenepeel, S., Coxon, A., et al. (2017). Selective MET Kinase Inhibition in MET-Dependent Glioma Models Alters Gene Expression and Induces Tumor Plasticity. Mol Cancer Res 15, 1587-1597.

## Claims

1. Method for *in vitro* determination of the susceptibility and/or resistance of a subject suffering from or at risk of a disease or condition for a drug to treat the disease or condition, comprising:
- performing RNA profiling on an *ex vivo* sample obtained from the subject,
- determining the susceptibility and/or resistance of the subject suffering from or at risk of a disease or condition for a drug to treat the disease or condition
wherein the presence of an alternative splice variant is an indication for the susceptibility and/or resistance,
wherein the RNA profiling is performed by multiplex mRNA sequencing, targeting multiple regions of interest using molecular inversion probes (MIPs),
wherein the sample RNA is converted to cDNA and wherein the MIPs of interest hybridize to the cDNA via an extension probe and a ligation probe that are connected by a backbone sequence, leaving a 112nt gap on the cDNA upon hybridization, and
wherein the MIPs further comprise a detectable moiety, preferably a unique identifier sequence of random nucleotides adjacent to the ligation part of the MIP or to the extension part of the MIP sequence.

2. A method according to claim 1, wherein the alternative splice variant is linked to an aberrance in a metabolic pathway which is in turn linked to the susceptibility and/or resistance of a subject suffering from or at risk of a disease or condition for a drug.

3. A method according to claim 1 or 2, wherein the disease or condition is at least one selected from the group consisting of a cancer, a viral infection, a bacterial infection, an autoimmune disease and a genetic disease.

4. A method according to any one of claims 1 - 3, wherein the sample is selected from the group consisting of a tissue, a tumor tissue, urine, sperm, saliva, blood, blood plasma, cerebrospinal fluid, blood platelets, and/or exosomes, preferably selected from tumor tissue and blood platelets.

5. A method according to any claim 3 or 4, wherein the metabolic pathway is a glucose processing pathway, a glutamine processing pathway and/or a fatty acid pathway.

6. A method according to any one of claims 1-5, wherein the multiple regions of interest are within the mRNA of:
- glucose processing genes: ABAT, ACACA, ACACB, ACLY, ACO2, ACSS2, ADPGK, ALDOA, ARHGAP26, ATG4A. ATP5A1, CBR1, CBS, CHKA, CKB, CPT1A, CYCS, EGLN1, ENO1, G6PC, GAD1, GCLC, GCLM, GFPT1, GLDC, GSS, HK1, HK2, HK3, GLY1, G6PD, Gluconolactonase, PGD, RPIA, RPE, TKT, PGI, ALDOA, GAPDH, PGAM1/2, ENO, PKM1/2, PDHA1, PDK1, PFKB1, PFKMb, PGAM1, PGD, PGK1, PKM, PRDX1, PRKAA1, RPIA, PC, CS, ACO1, IDH1, IDH2, IDH3A, IDH3B, IDH3G, OGDH, SUCLA2, SDHA/B/C/D, FH, MDH1, MDH2, PDK, LDHA, LDHB, SLC16A1, SLC16A3, CA9, CA12, SLC4A10, VHL, SDH, SDHAF2, HPGL/PCC, FH, CS, D-2HGDH, L-2HGDH, FH, IDH1-3A-G, MDH1-2, MYC, OGDH, SDHA-D, VHL, PHD, HIF1a, EPAS2 and/or PDCD1;
- glutamine processing genes: SLC1A5, ASCT2, GLS, GLUD1/2, GOT, GPI, GS, BCAT1, BCAT2, SLC1A2 and/or SLC7A11;
- fatty acid anabolism genes: SLC25A1, ACLY, ACACA, ACACB, FASN, CPT1, SLC5A7, CHKA, CPT2, VLCAD, HADHA/B, SCAD, MCAD, LCAD, SCHA-D, 2-Enoyl-VoA hydratase and/or MCKAT;
- transporter genes; SLC16A1, SLC16A7, SLC2A1, SLC2A3, SLC5A1, SLC5A5, SLC7A1, SLC9A1 and/or SLCA12;
- redox homeostasis genes: NAMPT, NAPRT1, NOX1, NOX3, NOX4A, NQO1, SOD, SOD2, CAT, TAL, TIGAR and/or TRX;
- DNA repair genes: PARP1; MGMT, XRCC2, XRCC3, RAD54, H2AX, MSH2, MLH1, PMS2, MSH6.
- genes with potential involvement in cancer: ALK, AXL, BRAF, KRAS, TP53, MAPK8, MYC, TP53I3, FGFR1, FGFR2, IGF1-R, KDR, NTRK1, NTRK2, PDGFRA, PDGFRB, EGFR, EGFRvIII, ERBB2, ERBB3, ERBB4, MERTK, PLXND1, RET, Androgen receptor (AR), AR variant 7, AR variant 12, FOLH1, KLK3, MET, METdelta14, METdelta7-8, KIT, RON and/or PTEN;
- genes involved in angiogenesis: VEGF-A121, VEGF-A144, VEGF-A165 and/or VEGF-A189
- genes involved in immune suppression: CD274 and/or CTLA4; and/or,
- viral genes: HPV-E2, HPV/E6 and/or HPV-E7.

7. A method according to any one of claims 1 - 6, wherein the presence of an alternative splice variant also provides an indication for treatment with dietary compounds or phytochemicals.

## Patentansprüche

1. Verfahren zur In-vitro-Bestimmung der Empfänglichkeit und/oder Resistenz eines Subjekts, das an einer Krankheit oder einem Zustand leidet oder gefährdet ist, für ein Medikament zur Behandlung der Krankheit oder des Zustands, umfassend:
- Durchführung der RNA-Profilierung an einer gewonnenen Ex-vivo-Probe vom Subjekt,
- Bestimmung der Empfänglichkeit und/oder Resistenz des Subjekts, das an einer Krankheit oder einem Zustand leidet oder gefährdet ist, für ein Medikament zur Behandlung der Krankheit oder des Zustands,
wobei die Präsenz einer alternativen Spleißvariante ein Hinweis auf die Empfänglichkeit und/oder Resistenz ist,
wobei die RNA-Profilierung durch Multiplex-mRNA-Sequenzierung, abzielend auf mehrere Regionen von Interesse unter Verwendung von Molekularinversionssonden (MIPs), durchgeführt wird,
wobei die Proben-RNA in cDNA umgewandelt wird und wobei die MIPs von Interesse mit der cDNA über eine Verlängerungssonde und eine Ligations-Sonde hybridisieren, die durch eine Backbonesequenz verbunden sind, wobei eine 112nt-Lücke auf der cDNA bei der Hybridisierung zurückbleibt, und
worin die MIPs ferner eine nachweisbare Gruppe, bevorzugt eine einzigartige Identifizierungssequenz aus zufälligen Nukleotiden, die an den Ligationsteil des MIP oder an den Verlängerungsteil der MIP-Sequenz angrenzt, umfassen.

2. Verfahren nach Anspruch 1, wobei die alternative Spleißvariante mit einer Abweichung in einem Stoffwechselweg verbunden ist, die wiederum mit der Empfänglichkeit und/oder Resistenz eines Subjekts, das an einer Krankheit oder einem Zustand leidet oder gefährdet ist, für ein Medikament, verbunden ist.

3. Verfahren nach Anspruch 1 oder 2, wobei die Krankheit oder der Zustand zumindest ein(e) ist, ausgewählt aus der Gruppe bestehend aus einem Krebs, einer viralen Infektion, einer bakteriellen Infektion, einer Autoimmunerkrankung und einer genetischen Erkrankung handelt.

4. Verfahren nach einem der Ansprüche 1 - 3, wobei die Probe aus der Gruppe ausgewählt ist, die aus einem Gewebe, einem Tumorgewebe, Urin, Sperma, Speichel, Blut, Blutplasma, Zerebrospinalflüssigkeit, Blutplättchen und/oder Exosomen, bevorzugt ausgewählt aus Tumorgewebe und Blutplättchen, besteht.

5. Verfahren nach einem der Ansprüche 3 oder 4, wobei der Stoffwechselweg ein Glukoseverarbeitungsweg, ein Glutaminverarbeitungsweg und/oder ein Fettsäureweg ist.

6. Verfahren nach einem der Ansprüche 1-5, wobei die mehrfachen Regionen von Interesse innerhalb der mRNA von:
- Glukoseverarbeitungsgenen: ABAT, ACACA, ACACB, ACLY, ACO2, ACSS2, ADPGK, ALDOA, ARHGAP26, ATG4A. ATP5A1, CBR1, CBS, CHKA, CKB, CPT1A, CYCS, EGLN1, ENO1, G6PC, GAD1, GCLC, GCLM, GFPT1, GLDC, GSS, HK1, HK2, HK3, GLY1, G6PD, Gluconolactonase, PGD, RPIA, RPE, TKT, PGI, ALDOA, GAPDH, PGAM1/2, ENO, PKM1/2, PDHA1, PDK1, PFKB1, PFKMb, PGAM1, PGD, PGK1, PKM, PRDX1, PRKAA1, RPIA, PC, CS, ACO1, IDH1, IDH2, IDH3A, IDH3B, IDH3G, OGDH, SUCLA2, SDHA/B/C/D, FH, MDH1, MDH2, PDK, LDHA, LDHB, SLC16A1, SLC16A3, CA9, CA12, SLC4A10, VHL, SDH, SDHAF2, HPGL/PCC, FH, CS, D-2HGDH, L- 2HGDH, FH, IDH1-3A-G, MDH1-2, MYC, OGDH, SDHA-D, VHL, PHD, HIF1a, EPASZ und/oder PDCD1;
- Glutaminverarbeitungsgenen: SLC1A5, ASCT2, GLS, GLUD1/2, GOT, GPI, GS, BCAT1, BCAT2, SLC1A2 und/oder SLC7A11;
- Fettsäureanabolismusgenen: SLC25A1, ACLY, ACACA, ACACB, FASN, CPT1, SLC5A7, CHKA, CPT2, VLCAD, HADHA/B, SCAD, MCAD, LCAD, SCHA-D, 2-Enoyl-VoA-Hydratase und/oder MCKAT;
- Transportergenen: SLC16A1, SLC16A7, SLC2A1, SLC2A3, SLC5A1, SLC5A5, SLC7A1, SLC9A1 und/oder SLCA12;
- Redox-Homöostasegenen: NAMPT, NAPRT1, NOX1, NOX3, NOX4A, NQO1, SOD, SOD2, CAT, TAL, TIGAR und/oder TRX;
- DNA-Reparaturgenen: PARP1; MGMT, XRCC2, XRCC3, RAD54, H2AX, MSH2, MLH1, PMS2, MSH6.
- Genen mit potenzieller Beteiligung an Krebs: ALK, AXL, BRAF, KRAS, TP53, MAPK8, MYC, TP5313, FGFR1, FGFR2, IGF1-R, KDR, NTRK1, NTRK2, PDGFRA, PDGFRB, EGFR, EGFRvlll, ERBB2, ERBB3, ERBB4, MERTK, PLXND1, RET, Androgenrezeptor (AR), AR Variante 7, AR Variante 12, FOLH1, KLK3, MET, METdeIta14, METdeIta7-8, KIT, RON und/oder PTEN;
- Genen, die an der Angiogenese beteiligt sind: VEGF-A121, VEGF-A144, VEGF-A165 und/oder VEGF-A189
- Genen, die an der Immunsuppression beteiligt sind: CD274 und/oder CTLA4; und/oder,
- viralen Genen: HPV-E2, HPV/E6 und/oder HPV-E7.

7. Verfahren nach einem der Ansprüche 1 bis 6, wobei die Präsenz einer alternativen Spleißvariante auch eine Indikation für Behandlung mit diätetischen Verbindungen oder Phytochemikalien bereitstellt.

## Revendications

1. Procédé de détermination *in vitro* de la sensibilité et/ou de la résistance d'un sujet souffrant ou risquant de développer une maladie ou une condition à un médicament pour traiter la maladie ou la condition, comprenant :
- effectuer un profilage d'ARN sur un échantillon *ex vivo* obtenu du sujet ;
- déterminer la sensibilité et/ou la résistance du sujet souffrant ou risquant de développer une maladie ou une condition à un médicament pour traiter la maladie ou la condition
où la présence d'un variant d'épissage alternatif est une indication de la sensibilité et/ou de la résistance,
où le profilage de l'ARN est effectué par séquençage multiplex d'ARNm, ciblant plusieurs régions d'intérêt à l'aide de sondes d'inversion moléculaire (MIP),
où l'échantillon d'ARN est converti en ADNc et où les MIP d'intérêt s'hybrident à l'ADNc via une sonde d'extension et une sonde de ligature qui sont connectées par une séquence principale,
laisser un espace de 112 nt sur l'ADNc lors de l'hybridation, et
où les MIP comprennent en outre une fraction détectable, de préférence une séquence d'identification unique de nucléotides aléatoires adjacents à la partie de ligature de la MIP ou à la partie d'extension de la séquence de la MIP.

2. Procédé selon la revendication 1, où le variant d'épissage alternatif est lié à une anomalie dans une voie métabolique qui est à son tour liée à la sensibilité et/ou à la résistance d'un sujet souffrant ou risquant de développer une maladie ou une condition à un médicament.

3. Procédé selon la revendication 1 ou 2, où la maladie ou la condition est au moins l'une choisie dans le groupe constitué d'un cancer, d'une infection virale, d'une infection bactérienne, d'une maladie auto-immune et d'une maladie génétique.

4. Procédé selon l'une quelconque des revendications 1 à 3, où l'échantillon est sélectionné dans le groupe constitué d'un tissu, d'un tissu tumoral, d'urine, de sperme, de salive, de sang, de plasma sanguin, de liquide céphalorachidien, de plaquettes sanguines et/ou d'exosomes, de préférence sélectionné parmi un tissu tumoral et des plaquettes sanguines.

5. Procédé selon l'une quelconque des revendications 3 ou 4, où la voie métabolique est une voie de transformation du glucose, une voie de transformation de la glutamine et/ou une voie des acides gras.

6. Procédé selon l'une quelconque des revendications 1 à 5, où les multiples régions d'intérêt se trouvent dans l'ARNm de :
- gènes de transformation du glucose : ABAT, ACACA, ACACB, ACLY, ACO2, ACSS2, ADPGK, ALDOA, ARHGAP26, ATG4A. ATP5A1, CBR1, CBS, CHKA, CKB, CPT1A, CYCS, EGLN1, ENO1, G6PC, GAD1, GCLC, GCLM, GFPT1, GLDC, GSS, HK1, HK2, HK3, GLY1, G6PD, Gluconolactonase, PGD, RPIA, RPE, TKT, PGI, ALDOA, GAPDH, PGAM1/2, ENO, PKM1/2, PDHA1, PDK1, PFKB1, PFKMb, PGAM1, PGD, PGK1, PKM, PRDX1, PRKAA1, RPIA, PC, CS, AC01, IDH1, IDH2, IDH3A, IDH3B, IDH3G, OGDH, SUCLA2, SDHA/B/C/D, FH, MDH1, MDH2, PDK, LDHA, LDHB, SLC16A1, SLC16A3, CA9, CA12, SLC4A10, VHL, SDH, SDHAF2, HPGL/PCC, FH, CS, D-2HGDH, L-2HGDH, FH, IDH1-3A-G, MDHI-2, MYC, OGDH, SDHA-D, VHL, PHD, H1F1a, EPAS2 et/ou PDCD1 ;
- gènes de transformation de la glutamine : SLC1A5, ASCT2, GLS, GLUD1/2, GOT, GPI, GS, BCAT1, BCAT2, SLC1A2 et/ou SLC7A11 ;
- gènes de l'anabolisme des acides gras : SLC25A1, ACLY, ACACA, ACACB, FASN, CPT1, SLC5A7, CHKA, CPT2, VLCAD, HADHA/B, SCAD, MCAD, LCAD, SCHA-D, 2-Enoyl-VoA hydratase et/ou MCKAT ;
- gènes de transporteurs : SLC16A1, SLC16A7, SLC2A1, SLC2A3, SLC5A1, SLC5A5, SLC7A1, SLC9A1 et/ou SLCA12 ;
- gènes d'homéostasie redox : NAMPT, NAPRT1, NOX1, NOX3, NOX4A, NQO1, SOD, SOD2, CAT, TAL, TIGAR et/ou TRX ;
- gènes de réparation de l'ADN : PARP1 ; MGMT, XRCC2, XRCC3, RAD54, H2AX, MSH2, MLH1, PMS2, MSH6.
- gènes potentiellement impliqués dans le cancer : ALK, AXL, BRAF, KRAS, TP53, MAPK8, MYC, TP53I3, FGFR1, FGFR2, IGF1-R, KDR, NTRK1, NTRK2, PDGFRA, PDGFRB, EGFR, EGFRvIII, ERBB2, ERBB3, ERBB4, MERTK, PLXND1, RET, récepteur aux androgènes (RA), variant 7 du RA, variant 12 du RA, FOLH1, KLK3, MET, METdelta4, METdelta7-8, KIT, RON et/ou PTEN ;
- gènes impliqués dans l'angiogenèse : VEGF-A121, VEGF-A144, VEGF-A165 et/ou VEGF-A189;
- gènes impliqués dans l'immunosuppression : CD274 et/ou CTLA4; et/ou,
- gènes viraux : HPV-E2, HPV/E6 et/ou HPV-E7.

7. Procédé selon l'une quelconque des revendications 1 à 6, où la présence d'un variant d'épissage alternatif fournit également une indication pour un traitement avec des composés alimentaires ou des composés phytochimiques.
